# FASCICULE DE BREVET EUROPEEN

(11) **EP 3 601 545 B1**
(45) Date de publication et mention de la délivrance du brevet: **23.02.2022**
(21) Numéro de dépôt: 18717217.6
(22) Date de dépôt: 21.03.2018
(51) Int. Cl.: C12N 7/04, C12N 15/113, C12N 15/86

(54) **PRODUCTION D'ARN PAR DES LEVURES A PARTICULES PSEUDO-VIRALES RECOMBINANTES**
HERSTELLUNG VON RNA DURCH HEFEN MIT REKOMBINANTEN PSEUDOVIRALEN PARTIKELN
PRODUCTION OF RNA BY YEASTS WITH RECOMBINANT PSEUDO-VIRAL PARTICLES

(30) Priorité: 21.03.2017 FR 1752309
(43) Date de publication de la demande: 05.02.2020
(73) Titulaire: Centre National de la Recherche Scientifique, 75794 Paris Cedex 16 (FR); Université d'Orléans, 45067 Orléans Cedex 2 (FR)
(72) Inventeur: PIGEON, Lucie, 45160 Olivet (FR); RAHMOUNI, A. Rachid, 45110 Germigny des Pres (FR); PICHON-RABENANDRASANA, Harivony, Chantal, 45550 St Denis de l'Hôtel (FR); MIDOUX, Patrick, 45550 St Denis de l'Hôtel (FR)
(74) Mandataire: Regimbeau
(86) Numéro de dépôt international: PCT/EP2018/025066
(87) Numéro de publication internationale: WO 2018/171946

(56) Documents cités:
- EP-A1- 2 377 938
- WO-A1-2004/108943
- WO-A1-2014/077354
- WO-A1-2016/185125
- WO-A2-2007/072056
- KATARZYNA PACHULSKA-WIECZOREK ET AL: "Determinants of Genomic RNA Encapsidation in the Saccharomyces cerevisiae Long Terminal Repeat Retrotransposons Ty1 and Ty3", VIRUSES, vol. 8, no. 7, 14 juillet 2016 (2016-07-14), page 193, XP055428321, DOI: 10.3390/v8070193
- L Cristofari ET AL: "A 5'-3' long-range interaction in Ty1 RNA controls its reverse transcription and retrotransposition", The EMBO Journal , vol. 21 15 août 2002 (2002-08-15), pages 4368-4379, XP055428562, DOI: 10.1093/emboj/cdf436 Extrait de l'Internet: URL:http://emboj.embopress.org/content/emb ojnl/21/16/4368.full.pdf [extrait le 2017-11-24]
- MARY ANN CHECKLEY ET AL: "Ty1 Gag Enhances the Stability and Nuclear Export of Ty1 mRNA : TY1gRNA Dynamics", TRAFFIC, vol. 14, no. 1, 17 octobre 2012 (2012-10-17), pages 57-69, XP055428317, DK ISSN: 1398-9219, DOI: 10.1111/tra.12013
- T. DERRIEN ET AL: "The GENCODE v7 catalog of human long noncoding RNAs: Analysis of their gene structure, evolution, and expression", GENOME RESEARCH, vol. 22, no. 9, 1 septembre 2012 (2012-09-01), pages 1775-1789, XP055193005, ISSN: 1088-9051, DOI: 10.1101/gr.132159.111
- ANNE PREL ET AL: "Highly efficient in vitro and in vivo delivery of functional RNAs using new versatile MS2-chimeric retrovirus-like particles", MOLECULAR THERAPY - METHODS & CLINICAL DEVELOPMENT, vol. 2, 21 octobre 2015 (2015-10-21), page 15039, XP055254802, DOI: 10.1038/mtm.2015.39
- LIZ JULIA ET AL: "lncRNAs and microRNAs with a role in cancer development", BIOCHIMICA ET BIOPHYSICA ACTA. GENE REGULATORY MECHANISMS, ELSEVIER, AMSTERDAM, NL, vol. 1859, no. 1, 4 juillet 2015 (2015-07-04), pages 169-176, XP029378019, ISSN: 1874-9399, DOI: 10.1016/J.BBAGRM.2015.06.015
- TONY GUTSCHNER ET AL: "The hallmarks of cancer: A long non-coding RNA point of view", RNA BIOLOGY, vol. 9, no. 6, 1 juin 2012 (2012-06-01), pages 703-719, XP055137471, ISSN: 1547-6286, DOI: 10.4161/rna.20481

## Description

### DOMAINE TECHNIQUE

La demande concerne la production d'ARN d'intérêt, plus particulièrement d'ARN messager (ARNm) d'intérêt ou d'ARN long non-codant (ARNlnc) d'intérêt, par des levures recombinantes, plus particulièrement par des levures à particules pseudo-virales recombinantes.

La demande concerne ces levures, ainsi que les particules pseudo-virales qu'elles produisent. La demande concerne également des moyens pour leur production, notamment des vecteurs d'acides nucléiques et des kits, ainsi que des applications médicales, plus particulièrement vaccinales, anti-tumorales, anti-infectieuses et pro-régénératives.

### ARRIÈRE-PLAN

Différentes méthodes de production d'ARN sont connues dans l'art. Ces méthodes comprennent notamment des méthodes de synthèse *in vitro* qui utilisent notamment des polymérases à ARN.

La production d'ARN, notamment d'ARNm, qui sont compétents pour la traduction en protéines dans des cellules de mammifère, pose le problème particulier de modifications post-transcriptionnelles telles que la présence d'une queue polyA en 3', une coiffe 7-méthylguanosine (m7G) en 5', voire certaines méthylations d'adénines et de cytosines. Les méthodes de synthèse *in vitro* de l'art antérieur proposent par exemple de placer des analogues de coiffe dans le mélange transcriptionnel, tels que m7G5'ppp5'G. Afin de stimuler l'incorporation du dinucléotide en début de chaîne, les méthodes de synthèse *in vitro* de l'art antérieur proposent en outre de diminuer la concentration de la guanosine triphosphate (GTP) dans le mélange transcriptionnel par rapport aux autres nucléosides triphosphate (NTP), ce qui diminue l'efficacité de transcription. En outre, l'analogue de coiffe peut alors être incorporé dans les deux orientations ce qui conduit à 50 % d'ARN mal coiffé. Ces méthodes de synthèse *in vitro* d'ARN sont donc peu efficaces.

D'autres méthodes de synthèse *in vitro* proposent d'utiliser une enzyme chimérique comprenant les domaines catalytiques d'une ARN triphosphatase, d'une guanylyltransférase, d'une N7-guanine méthyltransférase, et d'une polymérase à ARN ADN-dépendante (*cf*. par exemple, WO 2011/128444 au nom de EUKARYS). Ces méthodes de synthèse d'ARN *in vitro* restent néanmoins coûteuses.

Des méthodes de production recombinante d'ARN ont par ailleurs été développées. Par exemple, une méthode propose d'utiliser la machinerie de la mitochondrie, pour en faire une unité de production et de stockage d'ARN (*cf*. par exemple WO 2010/084371 au nom de MITOPROD). Les ARN ainsi produits ne présentent toutefois pas de coiffe en 5', ce qui limite d'autant leurs applications médicales.

La demande propose des moyens de production d'ARN qui ne présentent pas tous les inconvénients de l'art antérieur.

### RÉSUMÉ

La demande concerne la production d'ARN, plus particulièrement d'ARN messager (ARNm) ou d'ARN long non-codant (ARNlnc), par des levures recombinantes, plus particulièrement par des levures à particules pseudo-virales recombinantes. Les levures ont été modifiées génétiquement pour produire des particules pseudo-virales recombinantes qui contiennent cet ARN, et ne rétro-transcrivent pas cet ARN ni ne le rétro-intègrent dans leur génome.

L'ARN ainsi produit peut avantageusement présenter une queue polyA en 3' et une coiffe 7-méthylguanosine en 5'. Ils peuvent donc être compétents pour une éventuelle traduction dans des cellules de mammifère. En outre, l'ARN peut être produit en quantités relativement importantes.

La demande concerne ces levures, ainsi que les particules pseudo-virales qu'elles produisent. La demande concerne également des moyens pour leur production, notamment des vecteurs d'acides nucléiques et des kits, ainsi que des applications médicales, plus particulièrement vaccinales, anti-tumorales et pro-régénératives.

Les particules pseudo-virales recombinantes sont produites à partir de certains éléments de rétrotransposon Ty de levure, mais ne rétro-transcrivent pas l'ARN qu'elles contiennent. Les éléments de rétrotransposon Ty de levure mis en œuvre comprennent notamment :
- la protéine Gag de rétrotransposon Ty de levure, ainsi que
- un fragment de l'ARN qui correspond à cette protéine, en l'occurrence un fragment de cet ARN qui ne comprend pas le codon départ de traduction *[start]* de cette séquence ARN.

Ce fragment ARN n'a pas vocation à être traduit en protéine dans la levure : il y sert de séquence d'adressage, qui guide l'ARN (d'intérêt) vers la protéine Gag, venant ainsi former un complexe entre cet ARN et la protéine Gag. Ce complexe prend la forme d'une particule, en l'occurrence d'une particule pseudo-virale recombinante, qui est formée par la polymérisation de la protéine Gag, et dans laquelle l'ARN se trouve encapsulé.

Les éléments de rétrotransposon Ty de levure mis en œuvre ne comprennent pas les moyens de transcription inverse de Ty. Plus particulièrement, ils ne comprennent pas les moyens d'intégrase de Ty, ou plus généralement les moyens de protéine précurseur Pol de Ty.

### BRÈVE DESCRIPTION DES FIGURES

Certaines des figures de la demande sont en couleurs. La demande telle que déposée contient la version en couleurs de ces figures, qui peut donc être consultée par inspection du dossier de la demande telle que déposée.

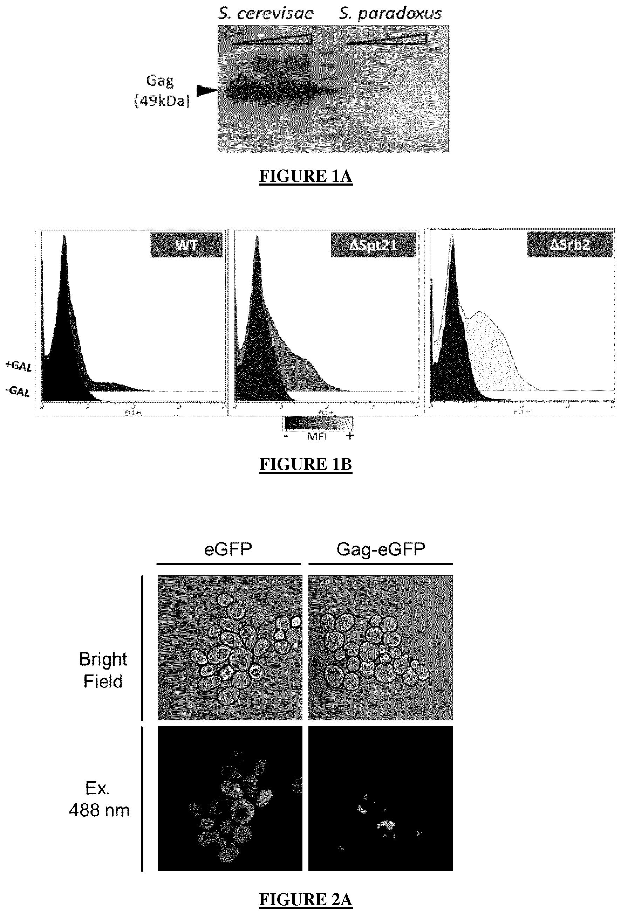

**Figure 1A** : La figure 1A illustre l'expression de la protéine Gag (Tyl) chez les levures *Saccharomyces cerevisae* et *Saccharomyces paradoxus.* L'analyse de l'expression de la protéine Gag a été réalisée par transfert de Western anti-Gag (49kDa) sur des échantillons de lysat cellulaire. Contrairement à *S. cerevisiae*, *S.paradoxus* est dépourvue de rétrotransposon Ty1.
**Figure 1B** : La figure 1B illustre la mesure par cytométrie en flux de l'expression de l'eGFP chez la levure *S. paradoxus* sauvage (WT), et les mutants transcriptionnels ΔSpt21 et ΔSrb2. Après transformation des levures avec un plasmide codant pour l'eGFP sous le contrôle d'un promoteur inductible au galactose, les mutants transcriptionnels permettent une augmentation significative du nombre de cellules eGFP positives ainsi que de l'intensité de la fluorescence (quantité par cellule, de *T-bodies* (particules pseudo-virales) dont la protéine Gag est marquée par la eGFP).
   WT = *S. paradoxus* sauvage ; ΔSpt21 = *S. paradoxus* délétée pour le gène Spt21 ; ΔSrb2 = *S. paradoxus* délétée pour le gène Srb2.
**Figure 2A** : La figure 2A illustre les analyses par microscopie confocale de fluorescence de l'expression de l'eGFP et de la protéine de fusion Gag-eGFP chez la levure *S. paradoxus.* eGFP = Protéine fluorescente verte enrichie ; *bright field* = exposition en lumière transmise ; Ex. 488nm = exposition à une longueur d'onde 488nm.

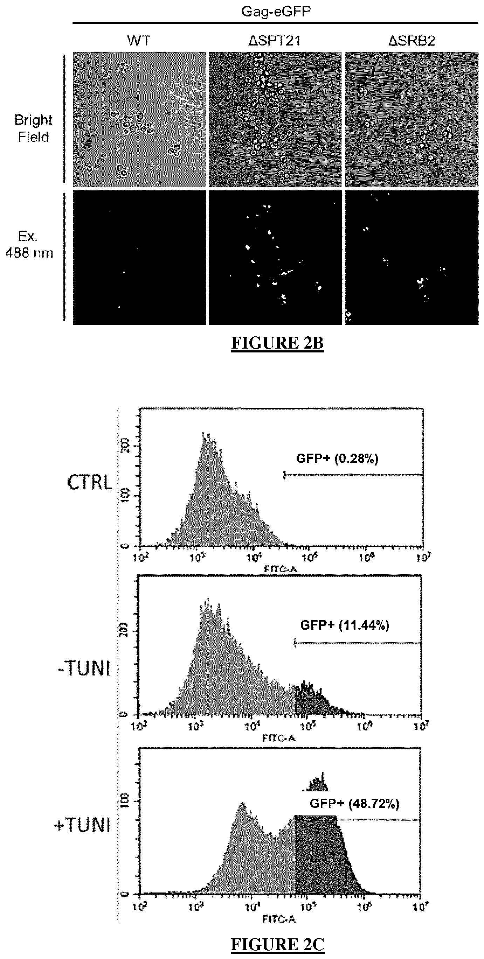

**Figure 2B** : La figure 2B illustre les analyses par microscopie confocale de fluorescence de l'expression de la protéine de fusion Gag-eGFP chez la levure *S. paradoxus* sauvage (WT), et les mutants transcriptionnels ΔSpt21 et ΔSrb2.
   WT = S. paradoxus sauvage ; ΔSpt21 = *S. paradoxus* délétée pour le gène Spt21 ; ΔSrb2 = *S. paradoxus* délétée pour le gène Srb2.
**Figure 2C** : La figure 2C illustre l'analyse par cytométrie en flux de l'expression de *T-bodies* eGFP (protéine de fusion Gag-eGFP) chez le mutant transcriptionnel de la levure *S. paradoxus* ΔSrb2 après un traitement de 8 heures à la tunicamycine (TUNI). L'expression des *T-bodies* est sous le contrôle d'un promoteur inductible au galactose. CTRL = contrôle ; -TUNI = sans tunicamycine ; +TUNI = avec tunicamycine.

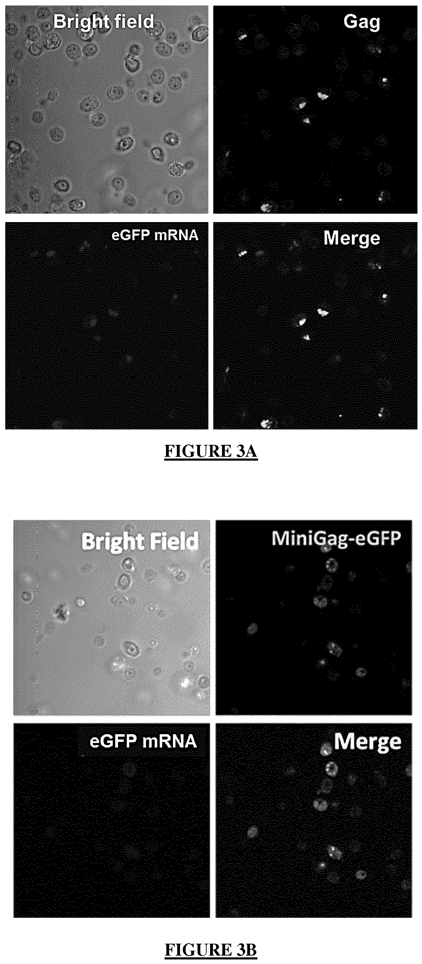

**Figure 3A** : La figure 3A illustre des expériences de FISH (*fluorescence in situ hybridization*) pour localiser les ARNm modèles codant la protéine eGFP, par l'utilisation des amorces ciblant l'ARNm eGFP marquées avec le fluorophore Cy5 sur la levure *S. paradoxus* ΔSrb2 transformée avec un plasmide de fusion Gag-eGFP. *Bright field* = exposition en lumière transmise ; *Merge* = combinaison des images.
**Figure 3B** : La figure 3B illustre des expériences de FISH (*fluorescence in situ hybridization*) pour localiser les ARNm codant la protéine eGFP (ARNm modèles), par l'utilisation des amorces ciblant l'ARNm eGFP marquées avec le fluophore Cy5 sur la levure *S. paradoxus* ΔSrb2 transformée avec un plasmide de fusion MiniGag-eGFP. *Bright field* = exposition en lumière transmise ; *Merge* = combinaison des images.

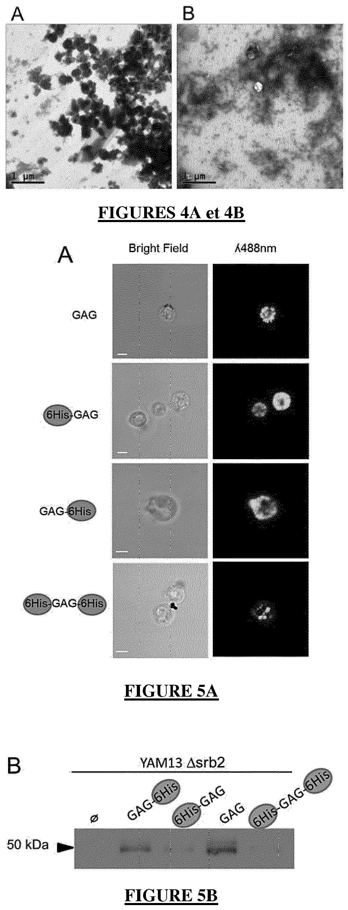

**Figure 4** : La figure 4 illustre l'image en microscopie électronique à transmission de *T-bodies* suite à l'extraction protéique par deux techniques différentes (à gauche : le culot obtenu par la méthode de lyse par les billes ; à droite le culot obtenu par la méthode de sonication). Les échantillons ont subi un marquage à l'acétate d'uranyle à 2%.
**Figure 5A** : La figure 5A illustre l'observation par microscopie confocale de fluorescence de *T-bodies* par immuno fluorescence (anticorps antiTyl/GAG) chez la levure *S. paradoxus* ΔSrb2 exprimant la protéine Gag, la protéine de fusion 6His-Gag, Gag-6His, 6His-Gag-6His. *Bright field* = exposition en lumière transmise ; λ488nm = exposition à une longueur d'onde 488nm.

**Figure 5B** : La figure 5B illustre la détection de l'expression de la protéine Gag par transfert de western (anticorps antiTyl/Gag) chez la levure *S. paradoxus* ΔSrb2 exprimant la protéine Gag, la protéine de fusion 6His-Gag, Gag-6His, 6His-Gag-6His. YAM13Δsrb2 = souche de levure *S. paradoxus* YAM13 délétée pour le gène Srb2.

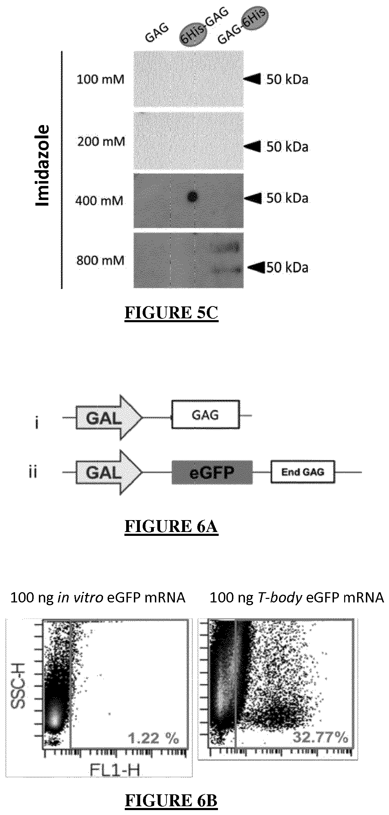

**Figure 5C** : La figure 5C illustre l'analyse par transfert de western (anticorps antiTyl/GAG) des fractions éluées par des tampons de concentration croissante en imidazole (100mM, 200mM, 400 mM, 800 mM). Ces fractions correspondent aux *T-bodies* extraits à partir de souches de levure exprimant Gag, 6His-Gag et Gag-6His puis purifiées sur une colonne chargée en nickel.
**Figure 6A** : La figure 6A est une représentation schématique des constructions transformées chez la levure *S. paradoxus* ΔSrb2. La première construction est responsable de la formation des *T-bodies* (i), la seconde est un plasmide permettant la transcription d'un ARN eGFP disposant en 3'd'une séquence « EndGAG » permettant le ciblage de l'ARN au sein des *T-bodies* (ii).
   GAL = promoteur galactose ; EndGAG = mini-GAG (= séquence d'adressage vers le rétrosome).
**Figure 6B** : La figure 6B illustre l'analyse par cytométrie en flux de cellules dendritiques de souris (lignée DC2.4) exprimant eGFP après 24 heures de transfection à l'aide d'un ARN eGFP soit produit par transcription in vitro (figure de gauche) soit produit à partir des *T-bodies* et vectorisés avec des liposomes cationiques (Lip100).
   SSC-H = hauteur de diffraction de la lumière ; FL1-H = intensité mesurée en vert.

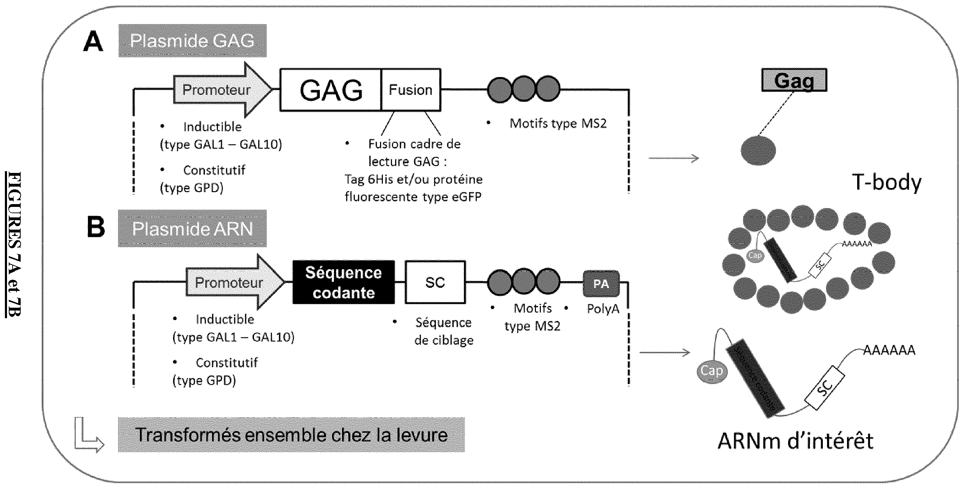

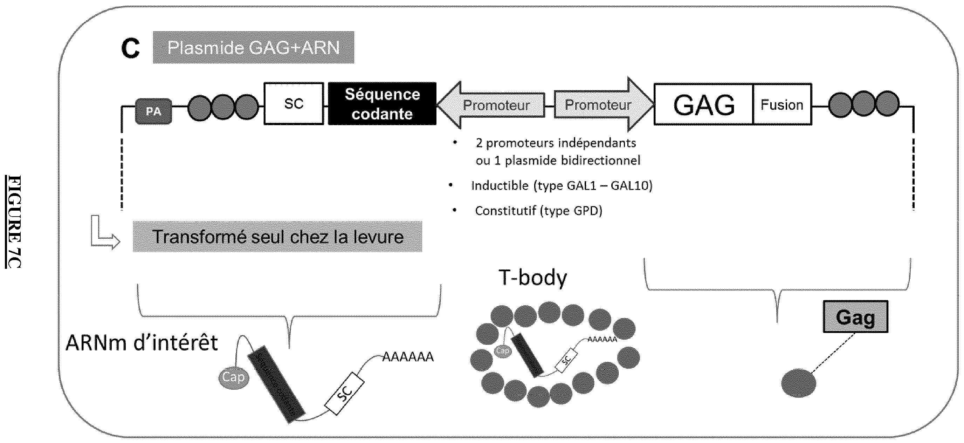

**Figures 7A, 7B** **et** **7C** : Les figures sont une représentation schématique des plasmides nécessaires à la formation de *T-bodies* chargés en ARN(m) d'intérêt utilisés pour modifier la souche de levure Ty-négative (*S. paradoxus* par exemple). Le plasmide GAG (figure 7A) et le plasmide ARN (figure 7B) sont utilisés ensemble pour transformer la souche de levure Ty-négative. La séquence codant la protéine Gag (Figure 7A) peut optionnellement être liée à une séquence codant une étiquette de purification (étiquette poly-histidine ou tag 6His), ou un marqueur de détection (par exemple la protéine fluorescente eGFP). MS2 = séquence du bactériophage MS2 (étiquette de purification).
   L'expression des deux plasmides permet la synthèse de la protéine Gag et de l'ARN(m) d'intérêt. La séquence de signal SC sur le plasmide ARN est une séquence d'adressage rétrosomal qui permet la formation du complexe ARNm/protéine Gag, qui est appelé rétrosome T (ou *T-body*). Cette séquence d'adressage rétrosomal peut par exemple être une séquence GAG modifiée pour ne plus coder la protéine Gag, notamment par délétion du codon départ de transcription *[start]* en début de séquence (séquence « miniGag » ou « End Gag »).
   Le plasmide GAG+ARN (figure 7C) permet à la fois la synthèse de la protéine Gag et de l'ARNm d'intérêt.

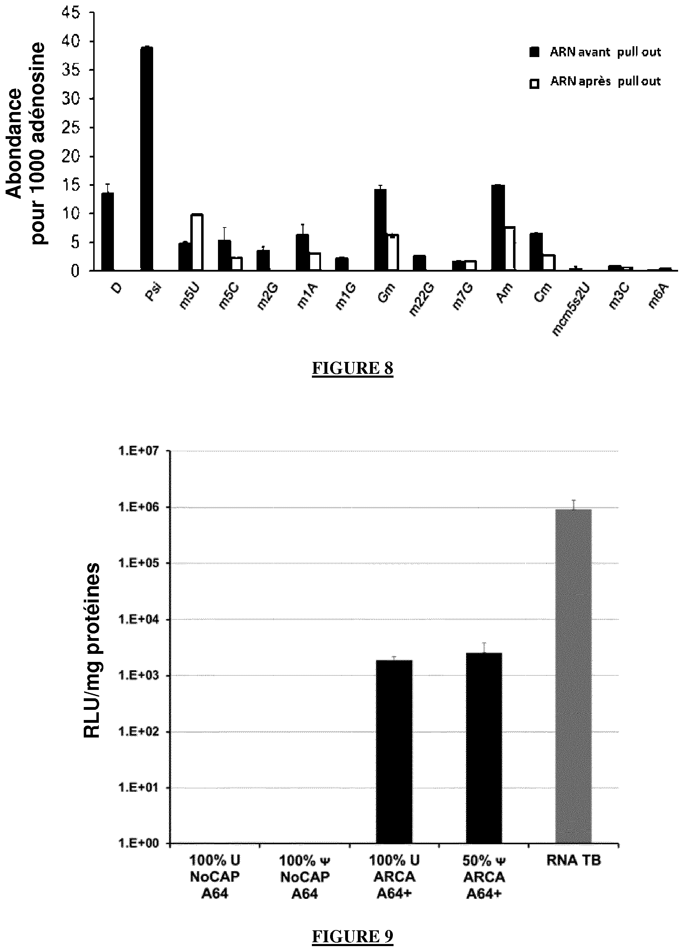

**Figure 8** : La figure 8 illustre l'étude en spectrométrie de masse des modifications chimiques présentes au niveau de l'ARN messager bioproduits. Cette technique repose sur une analyse par spectrométrie de masse (LC-MS) des fragments obtenus après hydrolyse complète par RNase T1 de l'ARN étudié. Les barres d'histogrammes noires (ARN avant « *pull out* ») correspondent à la moyenne de 2 échantillons d'ARNs extraits à partir de *T-bodies* issus d'une lyse mécanique. Les barres d'histogrammes blanches (ARN après *« pull out* ») correspondent à la moyenne de 2 échantillons d'ARNs extraits à partir de *T-bodies* issus d'une lyse mécanique suivie d'un protocole « *pull out* » permettant la purification exclusive de l'ARN d'intérêt.
   ABREVATIONS :
   **D** = dihydrouridine ; **Psi** = pseudouridine ; **m5U** = 5-méthyluridine ; **m5C** = 5-méthylcytidine ; **m2G** = N2-methylguanosine ; **m1A** = 1-méthyladénosine ; **m1G** = 1-methylguanosine ; **Gm** = 2'-O-méthylguanosine ; **m22G** = N2,N2-dimethylguanosine ; **m7G** = 7-méthylguanosine ; **Am** = 2'-O-méthyladénosine ; **Cm** = 2'-O-méthylcytidine ; **mcm5s2U** = 5-methoxycarbonylmethyl-2-thiouridine ; **m3C** = 3-méthylcytidine et **m6A** = N6-methyladenosine.
**Figure 9** : La figure 9 illustre la mesure de l'activité luciférase (RLU/mg protéins) sur cellules DC2.4 après 20h de transfection avec 50 ng d'ARN Luciférase produit *in vitro* (barre noires) et bioproduits (RNA TB, barre grise) vectorisé avec Lipofectamine Messenger Max (LFM). Les ARNs synthétisés *in vitro* :
   - n'ont pas subi des modifications : U = uridine ; NoCAP = absence de coiffe et A64 = pas de réaction de polyadénylation supplémentaire ; ou
   - ont subi des modifications : ARCA = ajout d'une coiffe ARCA ; Ψ = incorporation de 50 à 100% de résidus pseudouridine et A64+ = réaction de polyadénylation supplémentaire.

### DESCRIPTION DÉTAILLÉE

La demande décrit les objets définis dans les revendications telles que déposées, les objets décrits ci-dessous et les objets illustrés en partie « exemples ».

La demande est notamment relative à un procédé de production (recombinante) d'ARN, à un procédé de production de composition pharmaceutique, et à des produits susceptibles d'être mis en œuvre ou produits dans ces procédés, notamment des acides nucléiques (plus particulièrement des vecteurs d'acides nucléiques, tels que des plasmides), des kits, des cellules recombinantes de bactéries, des cellules recombinantes de levure, ainsi que des complexes, granules ou particules pseudo-virales recombinants.

La demande met en œuvre certains éléments de rétrotransposon Ty de levure (mais pas tous les éléments de ce rétrotransposon). Elle met notamment en œuvre des moyens de modifications génétiques de levure, qui dérivent de la protéine Gag de rétrotransposon Ty de levure et de l'ARN de cette protéine. La demande ne met toutefois pas en œuvre les moyens de transcription inverse de rétrotransposon Ty de levure.

La demande permet notamment de produire des ARN, notamment des ARN messagers (ARNm) ou ARN long non-codant (ARNlnc), qui présentent une queue polyA en 3' et/ou une coiffe 7-méthylguanosine (m7G) en 5', voire une méthylation de résidu(s) adénine et/ou cytosine et/ou pseudouridine. Les moyens de la demande permettent en outre de produire ces ARN en quantité relativement importante, et à coût réduit. Les moyens de la demande permettent donc d'accéder en clinique aux applications médicales des ARN (tels que ARNm et ARNlnc), notamment dans le domaine de l'immunothérapie et de la thérapie génique.

La demande est ainsi relative à un procédé de production (recombinante) d'ARN, ledit procédé comprenant :
- le fait de cultiver des cellules de levure, qui ont été modifiées génétiquement pour produire cet ARN, et
- le fait de collecter cet ARN.

Cet ARN comprend avantageusement une séquence d'adressage, à savoir une séquence qui lui permet d'être adressé à une protéine Gag de rétrotransposon Ty de levure.

En sus de la séquence d'adressage, l'ARN peut notamment comprendre au moins un ARN d'intérêt, tel qu'au moins un ARN de procaryote (notamment de bactérie), de virus (notamment de *Zika* ou *Influenza*), ou d'eucaryote, notamment d'eucaryote autre que la levure (par exemple d'humain), et/ou au moins un ARN artificiel ou non-naturel (par exemple un ARN dont la traduction est une séquence qui comprend différents épitopes de virus et/ou bactéries),

Cet ARN peut avantageusement être un ARNm ou un ARNlnc, plus particulièrement un ARNm.

Cet ARN peut être un ARN hétérologue à la levure qui le produit, voire hétérologue aux levures en général.

Les modifications génétiques qui ont été apportées aux cellules de levure pour produire cet ARN comprennent notamment le transfert dans ces cellules de levure (notamment par transfection ou transformation) d'une construction d'acide nucléique qui code la protéine Gag de rétrotransposon Ty de levure.

Alternativement ou complémentairement, plus particulièrement complémentairement, les modifications génétiques qui ont été apportées aux cellules de levure pour produire cet ARN comprennent notamment le transfert dans ces cellules de levure (notamment par transfection ou transformation) d'une construction d'acide nucléique dont le transcrit ARN comprend :
- ladite séquence d'adressage et/ou
- la séquence d'ARN d'intérêt,

plus particulièrement dont le transcrit ARN comprend au moins ladite séquence d'adressage,
plus particulièrement dont le transcrit ARN comprend la séquence d'un ARN d'intérêt liée à la séquence d'adressage qui adresse (ou guide) cet ARN vers la protéine Gag de rétrotransposon Ty de levure.

L'ARN (plus particulièrement l'ARNm ou ARNlnc) est produit par les cellules de levure avec la protéine Gag de rétrotransposon Ty de levure, sous la forme d'une structure que l'on peut qualifier de complexe, de granule ou de particule pseudo-virale.

Un ou plusieurs ARN différents peuvent ainsi être produits.

La(les), ou au moins une, des construction(s) d'acide nucléique transférée(s) peut(peuvent) être intégrée(s) dans le génome, ou bien ne pas être intégrée(s) dans le génome mais être présente(s) dans le noyau ou le cytoplasme, par exemple sous forme d'épisome.

Par exemple, une construction d'acide nucléique qui code la protéine Gag de rétrotransposon Ty de levure peut être intégrée dans le génome de la levure, tandis que la construction d'acide nucléique dont le transcrit ARN comprend ladite séquence d'adressage et/ou la séquence d'ARN d'intérêt, peut ne pas être intégrée dans le chromosome (mais être présente dans le noyau et/ou le cytoplasme, notamment sous forme d'épisome).

Alternativement, il peut n'y avoir qu'une seule construction qui contient les deux acides nucléiques (acide nucléique codant Gag, et acide nucléique dont le transcrit ARN comprend ladite séquence d'adressage et/ou la séquence d'ARN d'intérêt), et cette construction peut être intégrée dans le génome de la levure, ou être présente dans le noyau ou le cytoplasme (par exemple sous forme d'épisome).

La demande est ainsi relative à un procédé de production d'ARN (plus particulièrement d'ARNm ou d'ARNlnc) d'intérêt, ledit procédé comprenant :
- le fait de cultiver des cellules de levure, qui ont été modifiées génétiquement pour produire des particules pseudo-virales (recombinantes) qui comprennent un ARN dont la séquence comprend ladite séquence d'adressage et/ou l'ARN d'intérêt, plus particulièrement qui comprend ladite séquence d'adressage liée audit ARN d'intérêt, et
- le fait de collecter l'ARN qui est contenu dans les particules pseudo-virales (recombinantes) ainsi formées.

Le terme procaryote est entendu conformément à sa signification usuelle dans le domaine. Il vise ici plus particulièrement une bactérie, notamment une bactérie à potentiel infectieux et/ou pathogène pour l'espèce humaine.

Le terme virus est entendu conformément à sa signification usuelle dans le domaine. Il vise ici plus particulièrement un virus, notamment un virus à potentiel infectieux et/ou pathogène pour l'espèce humaine, notamment *Zika* ou *Influenza.*

Le terme eucaryote est entendu conformément à sa signification usuelle dans le domaine. Lorsqu'il se rapporte à l'ARN, l'ARNm ou à l'ARNlnc de la demande, il vise ici plus particulièrement un eucaryote autre que la levure, plus particulièrement un eucaryote pluricellulaire tel qu'un mammifère (plus particulièrement un humain ou un mammifère non-humain), plus particulièrement un humain.

Le terme Ty est entendu conformément à sa signification usuelle dans le domaine. Il vise ici plus particulièrement un rétrotransposon Ty1, Ty2, Ty3, Ty4 ou Ty5, plus particulièrement un rétrotransposon Ty1, Ty2 ou Ty3, plus particulièrement un rétrotransposon Ty1.

Le terme levure est entendu conformément à sa signification usuelle dans le domaine.

Il vise ici plus particulièrement une levure du genre *Saccharomyces* ou du genre *Pichia*, plus particulièrement du genre *Saccharomyces.*

Parmi les levures du genre *Saccharomyces*, sont plus particulièrement visées les espèces *paradoxus* et *cerevisiae*, notamment l'espèce *paradoxus.*

Parmi les levures du genre *Pichia*, sont plus particulièrement visées l'espèce *P. pastoris.* Un exemple de souche de *S. paradoxus* est la souche qui est accessible auprès de l'ATCC sous le numéro 76528^{™}. ATCC^{®} est l'*American Type Culture Collection* (10801 University Blvd. ; Manassas, Virginia 20110-2209 ; U.S.A.).

Un exemple de souche de *S. cerevisiae* est la souche YAM510, qui ne contient pas de rétrotransposon Ty1 fonctionnel, et qui a été décrite dans Rothstein *et al.* 1983 et Thomas *et al.* 1989.

En sus d'avoir été modifiées génétiquement pour produire des particules pseudo-virales (recombinantes), les cellules de levure de la demande sont des cellules d'une levure qui :
- est naturellement dépourvue de rétrotransposon Ty1, ou bien qui
- est naturellement pourvue de rétrotransposon Ty1, mais dont la séquence de rétrotransposon Ty1 a été modifiée pour ne pas produire ou être déficiente en rétrosome T.

Alternativement ou complémentairement, la levure peut être dépourvue de rétrotransposon Ty2 et Ty3, ou bien (en est naturellement pourvue mais) a été modifiée génétiquement pour ne pas produire de rétrosome T qui serait codé par un rétrotransposon Ty2 ou Ty3.

Alternativement ou complémentairement, la levure peut être dépourvue de rétro-transposon Ty2, Ty3, Ty4 et Ty5, ou (bien en est naturellement pourvue mais) a été modifiée génétiquement pour ne pas produire de rétrosome T qui serait codé par un rétrotransposon Ty2, Ty3, Ty4 ou Ty5.

Avantageusement, la levure est une levure qui est (naturellement) dépourvue de rétro-transposon Ty, ou bien est une levure qui est naturellement pourvue en un ou plusieurs rétro-transposon Ty mais dont la séquence du ou de ces rétro-transposons Ty a été modifiée génétiquement pour ne pas produire de rétrosome T.

Avantageusement, la levure est dépourvue de rétro-transposon Ty, ou a été modifiée génétiquement pour ne pas produire de rétrosome T.

Le fait de mettre en œuvre une levure qui est dépourvue de ces(s) rétrotransposon(s) Ty, ou qui ne produit pas ce(s) rétrosome(s) Ty, permet notamment de limiter ou éviter que l'ARN (ARNm ou ARNlnc) produit ne soit rétro-transposé (en ADNc) (et donc de limiter ou éviter la « perte » d'ARNm ou ARNlnc par une rétro-transcription ADNc non désirée). La levure *Saccharomyces paradoxus* est un exemple de levure qui est naturellement dépourvue de rétrotransposon Ty, notamment de rétrotransposon Ty1. *S. paradoxus* peut donc être directement mise en œuvre pour transfert des acides nucléiques de i. et/ou ii.

La levure *Pichia pastoris* est une levure qui est naturellement dépourvue de rétrotransposon Ty, et peut donc être directement mise en œuvre pour transfert des acides nucléiques de i. et/ou ii.

La levure *Saccharomyces cerevisiae* est un exemple de levure qui est naturellement pourvue de rétrotransposons Ty, notamment d'un rétrotransposon Ty1. Elle doit donc être modifiée génétiquement pour ne pas produire de rétrosome Ty, notamment de rétrosome Ty1.

Une modification génétique permettant de ne pas produire de rétrosome(s) Ty peut par exemple comprendre la délétion (dans le chromosome de la levure) du promoteur d'expression du(des) rétrotransposon(s) Ty, et/ou une mutation de ce chromosome (par délétion et/ou remplacement et/ou insertion d'un ou plusieurs nucléotides) venant interrompre au moins un des cadres ouverts de lecture du(des) rétrotransposon(s) Ty. Conformément à la demande, les modifications génétiques apportées aux cellules de levure pour qu'elles produisent lesdites particules pseudo-virales (recombinantes) comprennent la transfection ou transformation de ces cellules de levure par :
i. un acide nucléique comprenant une première séquence nucléotidique, et/ou, de préférence et,
ii. un acide nucléique comprenant une deuxième séquence nucléotidique,
   dans lequel :
      - la première séquence nucléotidique comprend ou est une séquence ADN qui code l'expression de la protéine Gag d'un rétrotransposon Ty de levure,
      - la deuxième séquence nucléotidique comprend ou est une séquence ADN dont le transcrit ARN comprend :
         a. la séquence dudit ARN (ARNm ou ARNlnc) d'intérêt, et/ou
         b. ladite séquence d'adressage,
   plus particulièrement dont le transcrit ARN comprend au moins ladite séquence d'adressage,
   plus particulièrement dont le transcrit ARN comprend :
      a. la séquence dudit ARN (ARNm ou ARNlnc) d'intérêt, liée à
      b. ladite séquence d'adressage.

L'acide nucléique de i. peut par exemple être sous forme d'épisomes se répliquant de manière autonome ou être intégré aux chromosomes de la levure.

L'acide nucléique de ii. peut par exemple être sous forme d'épisomes se répliquant de manière autonome ou être intégré aux chromosomes de la levure.

L'acide nucléique de i. code l'expression de la protéine Gag qui va former la particule pseudo-virale, ou être comprise dans le polymère protéique qui forme cette particule (*cf*. Figures 7A, 7B et 7C).

L'acide nucléique de ii. « code » la transcription de l'ARN qui contient l'ARN d'intérêt à produire, lié à une séquence ARN d'adressage qui guide cet ARN d'intérêt vers la protéine Gag de la particule pseudo-virale en formation (*cf*. Figures 7A, 7B et 7C).

La combinaison des acides nucléiques de i. et ii. produit ainsi une particule pseudovirale contenant l'ARN d'intérêt (par exemple un ARNm ou un ARNlnc) (*cf*. Figures 7A, 7B et 7C).

L'acide nucléique de ii. (ou chacune des séquences qu'il contient) a donc vocation à être transcrit en ARN dans la levure, mais n'a pas vocation particulière à y être traduit en protéine. L'acide nucléique de ii. (ou chacune des séquences qu'il contient) peut donc être une séquence qui, dans une levure, ne peut être traduite en protéine (ou polypeptide).

Par contre, l'ARN ainsi transcrit et produit dans la cellule de levure peut être un ARN qui code l'expression d'une protéine (ou d'un polypeptide) dans une cellule de mammifère (par exemple un ARN qui code l'expression d'un ou plusieurs antigènes dans une cellule de mammifère).

La protéine Gag peut notamment être :
- celle du rétrotransposon Ty1 de *S. cerevisiae,* c'est-à-dire la protéine de séquence SEQ ID NO : 3, ou
- une séquence qui est identique à au moins 90% à la séquence de SEQ ID NO : 3 [le pourcentage d'identité étant calculé sur la plus longue des deux séquences].

Par exemple, la protéine Gag peut notamment être une protéine Gag d'un autre rétrotransposon de levure autre que le rétrotransposon Ty1 de *S. cerevisiae* (rétrotransposon de *S. cerevisiae* autre que Ty1, et/ou rétrotransposon de levure autre que *S. cerevisiae*, par exemple rétrotransposon Ty1 de levure autre que *S. cerevisiae*), dont la séquence est identique à au moins 90% à la séquence de SEQ ID NO : 3.

La protéine Gag de rétrotransposon Ty de levure peut ainsi notamment être une protéine, dont la séquence est la séquence de SEQ ID NO : 3 ou une séquence qui est identique à au moins 90% (plus particulièrement à au moins 95%) à la séquence de SEQ ID NO : 3, et qui est une protéine Gag de rétrotransposon Ty1, Ty2 ou Ty3 de levure.

Dans la deuxième séquence nucléotidique, la « séquence b. » a notamment pour fonction d'adresser la « séquence a. » (en l'occurrence, l'ARN d'intérêt) vers la protéine Gag de rétrotransposon Ty qui est produite par la première séquence nucléotidique, plus particulièrement de l'adresser vers un polymère protéique contenant cette protéine Gag qui est synthétisé dans la cellule de levure génétiquement modifiée.

La deuxième séquence nucléotidique a vocation à être transcrite en ARN, mais n'a pas vocation particulière à être traduite en protéine dans la levure. C'est en effet sous forme ARN qu'elle y exerce sa fonction d'adressage ou de guide. La deuxième séquence nucléotidique peut donc être avantageusement dépourvue de cadre de lecture dont le premier codon serait le codon départ de traduction *[start]* (ATG).

Avantageusement, la séquence d'adressage ou « séquence b. » comprend un fragment de la séquence ARN dont la traduction en acides aminés est la séquence de ladite protéine Gag de rétrotransposon Ty de levure.

Ce fragment peut comprendre un fragment d'au moins 150 nucléotides, plus particulièrement d'au moins 200 nucléotides, plus particulièrement d'au moins 250 nucléotides, plus particulièrement d'au moins 300 nucléotides, plus particulièrement d'au moins 350 nucléotides, plus particulièrement d'au moins 398 nucléotides, plus particulièrement d'au moins 500 nucléotides, plus particulièrement d'au moins 1000 nucléotides, plus particulièrement d'au moins 1078 nucléotides. Dans ce qui suit, l'expression « au moins 150 nucléotides » inclut explicitement chacun des seuils supérieurs qui sont ci-dessus indiqués (au moins 200 nucléotides, etc.).

Ce fragment peut notamment être le fragment d'une séquence ARN dont la traduction en acides aminés serait :
- la séquence de la protéine Gag de rétrotransposon Ty1 de *S. cerevisiae,* à savoir la protéine de SEQ ID NO : 3, ou
- la séquence de la protéine Gag d'un autre rétrotransposon de levure (rétrotransposon autre que Ty1, par exemple Ty2 ou Ty3, et/ou levure autre que *S. cerevisiae*) qui est identique à au moins 90% avec la séquence de SEQ ID NO : 3 (le pourcentage d'identité étant calculé sur la longueur de la plus longue des deux séquences). Ce fragment d'au moins 150 nucléotides peut notamment comprendre ou être :
   - la séquence de SEQ ID NO : 16 (398 nucléotides), ou
   - un (sous-)fragment d'au moins 150 nucléotides de la séquence de SEQ ID NO : 16.

Par exemple, le fragment peut comprendre :
- la séquence de SEQ ID NO : 18 (1078 nucléotides), ou
- une séquence qui est identique à au moins 90% avec la séquence de SEQ ID NO : 18 (et qui comprend un fragment d'au moins 150 nucléotides de la séquence ARN dont la traduction en acides aminés est la séquence de ladite protéine Gag de rétrotransposon Ty de levure, plus particulièrement qui comprend la séquence de SEQ ID NO : 16 ou un (sous-)fragment d'au moins 150 nucléotides de la séquence de SEQ ID NO : 16) [le pourcentage d'identité étant calculé sur la longueur de la plus longue des deux séquences].

Avantageusement, la « séquence b. » ne comprend pas le codon départ de traduction [*start*] (AUG) de la séquence ARN de ladite protéine Gag de rétrotransposon Ty de levure. Dans la séquence ARN dont la traduction serait la séquence de SEQ ID NO : 3 (protéine Gag de Ty1 de *S. cerevisiae),* ce codon départ est le codon AUG qui est le transcrit du codon ATG en positions 1-3 de la séquence de SEQ ID NO : 2. Ce codon départ de traduction peut être délété, ou être muté en un codon autre que « *start* », par exemple en un codon stop (UAA, UAG ou AGA).

Plus généralement, le fragment ARN qui est compris dans ladite séquence b. d'adressage, et qui est un fragment de la séquence ARN dont la traduction en acides aminés serait la séquence de ladite protéine Gag de rétrotransposon Ty de levure, ne comprend avantageusement pas de cadre de lecture dont le premier codon serait un codon départ de traduction (AUG). Plus généralement, ce fragment ARN ne comprend pas de cadre de lecture qui, dans une levure, serait un cadre ouvert de lecture (cette séquence a vocation à être transcrite en ARN dans la levure, mais n'a pas vocation particulière à y être traduite en protéine).

Plus généralement, la séquence d'adressage b. ne comprend avantageusement pas de cadre de lecture dont le premier codon serait le codon départ de traduction (AUG). Plus généralement, elle ne comprend pas de cadre de lecture qui, dans une levure, serait un cadre ouvert de lecture (cette séquence a vocation à être transcrite en ARN dans la levure, mais n'a pas vocation particulière à y être traduite en protéine).

Par exemple, la « séquence b. » peut être ou comprendre :
- la séquence de SEQ ID NO : 14 ou
- une séquence qui est identique à au moins 90% à la séquence de SEQ ID NO : 14 (et qui comprend un fragment d'au moins 150 nucléotides de la séquence ARN dont la traduction en acides aminés serait la séquence de ladite protéine Gag de rétrotransposon Ty de levure, plus particulièrement qui comprend la séquence de SEQ ID NO : 16 ou un (sous-)fragment d'au moins 150 nucléotides de la séquence de SEQ ID NO : 16) [le pourcentage d'identité étant calculé sur la longueur de la plus longue des deux séquences],
sans pour autant comprendre le codon départ (AUG) de la séquence ARN de ladite protéine Gag de rétrotransposon Ty de levure, plus généralement sans comprendre de cadre de lecture dont le premier codon serait un codon départ de traduction AUG, plus généralement sans comprendre de cadre ouvert de lecture.

Complémentairement à l'absence de ce codon départ de traduction, la « séquence b. » peut comprendre un ou plusieurs codons stop (UAA, UAG, ou AGA) dans au moins un, voire chacun des trois, cadres de lecture.

Plus généralement, la deuxième séquence nucléotidique ne comprend avantageusement pas de cadre de lecture dont le premier codon serait le codon départ de traduction [ATG], plus généralement elle ne comprend pas de cadre de lecture qui, dans une levure, serait un cadre ouvert de lecture (cette séquence a vocation à être transcrite en ARN dans la levure, mais n'a pas vocation particulière à y être traduite en protéine).

Avantageusement, l'acide nucléique de ii. ne comprend pas de séquence ADN qui code la séquence ARN complète de ladite protéine Gag de rétrotransposon Ty de levure.

L'acide nucléique de i. et l'acide nucléique de ii. codent la formation, dans (le cytoplasme desdites) lesdites cellules de levure, de complexes qui comprennent :
- (un polymère protéique qui comprend) la protéine Gag traduite à partir de la première séquence nucléotidique et
- l'ARN (ARNm ou ARNlnc) transcrit à partir de la deuxième séquence nucléotidique.

Plus particulièrement, l'acide nucléique de i. et l'acide nucléique de ii. codent la formation, dans (le cytoplasme desdites) lesdites cellules de levure, de granules (recombinants) ou de particules pseudo-virales (*Virus-Like Particles* ou *VLP*) (recombinantes) qui :
- sont formées par (un polymère protéique qui comprend) la protéine Gag traduite à partir de la première séquence nucléotidique, et qui
- encapsulent l'ARN (ARNm ou ARNlnc), transcrit à partir de la deuxième séquence nucléotidique.

Le terme « code » (et ses équivalents grammaticaux) est entendu dans sa signification usuelle dans le domaine. Ce terme est appliqué tant à l'expression d'une séquence ADN en protéine, c'est-à-dire sa transcription et sa traduction (codons ADN transcrits en codons ARN, puis traduits en acides aminés conformément au code génétique universel, et en tenant dûment compte de la dégénérescence de ce code).

Par simplification, ce terme peut ici être aussi appliqué à la (simple) transcription d'une séquence ADN en séquence ARN (par exemple, nucléotides A, T, G et C transcrits en nucléotides A, U, G et C, respectivement), ou à la (simple) traduction d'une séquence ARN en acides aminés (conformément au code génétique universel, et en tenant dûment compte de la dégénérescence de ce code).

L'expression « code l'expression » ou « code la transcription » peut être comprise par la personne du métier comme impliquant la présence dans la séquence ADN de séquence(s) permettant d'induire, notamment d'initier, la transcription, par exemple la présence de la séquence d'(au moins) un promoteur.

La première séquence nucléotidique comprend donc avantageusement une séquence ADN (ou ADNc, ou ADNg) qui est la séquence codante [*CDS*] du cadre ouvert de lecture de la protéine Gag d'un rétrotransposon Ty de levure, sous le contrôle d'un promoteur pour l'expression de ce cadre ouvert.

La séquence ADN de la deuxième séquence nucléotidique comprend avantageusement une séquence ADN qui est le rétro-transcrit d'une séquence ARN qui comprend l'ARN (ARNm ou ARNlnc) d'intérêt, sous le contrôle d'un promoteur pour la transcription de cet ADN (mais de préférence sans comprendre de cadre de lecture, qui, dans une levure, serait un cadre ouvert de lecture).

Avantageusement, l'acide nucléique de i. et l'acide nucléique de ii. sont des vecteurs d'acide nucléique, plus particulièrement des vecteurs d'acide nucléique adaptés au transfert d'acides nucléiques dans des cellules de levure, par exemple adaptés à la transfection ou la transformation de ces cellules de levure. L'acide nucléique i. et l'acide nucléique de ii. peuvent (chacun indépendamment l'un de l'autre) être ou ne pas être intégratifs (c'est-à-dire être ou ne pas être destinés à s'intégrer au chromosome de la cellule qui les reçoit en transfert).

Le terme « vecteur d'acide nucléique » est entendu conformément à sa signification usuelle dans le domaine. Il vise plus particulièrement un plasmide, plus particulièrement un plasmide réplicatif ou un plasmide épisomique. Ainsi, l'acide nucléique de i. et l'acide nucléique de ii. peuvent chacun indépendamment l'un de l'autre être des plasmides, notamment des plasmides épisomiques ou des plasmides réplicatifs.

L'acide nucléique de i. peut par exemple être un vecteur d'acide nucléique, notamment un plasmide (épisomique ou non intégratif), qui comprend la première séquence nucléotidique en insert pour sa transcription et sa traduction en cellule de levure.

L'acide nucléique de ii. peut par exemple être un vecteur d'acide nucléique, notamment un plasmide (épisomique ou non intégratif), qui comprend la deuxième séquence nucléotidique en insert pour sa transcription en cellule de levure.

Par exemple, l'acide nucléique de i. est un plasmide épisomique, tandis que l'acide nucléique de ii. est un plasmide non intégratif, plus particulièrement un vecteur réplicatif.

L'acide nucléique de i. et l'acide nucléique de ii. peuvent être une seule et même molécule, ou bien être deux molécules distinctes ou séparées. Par exemple, l'acide nucléique de i. et l'acide nucléique de ii. peuvent être un seul et même vecteur d'acide nucléique, notamment un seul et même plasmide, ou être deux vecteurs d'acide nucléique séparés ou distincts, notamment deux plasmides séparés ou distincts.

Avantageusement, l'acide nucléique de i. et l'acide nucléique de ii. sont chacun (indépendamment l'un de l'autre) présents en plusieurs copies. Avantageusement, le nombre de copies de l'acide nucléique de ii. est supérieur (par exemple 2, 3, 4, 5 ou 6 fois supérieur) au nombre de copies de l'acide nucléique de i.

La première séquence nucléotidique peut être intégrée dans le génome de la levure qui a reçu l'acide nucléique de i. en transfert, ou ne pas y être intégrée.

La deuxième séquence nucléotidique peut être intégrée dans le génome de la levure qui a reçu l'acide nucléique de ii. en transfert, ou ne pas y être intégrée.

Par exemple, la première séquence nucléotidique est intégrée dans le génome de la levure, et la deuxième séquence nucléotidique n'y est pas intégrée (mais est présente dans le noyau et/ou cytoplasme, par exemple en étant comprise dans l'acide nucléique de ii.).

Ainsi, conformément à la demande, les modifications génétiques apportées aux cellules de levure pour qu'elles produisent lesdites particules pseudo-virales (recombinantes) comprennent l'insertion dans ces cellules de levure (par exemple par transfection ou transformation) de :
i. un acide nucléique comprenant une première séquence nucléotidique, et/ou, de préférence et,
ii. un acide nucléique comprenant une deuxième séquence nucléotidique,
   dans lequel :
   - l'acide nucléique de i. et l'acide nucléique de ii. sont une seule et même molécule, ou bien deux molécules distinctes ou séparées,
   - la première séquence nucléotidique comprend ou est une séquence ADN qui code l'expression de la protéine Gag d'un rétrotransposon Ty de levure,
   - la deuxième séquence nucléotidique comprend ou est une séquence ADN qui code la transcription d'un ARN dont la séquence comprend (c'est-à-dire une séquence ADN dont le transcrit ARN comprend) une séquence (d'adressage) qui comprend un fragment d'au moins 150 nucléotides de la séquence ARN de ladite protéine Gag de rétrotransposon Ty de levure, mais qui ne comprend pas le codon départ de traduction (*start*) AUG de la séquence ARN de cette protéine (plus généralement qui ne comprend pas de cadre de lecture dont le premier codon serait le codon départ de traduction AUG, plus généralement qui ne comprend pas de cadre de lecture qui, dans la levure, serait un cadre ouvert de lecture),
   - l'acide nucléique de ii. ne comprend pas de séquence ADN codant la séquence ARN complète de ladite protéine Gag de rétrotransposon Ty de levure,
   - l'acide nucléique de i. et l'acide nucléique de ii. ne comprennent pas de séquence codant la transcriptase inverse d'un rétrotransposon Ty de levure.

L'acide nucléique de i. et l'acide nucléique de ii. codent ainsi la formation, dans (le cytoplasme desdites) lesdites cellules de levure, d'une particule pseudo-virale qui est formée par un polymère protéique qui comprend la protéine Gag traduite à partir de la première séquence nucléotidique, et qui encapsule ledit ARN (ARNm ou ARNlnc) d'intérêt transcrit à partir de la deuxième séquence nucléotidique.

En sus de la séquence ADN qui code l'expression de la protéine Gag d'un rétrotransposon Ty de levure, la première séquence nucléotidique, l'acide nucléique de i., peut comprendre une séquence ADN qui code l'expression d'un marqueur pour la détection de la protéine Gag (par exemple, une séquence ADN qui code un marqueur fluorescent, par exemple un marqueur fluorescent lié ou fusionné à une protéine fluorescente verte (GFP), notamment une eGFP) et/ou l'expression d'une étiquette pour la purification de la protéine Gag (par exemple, une séquence ADN qui code une étiquette poly-histidine et/ou la séquence du bactériophage MS2, fusionnée la protéine Gag) (*cf*. Figures 7A, 7B et 7C).

En sus de la séquence d'adressage, la deuxième séquence nucléotidique comprend avantageusement un ARN (ARNm ou ARNlnc) d'intérêt lié à cette séquence d'adressage. La deuxième séquence nucléotidique peut en outre comprendre une séquence ADN étiquette de purification, telle que une ou plusieurs (par exemple, trois) copies de la séquence du bactériophage MS2 (*cf*. Figures 7A, 7B et 7C).

L'acide nucléique de i. peut être intégré dans le génome de la levure, ou bien ne pas être intégré dans le génome mais être présent dans le noyau et/ou le cytoplasme de la levure, par exemple sous forme d'épisome.

L'acide nucléique de ii. peut être intégré dans le génome de la levure, ou bien ne pas être intégré dans le génome mais être présent dans le noyau et/ou le cytoplasme de la levure. Par exemple, l'acide nucléique de i. peut être intégré dans le génome de la levure, et l'acide nucléique de ii. peut ne pas être intégré dans le génome mais être présent dans le noyau et/ou le cytoplasme de la levure, par exemple sous forme d'épisome.

Par exemple, l'acide nucléique de i. et l'acide nucléique de ii. peuvent être tous deux intégrés dans le génome de la levure (ils peuvent alors être une seule et même molécule d'acide nucléique).

Plus particulièrement, la deuxième séquence nucléotidique comprend ou est une séquence ADN qui code la transcription d'un ARN dont la séquence comprend (c'est-à-dire une séquence ADN dont le transcrit ARN comprend) :
a. la séquence dudit ARN (ARNm ou ARNlnc) d'intérêt, liée à
b. une séquence (d'adressage) qui comprend un fragment d'au moins 150 nucléotides de la séquence ARN de ladite protéine Gag de rétrotransposon Ty de levure, mais qui ne comprend pas le codon départ (*start*) AUG de la séquence ARN de cette protéine (plus généralement qui ne comprend pas de cadre de lecture dont le premier codon serait le codon départ de traduction AUG, plus généralement qui ne comprend pas de cadre de lecture qui, dans la levure serait un cadre ouvert de lecture).

Le plus généralement, l'acide nucléique de i. et l'acide nucléique de ii. sont chacun de l'ADN. Le plus généralement, la première séquence nucléotidique et la deuxième séquence nucléotidique sont chacune une séquence ADN, plus particulièrement ADNc. La première séquence nucléotidique est (ou comprend) une séquence ADN qui code l'expression de la protéine Gag d'un rétrotransposon Ty de levure. Elle comprend donc, ou est, un cadre de lecture ouvert qui code la séquence de cette protéine.

Le plus généralement, dans la deuxième séquence nucléotidique, la liaison entre la séquence a. dudit ARN (ARNm ou ARNlnc) d'intérêt, et la séquence d'adressage b., est une liaison covalente, ou une liaison directe, ou une liaison covalente et directe. Cette liaison peut notamment être à l'extrémité 5' ou 3' de la séquence de l'ARN d'intérêt.

Par exemple, la séquence d'adressage b. est liée par covalence à l'extrémité 5' de la séquence de l'ARN d'intérêt.

Par exemple, la séquence d'adressage b. est liée directement et par covalence à l'extrémité 3' de la séquence de l'ARN d'intérêt.

La première séquence nucléotidique contient avantageusement (au moins) un promoteur pour son expression, ou pour la transcription et la traduction du cadre ouvert de lecture qu'elle contient. La première séquence nucléotidique peut donc comprendre un cadre de lecture ouvert qui code la séquence de la protéine Gag d'un rétrotransposon Ty de levure, sous le contrôle d'un promoteur pour la transcription et la traduction de ce cadre ouvert de lecture dans une cellule de levure.

La deuxième séquence nucléotidique contient avantageusement (au moins) un promoteur pour sa transcription, notamment pour sa transcription dans une cellule de levure.

Le promoteur de la première séquence nucléotidique peut être le même que celui de la deuxième séquence nucléotidique, ou bien il peut s'agir de promoteurs différents.

Ces promoteurs peuvent chacun indépendamment l'un de l'autre être un promoteur constitutif ou un promoteur inductible (par exemple, un promoteur inductible par le galactose).

Ces promoteurs peuvent chacun indépendamment l'un de l'autre être un promoteur fort, c'est-à-dire un promoteur auquel le complexe de transcription (et notamment l'ARN polymérase) peut se lier pour initier la transcription, sans qu'il y ait nécessairement intervention de facteurs supplémentaires (tels que le facteur sigma). Par exemple, le promoteur de la deuxième séquence nucléotidique peut être un promoteur fort. Avantageusement, le promoteur de la première séquence nucléotidique est un promoteur pour l'expression de cette première séquence nucléotidique dans une cellule eucaryote, plus particulièrement une cellule de levure (plus particulièrement, pour la transcription et la traduction, dans une cellule eucaryote, notamment dans une cellule de levure, du cadre ouvert de lecture qui est contenu dans cette première séquence nucléotidique). Avantageusement, le promoteur de la deuxième séquence nucléotidique est un promoteur pour la transcription de la deuxième séquence nucléotidique dans une cellule eucaryote, plus particulièrement une cellule de levure.

Tout promoteur inductible ou constitutif que la personne du métier considère approprié peut être mis en œuvre.

Des exemples de promoteurs inductibles comprennent notamment les promoteurs Gall, GallO, Tet07, Met.

Des exemples de promoteurs constitutifs comprennent notamment les promoteurs ADH, CYC, PGK1, GPD.

Avantageusement, la première séquence nucléotidique contient (au moins) une origine pour sa réplication, ou pour la réplication du cadre ouvert de lecture qu'elle contient.

De même, la deuxième séquence nucléotidique contient avantageusement (au moins) une origine pour sa réplication.

L'origine de réplication contenue dans la première séquence nucléotidique est avantageusement une origine de réplication en procaryote et eucaryote, plus particulièrement une origine de réplication en bactérie et levure.

De même, l'origine de réplication contenue dans la deuxième séquence nucléotidique est avantageusement une origine de réplication en procaryote et eucaryote, plus particulièrement une origine de réplication en bactérie et levure.

De manière avantageuse, les cellules de levure sont des cellules de levure qui ne comprennent pas de séquence nucléotidique qui permettrait la rétrotransposition de l'ARN (ARNm ou ARNlc) d'intérêt. Un objectif est ici de limiter ou éviter la perte des ARN (ARNm ou ARNlnc) produits par leur rétro-transcription en ADNc.

Ainsi, de manière avantageuse, l'acide nucléique de i. et l'acide nucléique de ii. ne comprennent pas de séquence codant la transcriptase inverse d'un rétrotransposon Ty de levure, plus généralement de toute séquence codant l'expression d'une transcriptase inverse.

En d'autres termes, la séquence de l'acide nucléique de i. et la séquence de l'acide nucléique de ii. sont avantageusement :
- dépourvues de cadre ouvert de lecture, qui coderait la transcriptase inverse d'un rétrotransposon Ty de levure, ou plus généralement une transcriptase inverse, et/ou elles
- sont dépourvues de promoteur d'expression d'une telle transcriptase.

Alternativement ou complémentairement, l'acide nucléique de i. et l'acide nucléique de ii. peuvent être dépourvus de séquence codant l'expression d'une intégrase de rétrotransposon Ty de levure, plus généralement de toute séquence codant l'expression d'une intégrase. En d'autres termes, la séquence de l'acide nucléique de i. et la séquence de l'acide nucléique de ii. peuvent être dépourvues de cadre ouvert de lecture, qui code l'intégrase d'un rétrotransposon Ty de levure, ou plus généralement une intégrase, et/ou elles peuvent être dépourvues de promoteur d'expression d'une telle intégrase.

L'acide nucléique de i. et l'acide nucléique de ii. peuvent en outre être dépourvus de séquence codant l'expression d'une protéase de rétrotransposon Ty de levure, plus généralement de toute séquence codant l'expression d'une protéase. En d'autres termes, la séquence de l'acide nucléique de i. et la séquence de l'acide nucléique de ii. peuvent être dépourvues de cadre ouvert de lecture, qui code la protéase d'un rétrotransposon Ty de levure, ou plus généralement une protéase, et/ou elles peuvent être dépourvues de promoteur d'expression d'une telle protéase.

L'acide nucléique de i. et l'acide nucléique de ii. peuvent donc être dépourvus de séquence codant l'expression de la protéine Pol de rétrotransposon Ty de levure, plus généralement de toute séquence codant l'expression d'une protéine Pol.

En d'autres termes, la séquence de l'acide nucléique de i. et la séquence de l'acide nucléique de ii. peuvent être dépourvues de cadre ouvert de lecture, qui code la protéine Pol d'un rétrotransposon Ty de levure, ou plus généralement une protéine Pol, et/ou elles peuvent être dépourvues de promoteur d'expression d'une telle protéine Pol.

Ledit ARN (ARNm ou ARNlnc) d'intérêt peut notamment être à la souche de levure mise en œuvre, voire à l'ensemble des levures.

Ledit ARN (ARNm ou ARNlnc) d'intérêt peut notamment être :
- un ARN (ARNm ou ARNlnc) de procaryote, notamment de bactérie.
- un ARN (ARNm ou ARNlnc) de virus, notamment de *Zika* ou *Influenza*, ou
- un ARN (ARNm ou ARNlnc) d'eucaryote autre que la levure, notamment d'humain, ou
- un ARN artificiel ou non naturel, tel que par exemple un ARN dont la traduction est un polypeptide ou protéine comprenant des épitopes de protéines de différents microorganismes (par exemple des épitopes de différentes bactéries et/ou virus), notamment des épitopes d'antigènes (notamment des épitopes de protéines de différents microorganismes, qui sont antigéniques chez l'être humain),
- un ARN artificiel ou non naturel, tel que par exemple un ARN dont la traduction est un polypeptide ou protéine comprenant différents épitopes d'au moins une protéine d'un même microorganisme (par exemple des épitopes de bactéries et/ou virus), notamment des épitopes d'antigènes (notamment des épitopes de protéines d'un microorganisme, qui sont antigéniques chez l'être humain,
   plus particulièrement
- un ARN (ARNm ou ARNlnc) de virus, notamment de *Zika* ou *Influenza*, ou
- un ARN (ARNm ou ARNlnc) d'eucaryote autre que la levure, notamment d'humain,
- un ARN artificiel ou non naturel, tel que par exemple un ARN dont la traduction est un polypeptide ou protéine comprenant des épitopes de protéines de différents microorganismes (par exemple des épitopes de différentes bactéries et/ou virus), notamment des épitopes d'antigènes (notamment des épitopes de protéines de différents microorganismes, qui sont antigéniques chez l'être humain),
plus particulièrement un ARN (ARNm ou ARNlnc) d'eucaryote autre que la levure, notamment d'humain.

Ledit ARN (ARNm ou ARNlnc) d'intérêt peut comprendre au moins 120 nucléotides, notamment au moins 150 nucléotides, notamment au moins 180 nucléotides, notamment au moins 210 nucléotides, notamment au moins 240 nucléotides, notamment au moins 270 nucléotides, notamment au moins 2300 nucléotides.

Alternativement ou complémentairement, ledit ARN (ARNm ou ARNlnc) à produire peut comprendre moins de 6000 nucléotides, moins de 5800 nucléotides, notamment moins de 5000 nucléotides, notamment moins de 4000 nucléotides, notamment moins de 3000 nucléotides, notamment moins de 2000 nucléotides, notamment moins de 1800 nucléotides.

Toutes les combinaisons de nombre minimal et nombre maximal de nucléotides sont ici explicitement visées. Ledit ARN (ARNm ou ARNlnc) d'intérêt peut par exemple comprendre au moins 150 nucléotides et moins de 2000 nucléotides.

Ledit ARN (ARNm ou ARNlnc) d'intérêt peut notamment coder (c'est-à-dire la traduction protéique de sa séquence peut être celle de) :
- une protéine ou un polypeptide de bactérie,
- une enzyme d'édition génomique (telle que Cas9, transposase),
- une protéine ou un polypeptide de virus,
- un facteur de transcription (d'eucaryote autre que levure, notamment humain),
- un facteur de croissance (d'eucaryote autre que levure, notamment humain),
- une protéine CFTR (*Cystic fibrosis transmembrane conductance regulator*) (humaine),
- un anticorps (polyclonal ou monoclonal), ou fragment d'anticorps (notamment Fv, Fab ou F(ab')2)), notamment un intra-anticorps [*intrabody*] ou un anticorps humanisé, ou
- un polypeptide comprenant au moins un épitope (bactérien ou viral), notamment, plusieurs épitopes (bactériens et/ou viraux) éventuellement séparés par des séquences espaceurs,
   plus particulièrement
- une protéine ou un polypeptide de virus,
- un facteur de transcription (d'eucaryote autre que levure, notamment humain),
- un facteur de croissance (d'eucaryote autre que levure, notamment humain),
- une protéine CFTR (*Cystic fibrosis transmembrane conductance regulator*) (humaine),
- un anticorps (polyclonal ou monoclonal), ou fragment d'anticorps (notamment Fv, Fab ou F(ab')2)), notamment un intra-anticorps [*intrabody*] ou un anticorps humanisé, plus particulièrement
- un facteur de transcription (d'eucaryote autre que levure, notamment humain),
- un facteur de croissance (d'eucaryote autre que levure, notamment humain),
- une protéine CFTR (*Cystic fibrosis transmembrane conductance regulator*) (humaine),
- un anticorps (polyclonal ou monoclonal), ou fragment d'anticorps (notamment Fv, Fab ou F(ab')2)), notamment un intra-anticorps [*intrabody*] ou un anticorps humanisé, plus particulièrement un facteur de transcription (d'eucaryote autre que levure, notamment d'humain) ou un facteur de croissance (d'eucaryote autre que levure, notamment d'humain).

Plusieurs ARN (ARNm ou ARNlnc) différents peuvent être produits dans la même cellule recombinante de levure. Chacun de ces différents ARN a une deuxième séquence nucléotidique qui lui est propre (séquence ADN dont le transcrit ARN comprend la séquence ARN d'intérêt liée à une séquence d'adressage). Chacune de ces deuxièmes séquences nucléotidiques peut être comprise ou portée sur la même molécule d'acide nucléique de ii., ou sur des molécules distinctes d'acide nucléique de ii.

La levure qui a été modifiée génétiquement pour produire les complexes, granules ou particules pseudo-virales (recombinantes) de la demande peut avantageusement présenter des mutations génétiques, notamment chromosomiques, qui sont destinées à stimuler ou accroître la production de ces particules pseudo-virales.

Il peut par exemple s'agir d'une ou plusieurs mutations de gènes de levure, qui comprennent une ou plusieurs mutations choisies parmi :
- le remplacement d'un ou plusieurs codons chacun par un autre codon ou par un autre triplet de nucléotides, et
- la délétion d'un ou plusieurs nucléotides.

Les gènes de levure peuvent par exemple être un ou plusieurs gènes parmi les gènes *Rpbl, Spt21*, *Srb2* et *Srb5.*

Par exemple, le gène *Rpbl* peut comprendre une substitution de codons qui confère la mutation d'acide aminé C67Y, C70Y ou H80Y. Par exemple, le gène *Rpbl* de la souche peut être remplacé par le gène Rpbl d'une autre souche de levure, notamment d'une autre souche de levure qui appartient au même genre de levure, et qui porte au moins une de ces substitutions de codons. Par exemple, le gène *Rpbl* de *S. paradoxus* peut être remplacé par le gène *Rpbl* de *S. cerevisiae,* dans lequel on a introduit une ou plusieurs des mutations de codons qui confèrent les mutations d'acide aminé C67Y, C70Y et H80Y.

Par exemple, un ou plusieurs des gènes *Spt21*, *Srb2* et *Srb5* peuvent être (au moins partiellement) délétés [Δ*Spt21*, Δ*Srb2* et Δ*Srb5*].

Par exemple, la levure peut être une levure dont :
- le gène *Rpbl* comprend le remplacement de codon(s) par un(des) codon(s) qui confère(nt) l'une (ou plusieurs) des mutations d'acide aminé C67Y, C70Y et H80Y (ou a été remplacé par le gène *Rpbl* d'une autre souche de levure, dans lequel on a opéré au moins l'un de ces remplacements), et dont
- un ou plusieurs des gènes *Spt21*, *Srb2* et *Srb5* sont délétés (au moins partiellement délétés, à tout le moins suffisamment délétés pour ne plus produire de protéine).

Par exemple, la levure peut être une souche de *S. paradoxus* (qui est naturellement dépourvue de Ty, notamment de Ty1 (souche Ty0)), qui est :
- *Rpbl* C67Y,
- *Rpbl* C70Y,
- *Rpbl* H80Y,
- Δ*Srb2*,
- Δ*Spt21*,
- Δ*Srb2* et Δ*Spt21*,
- *Rpbl* C67Y Δ*Srb2*,
- *Rpbl* C70Y Δ*Srb2*,
- Rpbl H80Y Δ*Srb2*,
- Rpbl C67Y Δ*Spt21*,
- Rpbl C70Y Δ*Spt21*,
- Rpbl H80Y Δ*Spt21*,
- Rpb1 C67Y Δ*Srb2* Δ*Spt21*,
- Rpb1 C70Y Δ*Srb2* Δ*Spt21*, ou
- Rpb1 H80Y Δ*Srb2* Δ*Spt21*.

Il peut par exemple s'agir d'une souche de *S. cerevisiae* (qui a été modifiée génétiquement pour ne pas produire de rétrosomes T (souche Ty0), et) qui est (en outre) modifiée génétiquement pour être :
- *Rpbl* C67Y,
- Rpbl C70Y,
- *Rpbl* H80Y,
- Δ*Srb2*,
- Δ*Spt21*,
- Δ*Srb2* Δ*Spt21*,
- C67Y Δ*Srb2*,
- C70Y Δ*Srb2*,
- *Rpbl* H80Y Δ*Srb2*,
- *Rpbl* C67Y Δ*Spt21*,
- *Rpbl* C70Y Δ*Spt21*,
- *Rpbl* H80Y Δ*Spt21*,
- *Rpbl* C67Y Δ*Srb2* Δ*Spt21*,
- *Rpbl* C70Y Δ*Srb2* Δ*Spt21*, ou
- *Rpbl* H80Y Δ*Srb2* Δ*Spt21.*

Alternativement ou complémentairement aux mutations génétiques, notamment chromosomiques, qui sont destinées à stimuler ou accroître la production de ces particules pseudo-virales, un ou des produits peuvent être utilisés pour stimuler ou accroître cette production. Ce ou ces produits peuvent notamment être présents dans le milieu de transfection ou de culture dans lequel la levure est placée.

Les cellules (recombinantes) de levure qui ont été modifiées génétiquement pour notamment comprendre les acides nucléiques de i. et de ii. peuvent être placées dans des conditions permettant à l'acide nucléique de i. (à la première séquence nucléotidique) d'exprimer la protéine Gag qu'il code, et à l'acide nucléique de ii. (à la deuxième séquence nucléotidique) de transcrire l'ARN qu'il code.

Elles peuvent notamment être placées sur ou dans un milieu de culture adapté à la croissance ou multiplication de levure (*cf*. ci-dessous). Si la première séquence nucléotidique et/ou la deuxième séquence nucléotidique portent des promoteurs inductibles, ce milieu peut notamment comprendre tout composé ou produit qui serait nécessaire à l'induction du ou de ces promoteurs.

Les complexes, granules, ou particules pseudo-virales produits par les cellules (recombinantes) de levure sont collectés, par exemple par lyse des cellules, notamment par lyse chimique ou mécanique (par billes, plutôt que par sonication), et les ARN contenus dans ces complexes, granules ou particules pseudo-virales, et notamment les ARNm ou ARNlnc ainsi contenus, sont purifiés.

Alternativement, les ARN contenus dans ces complexes, granules ou particules pseudo-virales (recombinants), et notamment les ARNm ou ARNlnc qui y sont contenus, sont directement extraits ou purifiés des cellules de levure, sans extraction ou collecte préalable des complexes, granules ou particules pseudo-virales qui les contiennent.

De grandes quantités d'ARN (ARNm ou ARNlnc) peuvent ainsi être produites, par exemple au moins 0,9 gramme d'ARN (ARNm ou ARNlnc) par litre de culture de levure.

Avantageusement, l'ARN, qui est présent ou produit dans le complexe, granule, particule pseudo-virale de la demande, comprend des modifications post-transcriptionnelles qui le rendent compétent pour une traduction dans des cellules de mammifère. Avantageusement, l'ARN du complexe, granule, particule pseudo-virale de la demande peut être muni d'une queue polyA à l'extrémité 3'.

Avantageusement, l'ARN du complexe, granule, particule pseudo-virale de la demande peut être muni d'une coiffe à l'extrémité 5', plus particulièrement d'une coiffe comme connue de la personne du métier, plus particulièrement d'une coiffe qui comprend une 7-méthylguanosine (liée à l'ARNm par trois phosphates en configuration 5'-5'). Cette coiffe peut bloquer l'action des exonucléases et peut être nécessaire pour stimuler la (future) traduction de l'ARN d'intérêt (ARNm ou ARNlnc) dans les cellules de mammifères.

Avantageusement, l'ARN du complexe, granule, particule pseudo-virale de la demande peut être méthylé, notamment au niveau d'un ou plusieurs de ses résidus adénine et/ou cytosine et/ou pseudouridine, notamment au niveau d'un ou plusieurs de ses résidus adénine et/ou cytosine.

Avantageusement, l'ARN, qui est présent ou produit dans le complexe, granule, particule pseudo-virale de la demande, comprend des modifications post-transcriptionnelles type méthylation de résidu(s) cytosine et/ou adénine et/ou pseudouridine, plus particulièrement de résidu(s) cytosine et/ou adénine qui augmentent son efficacité de traduction dans des cellules de mammifère.

Les moyens de la demande sont donc particulièrement adaptés à la production d'ARN (ARNm ou ARNlnc) qui est destiné à une administration dans un eucaryote, notamment un eucaryote pluricellulaire, notamment un mammifère, notamment un humain.

Plusieurs exemples de souches de levure conformes à la demande ont été déposés auprès de la CNCM (*cf*. ci-dessous).

La demande est également relative à chacun des moyens mis en œuvre ou produits par la méthode de production d'ARN ici décrite, ainsi pour qu'à leur utilisation la production d'ARNm ou ARNlnc présentant une queue polyA en 3' et/ou une coiffe 7-méthylguanosine en 5' (voire une méthylation de résidu(s) adénine et/ou cytosine et/ou pseudouridine, notamment de résidu(s) adénine et/ou cytosine).

La demande est ainsi relative à l'acide nucléique de i. tel qu'ici décrit.

La demande est également relative à l'acide nucléique de ii. tel qu'ici décrit.

La demande est ainsi relative à l'association de l'acide nucléique de i. et de l'acide nucléique de ii. tels qu'ici décrits, et à leur association pour une utilisation simultanée, séparée ou différée dans le temps, plus particulièrement pour leur utilisation simultanée, séparée ou différée dans le temps dans la transfection ou transformation de cellules de levures, plus particulièrement de cellules de levure telles qu'ci décrite.

Plus particulièrement, la demande est relative à un kit qui comprend un acide nucléique i. et/ou, de préférence et, un acide nucléique ii. tels qu'ici décrits. Ce kit est spécialement adapté à la production recombinante d'ARN, notamment d'ARNm ou d'ARNlnc, tels qu'ici décrits.

Avantageusement, le kit ne comprend pas de protéine ou acide nucléique permettant la rétrotransposition de l'ARNm ou ARNlnc à produire, plus particulièrement sa rétrotransposition dans une cellule de levure.

Avantageusement, le kit ne comprend pas de transcriptase inverse de rétrotransposon Ty de levure, ni d'acide nucléique codant une telle transcriptase inverse.

Alternativement ou complémentairement, le kit ne comprend pas d'intégrase de rétrotransposon Ty de levure, ni d'acide nucléique codant une telle intégrase. Avantageusement, le kit ne comprend pas de protéase de rétrotransposon Ty de levure, ni d'acide nucléique codant une telle protéase.

Plus généralement, le kit peut ne pas comprendre de transcriptase inverse, ni d'acide nucléique codant une transcriptase inverse.

Plus généralement, le kit peut ne pas comprendre d'intégrase, ni d'acide nucléique codant une intégrase.

Plus généralement, le kit peut ne pas comprendre de protéase, ni d'acide nucléique codant une protéase.

La demande est ainsi relative à un kit (qui est spécialement adapté à la mise en œuvre du procédé de production recombinante d'ARN de la demande), qui comprend :
i. au moins un acide nucléique comprenant une première séquence nucléotidique et/ou, de préférence et,
ii. au moins un acide nucléique comprenant une deuxième séquence nucléotidique,
tels qu'ici décrits, plus particulièrement pour une utilisation simultanée, différée ou séparée dans le temps.

Plus particulièrement, l'acide nucléique de i. et/ou (de préférence et) l'acide nucléique de ii. du kit peuvent être définis comme suit :
- la première séquence nucléotidique comprend une séquence ADN qui code l'expression de la protéine Gag d'un rétrotransposon Ty de levure,
- la protéine Gag de rétrotransposon Ty de levure est une protéine Gag de rétrotransposon Ty1, Ty2 ou Ty3 de levure (Ty de levure, notamment Ty1, Ty2 ou Ty3 de levure, notamment Ty1, Ty2, Ty3, Ty4 ou Ty5 de levure), et la séquence de cette protéine Gag est la séquence de SEQ ID NO : 3 ou une séquence qui est identique à au moins 90% à la séquence de SEQ ID NO : 3,
- la deuxième séquence nucléotidique comprend une séquence ADN qui comprend la séquence de SEQ ID NO : 16 ou un fragment d'au moins 150 nucléotides de la séquence de SEQ ID NO : 16, et avantageusement ne comprend pas de cadre de lecture dont le premier codon serait le codon départ de traduction (ATG) (ou plus généralement, ne comprend pas de cadre de lecture qui, dans la levure, serait un cadre ouvert de lecture)
- l'acide nucléique qui comprend la deuxième séquence nucléotidique ne comprend pas de séquence ADN dont le transcrit ARN serait la séquence ARN complète de ladite protéine Gag de rétrotransposon Ty de levure, et
- l'acide nucléique qui comprend la première séquence nucléotidique et l'acide nucléique qui comprend la deuxième séquence nucléotidique sont une seule et même molécule, ou bien deux molécules distinctes ou séparées.

Le kit peut ne pas comprendre de transcriptase inverse de rétrotransposon Ty de levure, ni d'acide nucléique codant une telle transcriptase inverse, ni d'acide nucléique comprenant une séquence codant une telle transcriptase inverse.

Un kit de la demande peut en outre comprendre une ou des cellules d'une levure telle qu'ici décrite, plus particulièrement une ou des cellules d'une levure :
- qui est dépourvue de rétrotransposon Ty1 ou qui est naturellement pourvue d'une séquence de rétrotransposon Ty1 mais dont la séquence de rétrotransposon Ty1 a été modifiée génétiquement pour ne pas produire de rétrosome T, ou plus généralement d'une levure
- qui est dépourvue de rétrotransposon Ty ou qui a été modifiée génétiquement pour ne pas produire de rétrosome T.

La ou les cellules de levure du kit peuvent avantageusement ne comprendre pas de protéine qui permettrait la rétrotransposition de l'ARN (ARNm ou ARNlnc) à produire (transcriptase inverse, notamment), ou d'acide nucléique qui coderait une telle protéine. Avantageusement, la ou les cellules de levure du kit ne comprennent pas de transcriptase inverse de rétrotransposon Ty de levure, ni d'acide nucléique codant une telle transcriptase inverse.

Alternativement ou complémentairement, la ou les cellules de levure du kit ne comprennent pas d'intégrase de rétrotransposon Ty de levure, ni d'acide nucléique codant une telle intégrase.

Avantageusement, la ou les cellules de levure du kit ne comprennent pas de protéase de rétrotransposon Ty de levure, ni d'acide nucléique codant une telle protéase.

Plus généralement, la ou les cellules de levure du kit peuvent ne pas comprendre de transcriptase inverse, ni d'acide nucléique codant une transcriptase inverse.

Plus généralement, la ou les cellules de levure du kit peuvent ne pas comprendre d'intégrase, ni d'acide nucléique codant une intégrase.

Plus généralement, la ou les cellules de levure du kit peuvent ne pas comprendre de protéase, ni d'acide nucléique codant une protéase.

Bien entendu, la levure du kit peut comprendre une ou plusieurs copies d'au moins l'un de l'acide nucléique de i. et l'acide nucléique de ii.

Un kit de la demande peut par exemple comprendre :
- une ou des cellules d'une levure telle qu'ici décrite, ce(s) cellule(s) comprenant la première séquence nucléotidique (intégrée) dans ses chromosomes ; et
   sous forme distincte ou séparée,
- l'acide nucléique de ii. (ou la deuxième séquence nucléotidique), par exemple sous la forme d'un vecteur non intégratif, plus particulièrement d'un vecteur réplicatif.

Le kit peut en outre comprendre une notice contenant des instructions pour son utilisation dans la transfection ou transformation de cellules de levures, et/ou pour son utilisation dans la production (recombinante) d'ARN, plus particulièrement d'ARNm ou ARNlnc, tels qu'ici décrits, notamment pour cette production dans des cellules de levure.

Le kit peut notamment comprendre une notice contenant des instructions pour modifier génétiquement des cellules d'une levure, notamment d'une levure telle qu'ici décrite (ou, le cas échéant, telle que contenue dans le kit), ces modifications génétiques comprenant la transfection ou transformation de ces cellules de levure par les acides nucléiques i. et ii. du kit.

Le kit peut avantageusement être utilisé pour la production d'ARNm ou ARNlnc présentant une queue polyA en 3' et/ou une coiffe 7-méthylguanosine en 5' (voire une méthylation de résidu(s) de résidu(s) adénine et/ou cytosine et/ou pseudouridine, notamment de résidu(s) adénine et/ou cytosine).

La demande est également relative à une cellule de bactérie qui a été modifiée génétiquement pour comprendre l'acide nucléique de i. et/ou, de préférence et, l'acide nucléique de ii. tels qu'ici décrits, notamment pour les comprendre dans son cytoplasme. La demande est également relative à une pluralité de telles cellules de bactéries, et notamment à une souche de bactérie dont les cellules comprennent ou sont de telles cellules. La bactérie peut par exemple être *Escherichia*, notamment *E. coli,* par exemple la bactérie *E. coli* DH5a, qui est accessible auprès de l'ATCC sous le numéro 67877^{™}.

La ou les cellules de bactérie peuvent avantageusement être utilisées pour amplifier les acides nucléiques de i. et/ou de ii., par exemple avant de les transférer en cellule de levure.

La demande est également relative à un milieu de culture de microorganismes, notamment à un milieu artificiel de culture de bactéries, qui comprend au moins une cellule de bactérie de la demande ou au moins une souche de bactérie de la demande.

Ce milieu peut par exemple être sous forme liquide ou solide.

La composition de ce milieu de culture peut être celle d'un milieu de culture adapté à la croissance ou multiplication bactérienne. Elle notamment comprendre un ou plusieurs éléments parmi la tryptone, l'extrait de levure et NaCl, plus particulièrement au moins de l'extrait de levure, plus particulièrement au moins de l'extrait de levure et de la tryptone. Par exemple, la composition de ce milieu de culture peut notamment être ou comprendre celle d'un milieu SOB (*Super Optimal Broth*), d'un milieu SOC (*Super Optimal broth with Catabolite repression*), ou d'un milieu LB (*Lysogeny Broth*). Ce type de composition de milieu de culture est bien connu de la personne du métier.

Un milieu SOB peut par exemple comprendre de la tryptone et de l'extrait de levure, par exemple : 2% w/v tryptone, 0.5% w/v d'extrait de levure, 8,56 à 10 mM NaCl, 2.5mM KCl, 10mM MgCl₂, 10mM MgSO₄, H₂O qsp 1000 mL.

Un milieu SOC peut ne différer d'un milieu SOB que par l'ajout d'un sucre, par exemple du glucose, par exemple glucose 20mM.

Un milieu LB peut par exemple comprendre de la tryptone, de l'extrait de levure et du NaCl.

Le milieu de culture peut avantageusement être utilisé pour la production d'ARNm ou ARNlnc présentant une queue polyA en 3' et/ou une coiffe 7-méthylguanosine en 5' (voire une méthylation de adénine et/ou cytosine et/ou pseudouridine, notamment de résidu(s) adénine et/ou cytosine).

La demande est également relative à une cellule de levure qui a été modifiée génétiquement pour comprendre l'acide nucléique de i. et/ou, de préférence et, l'acide nucléique de ii. tels qu'ici décrits, notamment pour comprendre ces acides nucléiques dans son noyau (y compris ses chromosomes) et/ou son cytoplasme.

La demande est ainsi relative à une cellule de levure (recombinante) (qui est spécialement adaptée à la mise en œuvre du procédé de production recombinante d'ARN de la demande), et qui est caractérisée en ce que :
- la cellule de levure est dépourvue de rétrotransposon Ty1, Ty2 et Ty3 (Tyl, plus particulièrement Ty1, Ty2 et Ty3, plus particulièrement de rétrotransposons Ty1, Ty2, Ty3, Ty4 et Ty5 de levure, plus particulièrement de tout rétrotransposon de levure), et
- la cellule de levure ne comprend pas de transcriptase inverse de rétrotransposon Ty de levure, ni d'acide nucléique codant une telle transcriptase inverse, ni d'acide nucléique comprenant une séquence codant une telle transcriptase inverse, ni d'acide nucléique comprenant une séquence codant une telle transcriptase inverse,
- la cellule de levure a été génétiquement modifiée pour comprendre :
   i. au moins un acide nucléique comprenant une première séquence nucléotidique et/ou, de préférence et,
   ii. au moins un acide nucléique comprenant une deuxième séquence nucléotidique, tels qu'ici décrits.

Plus particulièrement, dans la cellule de levure, l'acide nucléique de i. et/ou l'acide nucléique de ii. peuvent être définis comme suit :
- la première séquence nucléotidique comprend une séquence ADN qui code l'expression de la protéine Gag d'un rétrotransposon Ty de levure,
- la protéine Gag de rétrotransposon Ty de levure est une protéine Gag de rétrotransposon Ty1, Ty2 ou Ty3 de levure (Ty de levure, notamment Ty1, Ty2 ou Ty3 de levure, notamment Ty1, Ty2, Ty3, Ty4 ou Ty5 de levure), et la séquence de cette protéine Gag est la séquence de SEQ ID NO : 3 ou une séquence qui est identique à au moins 90% à la séquence de SEQ ID NO : 3,
- la deuxième séquence nucléotidique comprend une séquence ADN dont le transcrit ARN comprend la séquence de SEQ ID NO : 16 ou un fragment d'au moins 150 nucléotides de la séquence de SEQ ID NO : 16, et avantageusement ne comprend pas de cadre de lecture dont le premier codon serait le codon départ de traduction (ATG) (ou plus généralement, ne comprend pas de cadre de lecture qui, dans la levure, serait un cadre ouvert de lecture),
- l'acide nucléique qui comprend la deuxième séquence nucléotidique ne comprend pas de séquence ADN dont le transcrit ARN serait la séquence ARN complète de ladite protéine Gag de rétrotransposon Ty de levure, et
- l'acide nucléique qui comprend la première séquence nucléotidique et l'acide nucléique qui comprend la deuxième séquence nucléotidique sont une seule et même molécule, ou bien deux molécules distinctes ou séparées.

Par exemple, la cellule de levure peut comprendre la première séquence nucléotidique, plus particulièrement la première séquence nucléotidique intégrée dans ses chromosomes. Cette cellule de levure peut en outre comprendre l'acide nucléique de i. dans son noyau et/ou cytoplasme, sous forme non intégrée à ses chromosomes, par exemple sous la forme d'un plasmide réplicatif.

Avantageusement, cette cellule de levure est une cellule d'une levure :
- qui est (naturellement) dépourvue de rétrotransposon Ty1, ou qui est naturellement pourvue d'une séquence de rétrotransposon Ty1 mais dont la séquence de rétrotransposon Ty1 a été modifiée génétiquement pour ne pas produire de rétrosome T, ou plus généralement
- qui est (naturellement) dépourvue de rétrotransposon Ty ou qui a été modifiée génétiquement pour ne pas produire de rétrosome T.

La cellule de levure peut avantageusement ne pas comprendre de protéine qui permettrait la rétrotransposition de l'ARNm ou ARNlnc à produire (transcriptase inverse, notamment), ou d'acide nucléique qui coderait une telle protéine.

Avantageusement, la cellule de levure ne comprend pas de transcriptase inverse de rétrotransposon Ty de levure, ni d'acide nucléique codant une telle transcriptase inverse. Alternativement ou complémentairement, la cellule de levure ne comprend pas d'intégrase de rétrotransposon Ty de levure, ni d'acide nucléique codant une telle intégrase. Avantageusement, la cellule de levure ne comprend pas de protéase de rétrotransposon Ty de levure, ni d'acide nucléique codant une telle protéase.

Plus généralement, la cellule de levure peut ne pas comprendre de transcriptase inverse, ni d'acide nucléique codant une transcriptase inverse.

Plus généralement, la cellule de levure peut ne pas comprendre d'intégrase, ni d'acide nucléique codant une intégrase.

Plus généralement, la cellule de levure peut ne pas comprendre de protéase, ni d'acide nucléique codant une protéase.

La ou les cellules de levure peuvent avantageusement être utilisées pour la production d'ARNm ou ARNlnc présentant une queue polyA en 3' et/ou une coiffe 7-méthylguanosine en 5' (voire une méthylation de résidu(s) cytosine et/ou adénine et/ou pseudouridine, plus particulièrement de résidu(s) cytosine et/ou adénine).

La demande est également relative à une pluralité de telles cellules de levure, et notamment à une souche de levure dont les cellules comprennent ou sont de telles cellules. La cellule ou souche de levure de la demande est spécialement adaptée à la production (recombinante) d'ARN, notamment d'ARN hétérologue à cette levure, plus particulièrement d'ARNm ou ARNlnc hétérologue à cette levure.

Une souche de levure de la demande peut notamment être la souche I-5171 qui a été déposée auprès de la CNCM en vertu du Traité de Budapest le 24 février 2017 (souche « TB16 »).

Cette levure est une souche de *S. paradoxus*, dont le gène *Rpbl* a été remplacé par une version mutée du gène *Rpbl* de *S. cerevisiae* (à savoir une version C67Y du gène *Rpbl* de *S. cerevisiae),* et dans laquelle a été transféré un acide nucléique de i. Cet acide nucléique de i. est intégré au génome de la levure, et comprend une séquence codant pour la protéine de fusion Gag-eGFP (Gag de Ty1, en l'occurrence Gag de SEQ ID NO : 3 ; eGFP de SEQ ID NO : 27) sous l'influence d'un promoteur galactose (Gal) [milieu sélectif URA].

Une souche de levure de la demande peut notamment être la souche I-5172 qui a été déposée auprès de la CNCM en vertu du Traité de Budapest le 24 février 2017 (souche « TB17 »).

Cette levure est une souche de *S. paradoxus*, dont le gène *Rpbl* a été remplacé par une version mutée du gène *Rpbl* de *S. cerevisiae* (à savoir une version C70Y du gène *Rpbl* de *S. cerevisiae),* et dans laquelle a été transféré un acide nucléique de i. Cet acide nucléique de i. est intégré au génome de la levure, et comprend une séquence codant pour la protéine de fusion Gag-eGFP (Gag de Ty1, en l'occurrence Gag de SEQ ID NO : 3 ; eGFP de SEQ ID NO : 27) sous l'influence d'un promoteur galactose (Gal) [milieu sélectif URA].

Une souche de levure de la demande peut notamment être la souche I-5173 qui a été déposée auprès de la CNCM en vertu du Traité de Budapest le 24 février 2017 (souche « TB18 »).

Cette levure est une souche de *S. paradoxus*, dont le gène *Rpbl* a été remplacé par une version mutée du gène *Rpbl* de *S. cerevisiae* (à savoir une version H80Y du gène *Rpbl* de *S. cerevisiae),* et dans laquelle a été transféré un acide nucléique de i. Cet acide nucléique de i. est intégré au génome de la levure, et comprend une séquence codant pour la protéine de fusion Gag-eGFP (Gag de Ty1, en l'occurrence Gag de SEQ ID NO : 3 ; eGFP de SEQ ID NO : 27) sous l'influence d'un promoteur galactose (Gal) [milieu sélectif URA].

Une souche de levure de la demande peut notamment être la souche I-5174 qui a été déposée auprès de la CNCM en vertu du Traité de Budapest le 24 février 2017 (souche « TB21 »).

Cette levure est une souche de *S. paradoxus*, dont le gène *Srb2* a été délété, dont le gène *Rpbl* a été remplacé par une version mutée du gène *Rpbl* de *S. cerevisiae* (à savoir une version H80Y du gène *Rpbl* de *S. cerevisiae),* et dans laquelle a été transféré un acide nucléique de i. Cet acide nucléique de i. est intégré au génome de la levure, et comprend une séquence codant pour la protéine de fusion Gag-eGFP (Gag de Ty1, en l'occurrence Gag de SEQ ID NO : 3 ; eGFP de SEQ ID NO : 27) sous l'influence d'un promoteur galactose (Gal) [milieu sélectif URA].

Une souche de levure de la demande peut notamment être la souche I-5175 qui a été déposée auprès de la CNCM en vertu du Traité de Budapest le 24 février 2017 (souche « TB32 »).

Cette levure est une souche de *S. paradoxus*, dont le gène *Srb2* a été délété, et dans laquelle a été transféré un acide nucléique de i. Cet acide nucléique de i. est intégré au génome de la levure, et comprend une séquence codant pour la protéine de fusion Gag-eGFP-6His-3MS2 (Gag de Ty1, en l'occurrence Gag de SEQ ID NO : 3 ; eGFP de SEQ ID NO : 27 ; 6His de SEQ ID NO : 6 ; 3MS2 = 3 copies de la séquence du bactériophage MS2, à titre d'étiquette de purification) sous l'influence d'un promoteur galactose (Gal) [milieu sélectif URA].

Une souche de levure de la demande peut notamment être la souche I-5176 qui a été déposée auprès de la CNCM en vertu du Traité de Budapest le 24 février 2017 (souche « TBSc1 »).

Cette levure est une souche de *S. cerevisiae,* qui est vierge d'éléments Ty (Ty0), et qui a été transformée par un plasmide épisomique contenant un acide nucléique de i., en l'occurrence un acide nucléique permettant l'expression d'une protéine de fusion Gag-eGFP (Gag de SEQ ID NO : 3 ; eGFP de SEQ ID NO : 27) sous l'influence d'un promoteur galactose (Gal) [milieu sélectif URA].

La CNCM est la Collection Nationale de Culture de Microorganismes (Institut Pasteur ; 28, rue du Dr Roux ; 75724 Paris Cedex 15 ; France).

Une souche de levure de la demande peut également être une des souches I-5171 à I-5176, dans laquelle un acide nucléique de ii. a été transféré, par exemple sous la forme d'un plasmide, notamment d'un plasmide non intégratif, plus particulièrement d'un plasmide réplicatif.

Une souche de levure de la demande peut notamment être la souche I-5293 qui a été déposée auprès de la CNCM en vertu du Traité de Budapest le 15 mars 2018 (souche « TB53 »).

Cette levure est une souche *S. paradoxus* dont le gène *Srb2* a été délété, et dans laquelle ont été transférés un acide nucléique de i. et ii.. L'acide nucléique de i. est intégré au génome de la levure, et comprend une séquence codant pour la protéine de fusion Gag-eGFP (Gag de Ty1, en l'occurrence Gag de SEQ ID NO : 3 ; eGFP de SEQ ID NO : 27) sous l'influence d'un promoteur galactose (Gal) [milieu sélectif URA]. L'acide nucléique ii., quant à lui, correspond à un plasmide non intégratif permettant l'expression d'un ARN modèle Luciférase (SEQ ID NO : 29) disposant en 3' d'une séquence de ciblage complète mutée (GAGm = SEQ ID NO : 14) de l'ARN dans les *T-bodies* sous l'influence d'un promoteur galactose (GAL) [Milieu sélectif LEU].

Une souche de levure de la demande peut notamment être la souche I-5294 qui a été déposée auprès de la CNCM en vertu du Traité de Budapest le 15 mars 2018 (souche « TB54 »).

Cette levure est une souche *S. paradoxus* dont le gène *Srb2* a été délété, et dans laquelle ont été transférés un acide nucléique de i. et ii.. L'acide nucléique de i. est intégré au génome de la levure, et comprend une séquence codant pour la protéine de fusion Gag-eGFP (Gag de Ty1, en l'occurrence Gag de SEQ ID NO : 3 ; eGFP de SEQ ID NO : 27) sous l'influence d'un promoteur galactose (Gal) [milieu sélectif URA]. L'acide nucléique ii., quant à lui, correspond à un plasmide non intégratif permettant l'expression d'un ARN modèle Luciférase (SEQ ID NO : 29) disposant d'une séquence de ciblage partielle (ou minimale) (SEQ ID NO : 18) de l'ARN dans les *T-bodies* sous l'influence d'un promoteur galactose (GAL) [Milieu sélectif LEU].

La CNCM est la Collection Nationale de Culture de Microorganismes (Institut Pasteur ; 28, rue du Dr Roux ; 75724 Paris Cedex 15 ; France).

Une cellule de la demande peut notamment être une cellule de l'une de ces souches.

La ou les cellules de levure peuvent avantageusement être utilisées pour la production d'ARNm ou ARNlnc présentant une queue polyA en 3' et/ou une coiffe 7-méthylguanosine en 5' (voire une méthylation de résidu(s) adénine et/ou cytosine et/ou pseudouridine, notamment de résidu(s) adénine et/ou cytosine).

La demande est également relative à un milieu de culture de microorganismes, notamment à un milieu artificiel de culture de microorganismes, qui comprend au moins une cellule de levure de la demande ou au moins une souche de levure de la demande. Ce milieu peut par exemple être sous forme liquide.

La composition de ce milieu de culture peut être celle d'un milieu adapté à la croissance ou multiplication de cellules de levure. Elle peut notamment comprendre un ou plusieurs éléments parmi l'extrait de levure, le glucose, la peptone et le NaCl, plus particulièrement au moins de l'extrait de levure et/ou du glucose, plus particulièrement au moins de l'extrait de levure.

Ce milieu peut notamment comprendre en outre un composé ou produit qui serait nécessaire à l'induction de promoteur(s).

Le milieu de culture peut avantageusement être utilisée pour la production d'ARNm ou ARNlnc présentant une queue polyA en 3' et/ou une coiffe 7-méthylguanosine en 5' (voire une méthylation de résidu(s) adénine et/ou cytosine et/ou pseudouridine, notamment de résidu(s) adénine et/ou cytosine).

La demande est également relative à un milieu de transfection ou de transformation de microorganismes, notamment de levure, plus particulièrement à un milieu artificiel de transfection ou de transformation de microorganismes, notamment de levure. Le milieu de transfection ou de transformation de la demande comprend au moins une cellule de levure de la demande ou au moins une souche de levure de la demande. Ce milieu peut être sous forme liquide, notamment sous forme de suspension.

La composition de ce milieu de transfection ou transformation peut notamment comprendre du polyéthylène glycol (PEG), notamment du PEG 50, et peut en outre optionnellement comprendre de l'ADN de saumon dénaturé et/ou de l'acétate de lithium. Le milieu de transfection ou de transformation peut avantageusement être utilisé pour la production d'ARNm ou ARNlnc présentant une queue polyA en 3' et/ou une coiffe 7-méthylguanosine en 5' (voire une méthylation de résidu(s) adénine et/ou cytosine et/ou pseudouridine, notamment de résidu(s) adénine et/ou cytosine).

La demande est également relative à un complexe, un granule, une particule ou une particule pseudo-virale (*virus-like particle* ou *VLP*) (recombinants). Ce complexe, granule, particule pseudo-virale (recombinant) est susceptible d'être produit lors de la mise en œuvre de la méthode de production (recombinante) d'ARN de la demande (et par collecte du complexe, granule, particule pseudo-virale ainsi produit).

Ce complexe, granule, particule pseudo-virale comprend un ARN complexé à, ou encapsulé par, un polymère protéique, dans lequel le polymère protéique comprend la protéine Gag d'un rétrotransposon Ty de levure.

La séquence nucléotidique de l'ARN comprend ladite séquence d'adressage (« séquence b. ») et/ou la séquence d'une molécule d'un ARN (notamment d'un ARNm ou ARNlnc) d'intérêt (« séquence a. »), plus particulièrement ladite séquence d'adressage liée à la la séquence d'une molécule d'un ARN (notamment d'un ARNm ou ARNlnc) d'intérêt.

La demande est ainsi relative à une particule pseudo-virale (recombinante) (qui est susceptible d'être obtenue par collecte d'une particule pseudo-virale produite lors de la mise en œuvre du procédé de production recombinante d'ARN de la demande), et qui est caractérisée en ce que la particule pseudo-virale comprend un ARN encapsulé par un polymère protéique, dans laquelle :
- le polymère protéique comprend la protéine Gag d'un rétrotransposon Ty de levure,
- la protéine Gag de rétrotransposon Ty de levure est une protéine Gag de rétrotransposon Ty1, Ty2 ou Ty3 de levure, et la séquence de cette protéine Gag est la séquence de SEQ ID NO : 3 ou une séquence qui est identique à au moins 90% à la séquence de SEQ ID NO : 3,
- la séquence nucléotidique de l'ARN encapsulé comprend la séquence de SEQ ID NO : 16 ou un fragment d'au moins 150 nucléotides de la séquence de SEQ ID NO : 16 (mais de préférence ne comprend pas de cadre de lecture dont le premier codon serait le codon départ de traduction *[start]* (AUG)), et ne comprend pas la séquence ARN qui est le transcrit ARN de la SEQ ID NO : 2, et
- la particule pseudo-virale ne comprend ni transcriptase inverse, ni acide nucléique codant une transcriptase inverse, ni acide nucléique comprenant une séquence codant une telle transcriptase inverse.

Avantageusement, l'ARN du complexe, granule, particule pseudo-virale de la demande, plus particulièrement l'ARN d'intérêt, notamment l'ARNm ou ARNlnc d'intérêt, (« séquence a. »), peut être muni d'une queue polyA en position 3' (*cf*. Figures 7A, 7B et 7C).

Avantageusement, l'ARN du complexe, granule, particule pseudo-virale de la demande, plus particulièrement l'ARN, notamment l'ARNm ou ARNlnc, (« séquence a. »), peut être muni d'une coiffe en position 5', plus particulièrement à son extrémité 5' (*cf*. Figures 7A, 7B et 7C), notamment d'une coiffe qui comprend une 7-méthylguanosine (liée à l'ARNm par trois phosphate). Cette coiffe peut bloquer l'action des exonucléases, et peut être nécessaire pour stimuler la (future) traduction dudit ARN dans les cellules de mammifères.

Avantageusement, l'ARN du complexe, granule, particule pseudo-virale de la demande, plus particulièrement l'ARN d'intérêt, notamment l'ARNm ou ARNlnc, (« séquence a. »), peut être méthylé, notamment au niveau d'un ou plusieurs de ses résidus adénine et/ou cytosine et/ou pseudouridine, notamment résidu(s) adénine et/ou cytosine.

Ce complexe, granule, particule pseudo-virale (et notamment le polymère protéique de ce complexe, granule, particule pseudo-virale) peut ne pas comprendre de protéine qui permettrait la rétrotransposition de l'ARN (ARNm ou ARNlnc) d'intérêt (transcriptase inverse, notamment), ou d'acide nucléique qui coderait une telle protéine.

Ce complexe, granule, particule pseudo-virale (et notamment le polymère protéique de ce complexe, granule, particule pseudo-virale) peut ne pas comprendre de transcriptase inverse de rétrotransposon Ty de levure, ni d'acide nucléique codant une telle transcriptase inverse.

Ce complexe, granule, particule pseudo-virale (et notamment le polymère protéique de ce complexe, granule, particule pseudo-virale) peut ne pas comprendre d'intégrase de rétrotransposon Ty de levure, ni d'acide nucléique codant une telle intégrase.

Ce complexe, granule, particule pseudo-virale (et notamment le polymère protéique de ce complexe, granule, particule pseudo-virale) peut ne pas comprendre de protéase de rétrotransposon Ty de levure, ni d'acide nucléique codant une telle protéase.

Plus généralement, ce complexe, granule, particule pseudo-virale (et notamment le polymère protéique de ce complexe, granule, particule pseudo-virale) peut ne pas comprendre de transcriptase inverse, ni d'acide nucléique codant une transcriptase inverse. Plus généralement, ce complexe, granule, particule pseudo-virale (et notamment le polymère protéique de ce complexe, granule, particule pseudo-virale) peut ne pas comprendre d'intégrase, ni d'acide nucléique codant une intégrase.

Plus généralement, ce complexe, granule, particule pseudo-virale (et notamment le polymère protéique de ce complexe, granule, particule pseudo-virale) peut ne pas comprendre de protéase, ni d'acide nucléique codant une protéase.

La demande est également relative à l'ARN isolé de ce complexe, granule, particule pseudo-virale.

Le complexe, granule ou particule pseudo-virale peut avantageusement être utilisée pour la production d'ARNm ou ARNlnc présentant une queue polyA en 3' et/ou une coiffe 7-méthylguanosine en 5' (voire une méthylation d'un ou plusieurs résidu(s) adénine et/ou cytosine et/ou pseudouridine, notamment résidu(s) adénine et/ou cytosine).

La demande est également relative à un procédé de production (*in vitro*) d'ADN (notamment d'ADNc), qui comprend le fait de produire un ARN (notamment un ARNm ou ARNlnc) selon le procédé de production d'ARN de la demande, et de retro-transcrire cet ARN en ADN (notamment en ADNc), et de collecter cet ADN (ou ADNc).

La demande est également relative à un procédé de production (*in vitro*) de protéine, qui comprend le fait de produire un ARNm selon le procédé de production d'ARN de la demande, et de traduire cet ARNm en protéine *in vitro* (par exemple *in vitro* dans des cellules, notamment dans des cellules de mammifère, notamment dans des cellules humaines), et de collecter cette protéine.

La demande est également relative à un procédé pour la production d'une composition pharmaceutique, comprenant au moins un ARN d'intérêt, notamment au moins un ARNm ou ARNlnc d'intérêt.

Le procédé comprend le fait de produire au moins un ARN par le procédé de production d'ARN de la demande, et le fait de placer cet ARN en contact avec (en mélange avec, en suspension dans) ou dans un véhicule pharmaceutiquement acceptable pour produire une composition pharmaceutique.

Le terme « composition pharmaceutique » est entendu conformément à sa signification usuelle dans le domaine. Il inclut la signification de médicament, notamment de vaccin ou composition immunogène.

L'expression « véhicule pharmaceutiquement acceptable » est entendue conformément à sa signification usuelle dans le domaine. Elle inclut la signification de « véhicule physiologiquement acceptable », notamment physiologiquement acceptable pour une administration à l'être humain.

Un véhicule pharmaceutiquement (ou physiologiquement) acceptable peut par exemple tenir une ou plusieurs des fonctions suivantes : fonctions de diluant, excipient, additif, émulsifiant, agent dispersant, agent pour l'ajustement du pH, conservateur, surfactant, agent gélifiant, agent tampon, agent stabilisant, un agent stabilisateur d'ARN (notamment un agent protégeant l'ARN de la dégradation enzymatique), un agent solubilisant.

Des exemples de véhicules pharmaceutiquement (ou physiologiquement) acceptables sont connus de la personne du métier, et sont par exemple décrits dans "Remington: The Science and Practice of Pharmacy", 20th edition, Mack Publishing Co. et dans "Ansel's Pharmaceutical Dosage Forms and Drug Delivery Systems", Ansel, Popovich and Allen Jr., Lippincott Williams and Wilkins (neuvième édition).

Des exemples de véhicules pharmaceutiquement (ou physiologiquement) acceptables peuvent par exemple comprendre des fluides injectables, tels que l'eau, le sérum physiologique, les tampons, les émulsions.

La composition pharmaceutique (ou médicament) peut notamment être destinée à :
- la prévention ou le traitement d'une infection microbiologique, notamment bactérienne et/ou virale et/ou parasitaire (maladies infectieuses ou bio-défense),
- la prévention ou le traitement d'une prolifération tumorale,
- à la prévention ou au traitement d'une maladie chronique (mucoviscidose, par exemple),
- la thérapie par régénération tissulaire ou cellulaire (maladies cardio-vasculaires, maladies rares), ou à
- la thérapie génique (notamment par correction de gène ou d'un déficit génétique, notamment par réexpression de protéine qui se trouve être non fonctionnelle ou non exprimée).

Par exemple, un ARN produit conformément à la demande peut être formulé sous la forme de :
- un vaccin à ARN ou d'une composition immunogène à ARN (l'ARN administré étant destiné à être traduit dans les cellules, notamment dendritiques, du sujet, notamment du sujet humain, auquel il est administré) ;
- un médicament anti-tumoral ;
- un médicament pour le traitement ou la palliation de la mucoviscidose (par exemple, par apport d'ARN codant la CFTR) ; ou
- un médicament ou produit de régénération tissulaire ou cellulaire.

Le fait que l'ARN produit conformément à la demande puisse présenter une queue polyA en 3' et/ou une coiffe m7G en 5', voire une méthylation de résidu(s) adénine et/ou cytosine et/ou pseudouridine, notamment adénine et/ou cytosine, le rend directement compétent pour une traduction dans des cellules de mammifère, notamment dans des cellules humaines.

Dans la demande, à moins que cela ne soit autrement spécifié, ou que le contexte n'en dicte autrement, tous les termes ont leur sens habituel dans le(s) domaine(s) concerné(s).

Le terme "comprenant", avec lequel "incluant" ou "contenant" est synonyme, est un terme ouvert, et n'exclut pas la présence d'un ou plusieurs élément(s), ingrédient(s) ou étape(s) de méthode additionnel(s) qui ne serait(seraient) pas explicitement indiqué(s), tandis que le terme "consistant" ou "constitué" est un terme fermé, qui exclut la présence de tout autre élément additionnel, étape, ou ingrédient qui ne serait pas explicitement exposé.

Le terme "consistant essentiellement" ou " essentiellement constitué" est un terme partiellement ouvert, qui n'exclut pas la présence d'un ou plusieurs élément(s), ingrédient(s) ou étape(s) additionnel(s), dans la mesure où ce(s) élément(s), ingrédient(s) ou étape(s) additionnel(s) n'affecte(nt) pas matériellement les propriétés de base de l'invention.

Par conséquent, le terme "comprenant" (ou "comprend(comprennent)") inclut les termes "consistant", "constitué", aussi bien que les termes "consistant essentiellement" et "essentiellement constitué".

Dans le but de faciliter la lecture de la demande, la description a été séparée en divers paragraphes et sections. Il ne doit pas être considéré que ces séparations déconnectent la substance d'un paragraphe ou section de celle d'un autre paragraphe ou section. Au contraire, la description englobe toutes les combinaisons possibles des différents paragraphes, sections et phrases qu'elle contient.

Les exemples qui suivent sont donnés à titre purement illustratifs. Ils ne sont en aucune façon limitatifs.

### EXEMPLES

### EXEMPLE 1 :

Ont été produites :
- des constructions plasmidiques codant la protéine Gag (sous le contrôle d'un promoteur inductible), éventuellement en fusion avec une étiquette de purification (tag 6His) ou avec un marqueur de détection (eGFP) [*cf.* Figure 6A construction i. ; *cf.* Figure 7A], ainsi que
- des constructions plasmidiques codant un ARN hétérologue d'intérêt lié à une séquence d'adressage rétrosomal (sous le contrôle d'un promoteur inductible), par exemple des constructions plasmidiques contenant (en insert) un ADN codant un ARNm de eGFP (à titre d'ARN hétérologue) lié (en 3') à une séquence ADN GAG dépourvue du promoteur d'expression de la protéine Gag (à titre de séquence d'adressage rétrosomal) [*cf.* Figure 6A construction ii. ; *cf.* Figure 7B],
ont été produites et mises en œuvre pour transformation d'une souche de levure dépourvue de rétrotransposon Ty endogène (*Saccharomyces paradoxus*).

Les cellules de levure ainsi transformées produisent des rétrosomes T (*T-bodies* ou particules pseudo-virales) qui contiennent l'ARN hétérologue, sans rétro-transcrire cet ARN hétérologue ni le rétro-intégrer dans le génome.

Les ARN hétérologues ainsi produits ont été purifiés.

### Matériel et Méthodes

### 1. Souches et plasmides utilisés

### Souches :

Une souche de bactérie *Escherichia coli* DH5α est accessible auprès de l'ATCC^{®} sous le numéro 67877^{™}.

Une souche de levure *Saccharomyces paradoxus* sauvage est accessible auprès de l'ATCC^{®} sous le numéro 76528^{™}.

La souche de levure YAM13 est une souche de *S. paradoxus MATalpha Ty0 leu2 lys2 ura3.*

La souche de levure YAM13 ΔSrb2 est une souche *S. paradoxus* YAM13, dont le gène Srb2 a été délété.

La souche de levure YAM13 ΔSpt21 est une souche *S. paradoxus* YAM13, dont le gène Spt21 a été délété.

Une souche de levure *Saccharomyces cerevisiae* sauvage est accessible auprès de l'ATCC^{®} sous le numéro 52530^{™}.

ATCC^{®} est l'*American Type Culture Collection* (10801 University Blvd. ; Manassas, Virginia 20110-2209 ; U.S.A.).

*S. paradoxus* est naturellement dépourvue de rétrotransposon, notamment de rétrotransposon Ty1, contrairement à *S*. *cerevisiae* qui est naturellement pourvue de rétrotransposon Ty1.

### Construction codant la protéine Gag (cf. Figure 7A) :

Pour la construction codant la protéine Gag, deux plasmides ont été mis en œuvre, l'un pour amplifier la séquence *GAG* (SEQ ID NO : 1) et l'autre pour exprimer la protéine Gag avec et sans étiquette poly-histidine (tag 6-His).

Les séquences ADN, ARN et protéique du tag 6-His sont les séquences de SEQ ID NO : 4, 5 et 6, respectivement.

Le premier plasmide (pBDG1130Δpol) contient la séquence *GAG* et a servi de matrice pour l'amplification et l'ajout du tag 6-His en amont et/ou en aval (SEQ ID NO : 7, SEQ ID NO : 9 et SEQ ID NO : 11) de cette séquence par PCR.

Le second plasmide pAG425GAL-ccdB a été utilisé pour exprimer les protéines Gag avec ou sans tag 6-His en position N-terminale et /ou C-terminale dans la levure (*S. paradoxus* YAM13 ΔSrb2).

Les protéines ont été exprimées de façon inductible par la présence du promoteur au galactose GAL1. Ce plasmide est constitué d'une origine de réplication bactérienne et eucaryote (2µ- 20-50 copies par cellule) qui permet l'amplification du plasmide dans la bactérie *E. coli* DH5α et une expression des protéines dans la levure. Afin de sélectionner spécifiquement les cellules transfectées par le plasmide, différents marqueurs de sélection ont été utilisés. Pour la bactérie *E.coli*, le gène de résistance à l'ampicilline a permis la sélection des bactéries transfectées et le gène ccdB a permis d'induire la mort des bactéries transfectées n'ayant pas de recombinaison entre la séquence d'intérêt et le plasmide. Pour la souche de levure, le gène de synthèse de la leucine (LEU2) a permis la sélection des levures transformées par le plasmide. La levure YAM13 ΔSrb2 est auxotrophe pour cet acide aminé et la transformation par le plasmide permet la croissance des levures sur un milieu dépourvu en leucine (-LEU).

Le milieu d'expression des protéines d'intérêt par les levures s'est donc fait sur un milieu - LEU et contenant 2% de galactose.

Une construction plasmidique similaire a été produite pour exprimer la protéine Gag (SEQ ID NO : 3 en fusion avec la protéine eGFP au sein du plasmide pAG426-ccdB-eGFP (*cf*. Figure 7A). Le plasmide pAG426-ccdB-eGFP dispose du gène de synthèse de l'uracile (URA3) qui permet la sélection des levures transformées par le plasmide. La levure YAM13 ΔSrb2 est auxotrophe pour cet acide aminé et la transformation par le plasmide permet la croissance des levures sur un milieu dépourvu en uracile (-URA).

Les séquences ADN, ARN et protéique de la eGFP sont les séquences de SEQ ID NO : 25, 26 et 27, respectivement.

Construction codant l'ARN hétérologue présentant la séquence d'adressage Gag (*cf*. Figure 7B ; *cf.* construction ii) de Figure 6A) :
Un ARNm codant la eGFP (*enhanced Green Fluorescent Protein*) a été mis en œuvre à titre d'ARN hétérologue.
Un ARNm codant la luciférase a été mis en œuvre à titre d'ARN hétérologue et les séquences ADN, ARN et protéique de la luciférase sont les séquences de SEQ ID NO : 28, 29 et 30, respectivement.

La séquence d'adressage Gag est une séquence ARN d'adressage rétrosomal (séquence ARN qui adresse l'ARN hétérologue auquel elle est liée, vers les rétrosomes qui sont par ailleurs formés par la protéine Gag). La séquence d'adressage rétrosomal est une séquence

Gag non codante (séquence codant Gag qui a été modifiée par délétion du promoteur d'expression de Gag ; séquence Gag non codante pouvant également être désignée « mini-GAG » ou « MiniGag » ou « End GAG »).

Les séquences ADN et ARN de la séquence d'adressage Gag sont les séquences de SEQ ID NO : 13 et 14, respectivement.

L'ADN codant l'ARNm hétérologue et l'ADN codant la séquence d'adressage Gag ont été insérés dans un plasmide (plasmide pAG425-GAL-ccdB).

### 2. Clonage du plasmide épisomique codant la protéine GAG

### 2.1. Séquence GAG avec et sans Tag 6-His

### 2.1.1. Amplification de la séquence de GAG pour l'ajout du Tag 6-His par PCR

La stratégie adoptée pour le clonage est une stratégie reposant sur la ligation entre deux séquences nucléotidiques présentant des extrémités cohésives obtenues par digestions enzymatiques.

L'expression de la protéine Gag avec le tag 6-His facilite la purification sur colonne de nickel. La séquence *GAG* codante (SEQ ID NO : 1) a été amplifiée de façon à avoir en amont et/ou en aval une séquence permettant l'expression du tag 6-His (SEQ ID NO : 7, SEQ ID NO : 9 et SEQ ID NO : 11). Des sites de restrictions pour SpeI et HindIII ont été ajoutés de part et d'autre, et les séquences ont été clonées dans le plasmide pAG425GAL-ccdB. La PCR (*Polymerase Chain Reaction* ou réaction en chaîne de polymérase) a été utilisée pour amplifier la séquence *GAG* et permettre l'ajout des sites de restrictions et de la séquence codant le tag 6-His. La PCR a été réalisée à l'aide du kit KAPA HIFI HOTSTART READYMIX PCR^{™} (KAPABIOSYSTEMS) selon les recommandations du fournisseur, en utilisant différents couples d'amorces :
- amplification de la séquence codante *GAG* avec tag 6-His en amont de SEQ ID NO : 7 (appelée 6His-GAG) : amorce sens FQ3 de SEQ ID NO : 19 et amorce antisens RQ3 de SEQ ID NO : 20 ;
- amplification de la séquence codante *GAG* avec tag 6-His en aval de SEQ ID NO : 9 (appelée GAG-6His) : amorce sens FQ4 de SEQ ID NO : 21 et amorce antisens RQ4 de SEQ ID NO : 22 ;
- amplification de la séquence codante *GAG* avec tag 6-His en amont et en aval de SEQ ID NO : 11 (appelée 6His-GAG-6His) : amorce sens FQ3 de SEQ ID NO : 19 et amorce antisens RQ4 de SEQ ID NO : 22.

Les amorces RQ3 et FQ4 ont été utilisées pour amplifier *GAG* et ajouter les sites de restrictions aux extrémités. La réaction PCR a été faite avec des concentrations finales en amorces de 0,3 µM, 1X de KAPA HIFI HOTSTART READYMIX PCR^{™} et 200 ng/µL d'ADN. Du diméthylsulfoxyde (DMSO), à une concentration finale de 5%, a été ajouté pour les réactions d'amplification avec les couples d'amorces FQ4/RQ4 et FQ3/RQ4 afin d'éviter les structures secondaires pouvant être prises par les amorces. L'ADN matrice utilisé est le plasmide pBDG1130 contenant la séquence *GAG* à amplifier.

La dénaturation a été réalisée à 95°C durant 30 min, et suivie de 30 cycles de 20 sec à 98°C puis 15 sec à 65°C (couple d'amorces FQ3/RQ3) ou à 60°C (couples d'amorces FQ4/RQ4, FQ3/RQ4 et FQ4/RQ3), et 1 min à 72 °C. L'élongation terminale a été faite à 72°C pendant 3 min. A la fin de la réaction d'amplification, 8 µL de produit PCR ont été additionnés de 2 µL de tampon de charge 5X. Le mélange obtenu a été déposé sur un gel d'agarose 0,6 % + 5% de bromure d'éthidium (BET) puis la migration est réalisée pendant 1 h à 80 V. Le marqueur de taille utilisé est le 1 KB DNA LADDER ^{™} (NEW ENGLAND BIOLABS). Après migration, une révélation sous UV a permis de vérifier qu'il y a eu amplification spécifique.

Les produits PCR spécifiques ont été extraits avec le kit PCR CLEAN-UP, GEL EXTRACTION^{™} (MACHEREY-NAGEL) selon les recommandations du fournisseur. Les fragments extraits sont quantifiés au NANODROP puis digérés par HindIII et SpeI pour être ligués avec le plasmide d'expression pAG425GAL-ccdB.

### 2.1.2. Ligation

La digestion enzymatique par HindIII et SpeI du plasmide d'expression épisomique pAG425GAL-ccdB et des inserts (GAG avec tag 6-His C-terminal (SEQ ID NO : 7), GAG avec tag 6-His N-terminal (SEQ ID NO : 9), GAG avec tag 6-His C-terminal et N-terminal (SEQ ID NO : 11) a permis de linéariser le plasmide et de former des extrémités saillantes cohésives entre le plasmide et les différents inserts. La digestion par les deux enzymes de restrictions a été faite en une réaction dans les conditions suivantes : 1 µg d'ADN (insert ou plasmide), 5 U/mL de chacune des deux enzymes HindIII et SpeI, 1X de Tampon CUTSMART ^{™} 10X pour un volume réactionnel de 40 µL. La digestion a été réalisée pendant 1h30 à 37°C. Après digestion, le mélange réactionnel est déposé sur gel d'agarose 0,6 % afin de vérifier qu'il y a eu digestion et une extraction sur gel du fragment d'intérêt a été faite avec le kit PCR CLEAN-UP GEL EXTRACTION^{™} (MACHEREY-NAGEL) selon les recommandations du fournisseur.

Après quantification des extraits, la ligation a été faite en fonction d'un ratio molaire ADNp/insert de 1 : 3. La réaction a été faite à 25°C pendant 5 min (Quick ligation kit NEB). Après obtention des différents plasmides recombinants, une amplification par *E. coli DH5α* a été réalisée des plasmides construits pAG425GAL-GAG, pAG425GAL-6His-GAG. pAG425GAL-GAG-6His, pAG425GAL-6His-GAG-6His.

### 2.2 Séquence de fusion GAG-eGFP

### 2.2.1. Amplification de la séquence de GAG pour la fusion avec eGFP

La séquence *GAG* codante (SEQ ID NO : 1) a été amplifiée par PCR sans le codon stop (TGA) de façon à permettre la fusion avec eGFP. Les amorces d'amplification permettent d'avoir en amont et en aval de la séquence amplifiée les sites de restrictions SpeI et EcoRI. La PCR a été réalisée à l'aide du kit KAPA HIFI HOTSTART READYMIX PCR^{™} (KAPABIOSYSTEMS) selon les recommandations du fournisseur, en utilisant le couple d'amorce :
- amplification de la séquence codante *GAG* sans codon stop (TGA) : amorce sens FQ5 de SEQ ID NO : 31 et amorce antisens RQ5 de SEQ ID NO : 32 ;

La réaction PCR a été faite avec des concentrations finales en amorces de 0,3 µM, 1X de KAPA HIFI HOTSTART READYMIX PCR^{™} et 200 ng/µL d'ADN. Du diméthylsulfoxyde (DMSO), à une concentration finale de 5 %, a été ajouté pour les réactions d'amplification avec les couples d'amorces FQ5/RQ5. L'ADN matrice utilisé est le plasmide pBDG1 130 contenant la séquence *GAG* à amplifier.

A la fin de la réaction d'amplification, 8 µL de produit PCR ont été additionnés de 2 µL de tampon de charge 5X. Le mélange obtenu a été déposé sur un gel d'agarose 0,6 % + 5 % de bromure d'éthidium (BET) puis la migration est réalisée pendant 1 h à 80 V. Le marqueur de taille utilisé est le 1 KB DNA LADDER ^{™} (NEW ENGLAND BIOLABS). Après migration, une révélation sous UV a permis de vérifier qu'il y a eu amplification spécifique.

Les produits PCR spécifiques ont été extraits avec le kit PCR CLEAN-UP, GEL EXTRACTION^{™} (MACHEREY-NAGEL) selon les recommandations du fournisseur. Les fragments extraits sont quantifiés au NANODROP puis digérés par SpeI et EcoRI pour être ligués avec le plasmide d'expression pAG426GAL-ccdB-eGFP.

### 2.2.2. Digestion et ligation

La digestion enzymatique par SpeI et EcoRI du plasmide d'expression pAG426GAL-ccdB-eGFP et de l'insert (GAG sans codon stop (SEQ ID NO : 37) a permis de linéariser le plasmide et de former des extrémités saillantes cohésives entre le plasmide et l'insert. La digestion par les deux enzymes de restrictions a été faite en une réaction dans les conditions suivantes : 1 µg d'ADN (insert ou plasmide), 5 U/mL de chacune des deux enzymes HindIII et SpeI, 1X de Tampon NEB 2.1 1X pour un volume réactionnel de 40 µL. La digestion a été réalisée pendant 1h30 à 37°C. Après digestion, le mélange réactionnel est déposé sur gel d'agarose 0,6 % afin de vérifier qu'il y a eu digestion et une extraction sur gel du fragment d'intérêt a été faite avec le kit PCR CLEAN-UP GEL EXTRACTION^{™} (MACHEREY-NAGEL) selon les recommandations du fournisseur. Après quantification des extraits, la ligation a été faite en fonction d'un ratio molaire ADNp/insert de 1 : 3. La réaction a été faite à 25°C pendant 5 min (Quick ligation kit NEB).. Après obtention des différents plasmides recombinants, une amplification par *E. coli DH5α* a été réalisée du plasmide construit pAG426GAL-GAGeGFP.

### 2.3 Séquence GAG-eGFP-6-His avec et sans 3MS2

### 2.3.1. Synthèse et digestion de la séquence de GAG- eGFP-6-His-3MS2

La cassette « GAG-eGFP-6HIS-3MS2 » est commandée (SEQ ID NO : 38) et synthétisée par GenScript et insérée dans le plasmide pUC57 (pUC57-GeGHM). Cette séquence code pour la protéine de fusion Gag-eGFP avec un tag histidine en C-terminal avec en 3' de cette séquence trois répétitions MS2 (SEQ ID NO : 35).

Le plasmide pUC57- GAG-eGFP-6HIS (pUC57-GeGH) est cloné en retirant la séquence 3MS2 (SEQ ID NO : 35) par digestion NotI suivi d'une ligation à 25°C pendant 5 min (Quick ligation kit NEB).

Les plasmides pUC57-GeGHM et pUC57-GeGH sont digérés par les enzymes de restriction SpeI et KpnI. Le fragment correspondant à la séquence GAG-eGFP-6HIS-3MS2 (SEQ ID NO : 38) (2369 bp) et GAG-eGFP-6HIS (SEQ ID NO : 39) (2101 bp) sont purifiés après dépôt sur gel 0,6 % agarose afin de vérifier qu'il y a eu digestion et une extraction sur gel du fragment d'intérêt a été faite avec le kit PCR CLEAN-UP GEL EXTRACTION^{™} (MACHEREY-NAGEL) selon les recommandations du fournisseur.

### 2.3.2. Ligation

La digestion enzymatique par SpeI et KpnI du plasmide d'expression pAG426GAL-ccdB-eGFP et des inserts GAG sans codon stop (SEQ ID NO : 37) a permis de linéariser le plasmide et de former des extrémités saillantes cohésives entre le plasmide et les inserts GAG-eGFP-6HIS-3MS2 (SEQ ID NO : 38) et GAG-eGFP-6HIS (SEQ ID NO : 39). Après digestion, le mélange réactionnel est déposé sur gel d'agarose 0,6 % afin de vérifier qu'il y a eu digestion et une extraction sur gel du fragment d'intérêt a été faite avec le kit PCR CLEAN-UP GEL EXTRACTION^{™} (MACHEREY-NAGEL) selon les recommandations du fournisseur. Après quantification des extraits, la ligation a été faite en fonction d'un ratio molaire ADNp/insert de 1 : 3. La réaction a été faite à 25°C pendant 5 min (Quick ligation kit NEB). Après obtention des différents plasmides recombinants, une amplification par *E. coli DH5α* a été réalisée.

Après quantification des extraits, la ligation a été faite en fonction d'un ratio molaire ADNp/insert de 1 : 3. La réaction a été faite à 25°C pendant 5 min (Quick ligation kit NEB). Après obtention des différents plasmides recombinants, une amplification par *E. coli DH5α* a été réalisée des plasmides construits pAG426GAL-GeGHM et pAG426GAL-GeGH.

### 3. Clonage du plasmide intégratif codant la protéine GAG

### 3.1. Amplification de la séquence de GAL-GAG-eGFP par PCR

La séquence GAL-GAG-eGFP codant pour le promoteur Galactose la protéine de fusion Gag-eGFP (SEQ ID NO : 42) a été amplifiée par PCR. Les amorces d'amplification permettent d'avoir en amont et en aval de la séquence amplifiée les sites de restrictions SacI et KpnI. La PCR a été réalisée à l'aide du kit KAPA HIFI HOTSTART READYMIX PCR^{™} (KAPABIOSYSTEMS) selon les recommandations du fournisseur, en utilisant le couple d'amorce :
- amplification de la séquence codante *GAG*-eGFP : amorce sens FQ5 de SEQ ID NO : 31 et amorce antisens RQ5 de SEQ ID NO : 32 ;

A la fin de la réaction d'amplification, 8 µL de produit PCR ont été additionnés de 2 µL de tampon de charge 5X. Le mélange obtenu a été déposé sur un gel d'agarose 0,6 % + 5 % de bromure d'éthidium (BET) puis la migration est réalisée pendant 1 h à 80 V. Le marqueur de taille utilisé est le 1 KB DNA LADDER ^{™} (NEW ENGLAND BIOLABS). Après migration, une révélation sous UV a permis de vérifier qu'il y a eu amplification spécifique.

### 3.2. Ligation

Le plasmide de levure intégratif pRS306 possède un marqueur URA3 et un gène de résistance à l'Ampiciline AmpR.

Le plasmide d'expression pRS306 subi une digestion enzymatique par SacI et KpnI.

La digestion par les deux enzymes de restrictions a été faite en une réaction dans les conditions suivantes : 1 µg d'ADN (insert ou plasmide), 5 U/mL de chacune des deux enzymes SacI et KpnI, 1X de Tampon NEB 1.1 1X pour un volume réactionnel de 40 µL. La digestion a été réalisée pendant 1h30 à 37°C. Après digestion, le mélange réactionnel est déposé sur gel d'agarose 0,6 % afin de vérifier qu'il y a eu digestion et réaliser une extraction sur gel du fragment d'intérêt a été faite avec le kit PCR CLEAN-UP GEL EXTRACTIONTM (MACHEREY-NAGEL) selon les recommandations du fournisseur. La ligation est réalisée à l'aide du kit Gibson Assembly dans les conditions suivantes : 5 µL Gibson Master Mix + 50 ng de vecteur pRS306 digéré SacI/KpnI (4271pb) + 0,5 pM soit 926 ng de PCR GAL-GAG-EGFP + Qsp H2O 10 µL. La réaction est incubée 1h à 50°C. Après obtention des différents plasmides recombinants, une amplification par *E. coli DH5α* a été réalisée.

### 4. Clonage du plasmide codant l'ARN hétérologue

Un ARNm codant la eGFP (*enhanced Green Fluorescent Protein*) a été mis en œuvre à titre d'ARN hétérologue. Les séquences ADN, ARN et protéique de la eGFP sont les séquences de SEQ ID NO : 25, 26 et 27, respectivement.

Un ARNm codant la luciférase a été mis en œuvre à titre d'ARN hétérologue. Les séquences ADN, ARN et protéique de la luciférase sont les séquences de SEQ ID NO : 28, 29 et 30, respectivement

### 4.1 Séquence Luciférase au titre d'ARN hétérologue

### 4.1.1. Séquence EndGAG au titre de séquence de ciblage

La cassette « Luc-EndGAG-3MS2 » est commandée (SEQ ID NO : 43) et synthétisée par GenScript et insérée dans le plasmide pUC57 (pUC57-LEGM). Cette séquence code pour la protéine luciférase en 3' de cette séquence la séquence de ciblage dans les *T-bodies* EndGAG suivi de trois répétitions MS2 (SEQ ID NO : 35).

Le plasmide pUC57- Luc-EndGAG (pUC57-LEG) est cloné en retirant la séquence 3MS2 (SEQ ID NO : 35) par digestion NotI suivi d'une ligation à 25°C pendant 5 min (Quick ligation kit NEB).

Les plasmides pUC57-LEGM et pUC57-LEG sont digérés par les enzymes de restriction SpeI et HindIII. Le fragment correspondant à la séquence Luc-EndGAG-3MS2 (SEQ ID NO : 43) (3425 bp) et Luc-EndGAG (SEQ ID NO : 44) (3039 bp) sont purifiés après dépôt sur gel 0,6% agarose afin de vérifier qu'il y a eu digestion et une extraction sur gel du fragment d'intérêt a été faite avec le kit PCR CLEAN-UP GEL EXTRACTION^{™} (MACHEREY-NAGEL) selon les recommandations du fournisseur. La digestion enzymatique par SpeI et KpnI du plasmide d'expression pAG425GAL-ccdB et des inserts Luc-EndGAG-3MS2 (SEQ ID NO : 43) et Luc-EndGAG (SEQ ID NO : 44) a permis de linéariser le plasmide et de former des extrémités saillantes cohésives entre le plasmide et les inserts. Après digestion, le mélange réactionnel est déposé sur gel d'agarose 0,6 % afin de vérifier qu'il y a eu digestion et une extraction sur gel du fragment d'intérêt a été faite avec le kit PCR CLEAN-UP GEL EXTRACTION^{™} (MACHEREY-NAGEL) selon les recommandations du fournisseur.

Après quantification des extraits, la ligation a été faite en fonction d'un ratio molaire ADNp/insert de 1 : 3. La réaction a été faite à 25°C pendant 5 min (Quick ligation kit NEB). Après obtention des différents plasmides recombinants, une amplification par *E. coli DH5α* a été réalisée des plasmides construits pAG425GAL-LEGM et pAG425GAL-LEG.

### 4.1.2 Séquence GAGm au titre de séquence de ciblage

La séquence « GAGm » est commandée (SEQ ID NO : 13) et synthétisée par GenScript et insérée dans le plasmide pUC57 (pUC57-Gm). Cette séquence est la séquence de ciblage dans les *T-bodies* complète et mutée (SEQ ID NO : 14).

Le plasmide pAG425GAL-LEGM est digéré par l'enzyme de restriction NdeI puis partiellement par l'enzyme de restriction NotI. Les fragments correspondant au plasmide libéré de la séquence EndGAG (9850 bp) et au plasmide libéré de la séquence EndGAG et 3MS2 (9464 bp) sont purifiés après dépôt sur gel 0,6% agarose afin de vérifier qu'il y a eu digestion et une extraction sur gel du fragment d'intérêt a été faite avec le kit PCR CLEAN-UP GEL EXTRACTION^{™} (MACHEREY-NAGEL) selon les recommandations du fournisseur.

Le plasmide pUC57-GAGm est digéré par les enzymes de restriction NdeI et NotI, Le fragment correspondant à GAGm (1336 bp) est purifié après dépôt sur gel 0,6 % agarose et une extraction sur gel du fragment d'intérêt a été faite avec le kit PCR CLEAN-UP GEL EXTRACTION^{™} (MACHEREY-NAGEL) selon les recommandations du fournisseur.

Après quantification des extraits, la ligation a été faite en fonction d'un ratio molaire ADNp/insert de 1 : 3. La réaction a été faite à 25°C pendant 5 min (Quick ligation kit NEB). Après obtention des différents plasmides recombinants, une amplification par *E. coli DH5α* a été réalisée des plasmides construits pAG425GAL-LGmM et pAG425GAL-LGm.

### 4.2 Séquence eGFP au titre d'ARN hétérologue

La séquence eGFP est amplifiée par PCR à partir du plasmide pCMV-eGFP Les amorces d'amplification permettent d'avoir en amont et en aval de la séquence amplifiée les sites de restrictions SpeI et AleI. La PCR a été réalisée à l'aide du kit KAPA HIFI HOTSTART READYMIX PCR^{™} (KAPABIOSYSTEMS) selon les recommandations du fournisseur, en utilisant le couple d'amorce. A la fin de la réaction d'amplification, 8 µL de produit PCR ont été additionnés de 2 µL de tampon de charge 5X. Le mélange obtenu a été déposé sur un gel d'agarose 0,6 % + 5 % de bromure d'éthidium (BET) puis la migration est réalisée pendant 1 h à 80 V. Le marqueur de taille utilisé est le 1 KB DNA LADDER ^{™} (NEW ENGLAND BIOLABS). Après migration, une révélation sous UV a permis de vérifier qu'il y a eu amplification spécifique.

Les plasmides pAG425GAL-LGmM, pAG425GAL-LGm, pAG425GAL-LEGM et pAG425GAL-LEG sont digérés par les enzymes de restriction SpeI et AleI. Les fragments correspondant aux plasmides libérés de la luciférase est purifié après dépôt sur gel 0,6 % agarose et une extraction sur gel du fragment d'intérêt a été faite avec le kit PCR CLEAN-UP GEL EXTRACTION^{™} (MACHEREY-NAGEL) selon les recommandations du fournisseur.

Après quantification des extraits, la ligation a été faite en fonction d'un ratio molaire ADNp/insert de 1 : 3. La réaction a été faite à 25°C pendant 5 min (Quick ligation kit NEB). Après obtention des différents plasmides recombinants, une amplification par *E. coli DH5α* a été réalisée des plasmides construits pAG425GAL-eGFPGmM, pAG425GAL-eGFPGm, pAG425GAL-eGFPEGM et pAG425GAL-eGFPEG

### 5. Amplification des plasmides recombinants par E. coli DH5α

La transformation des bactéries *E. coli* DH5α a été faite par ajout de 5 µL du produit de ligation pour 50 µL de bactéries *E. coli* DH5α compétentes. Une incubation de 30 min sur la glace a été faite avant d'induire pendant 30s un choc thermique à 42 °C. Puis les échantillons ont été incubés sur la glace pendant 5 min avant de les mettre à 37 °C pendant 50 min dans 300 µL de milieu SOC. Les bactéries transformantes ont été étalées sur boîtes milieu LB + 100 µg/mL d'ampicilline.

Après culture des transformants, plusieurs clones ont été repiqués et repris en milieu liquide afin d'extraire les plasmides recombinants. L'extraction a été faite à l'aide du kit PLASMID DNA PURIFICATION^{™} (MACHEREY-NAGEL) selon les recommandations du fournisseur. Après purification, une digestion enzymatique par HpaI (NEW ENGLAND BIOLABS) est réalisée. Cette digestion a été effectuée dans les mêmes conditions que celles par HindIII/SpeI (NEW ENGLAND BIOLABS). Pour les plasmides recombinants, deux sites de restrictions ont été observés tandis que pour les plasmides non recombinants un seul site de restriction a été observé. Trois profils ont pu être retrouvés : plasmide linéarisé non digéré par HindIII/SpeI (9249 pb), plasmide linéarisé digéré par HindIII/SpeI (7495 pb) et plasmide linéarisé recombinant (6752 pb + 2036 pb). Un séquençage a été fait pour les plasmides recombinants.

### 6. Transformation des levures

### 6.1. Transformation avec plasmide épisomique

Les différents plasmides recombinants épisomiques obtenus ont été utilisés pour transformer les levures *S. paradoxus* et *S. cerevisae* (*cf.* Figures 7A et 7B).

Les levures sont lavées avec de l'eau puis avec 0,1 M d'acétate de lithium (LiOAc). Le culot a été remis en suspension dans 240 µL de PEG 50 %, 36 µL de LiOAc 1 M + 8 µL d'ADN plasmidique + 5 µL d'ADN de saumon dénaturé. Le mélange obtenu a été agité durant 1 min puis incubé au moins 30 min à 25°C. Un choc thermique a été induit à 42°C ou 37°C durant 20 min. Les cellules sont lavées puis remises en suspension dans de l'eau distillée stérile. Les levures transformées ont été étalées sur la boîte sélective dépourvue en Leucine (dans le cas de la transformation d'un plasmide possédant le gène Leu2 dont le backbone est nomMé pAG425) en Uracile (dans le cas de la transformation d'un plasmide possédant le gène URA3 dont le backbone est nommé pAG426), en Leucine et Uracile (dans le cas de la transformation de deux plasmides l'un possédant le gène URA3 dont le backbone est nommé pAG426 et l'autre possédant le gène Leu2 dont le backbone est nmomé pAG425).

Des exemples de souches de *S. paradoxus* illustrant cette transformation avec plasmide épisomique ont été déposées auprès de la CNCM en vertu du Traité de Budapest, voir ci-après le tableau 1.

### 6.2. Transformation avec plasmide intégratif

Le plasmide pRS306-GAL-GAG-EGFP est linéarisé par digestion avec AatII FastDigest (ThermoScientifique) dans les conditions suivantes : 2µL d'enzyme AatII + 2 µg d'ADNpl pRS306-GAL-GAG-EGFP + 5µL de Tampon Green FastDigest + Qsp 50 µL d'H2O. La réaction est incubée 25min à 37°C. Après digestion, le mélange réactionnel est déposé sur gel d'agarose 0,6 % afin de vérifier qu'il y a eu digestion et une extraction sur gel du fragment d'intérêt a été faite avec le kit PCR CLEAN-UP GEL EXTRACTION^{™} (MACHEREY-NAGEL) selon les recommandations du fournisseur.

Les levures sont lavées avec de l'eau puis avec 0,1 M d'acétate de lithium (LiOAc). Le culot a été remis en suspension dans 240 µL de PEG 50 %, 36 µL de LiOAc 1 M + 8 µL d'ADN plasmidique + 5 µL d'ADN de saumon dénaturé. Le mélange obtenu a été agité durant 1 min puis incubé au moins 30 min à 25°C. Un choc thermique a été induit à 42°C ou 37°C durant 20 min. Les cellules sont lavées puis remises en suspension dans de l'eau distillée stérile. Les levures transformées ont été étalées sur la boîte sélective dépourvue en Leucine en Uracile (dans le cas de la transformation d'un plasmide possédant le gène URA3 dont le backbone est nommé pRS306), en Leucine et Uracile (dans le cas de la transformation de deux plasmides l'un possédant le gène URA3 dont le backbone est nommé pRS306 et l'autre possédant le gène Leu2 dont le backbone est nomMé pAG425).

Des exemples de souches de *S. paradoxus* illustrant cette transformation avec plasmide intégratif ont été déposées auprès de la CNCM en vertu du Traité de Budapest, voir ci-après le tableau 1.

**Tableau 1 :**

| **Identité de la souche** | **Numéro CNCM** | **Date de dépôt** |
|---|---|---|
| Souche « TB16 » = *S. paradoxus* avec remplacement du gène *Rpbl* par une version mutée (C67Y) du gène *Rpbl* de *S. cerevisae,* intégration de la séquence codant pour la protéine de fusion Gag-eGFP (SEQ ID NO : 3 - SEQ ID NO : 27) sous l'influence d'un promoteur galactose (GAL) [Milieu sélectif URA] | I-5171 | 24 février 2017 |
| Souche « TB17 » : *S. paradoxus* avec remplacement du gène *Rpbl* par une version mutée (C70Y) du gène *Rpbl* de *S. cerevisae,* et intégration de la séquence codant pour la protéine de fusion Gag-eGFP (SEQ ID NO : 3 - SEQ ID NO : 27) sous l'influence d'un promoteur galactose (GAL) [Milieu sélectif URA] | I-5172 | 24 février 2017 |
| Souche « TB18 » : *S. paradoxus* avec remplacement du gène *Rpbl* par une version mutée (H80Y) du gène *Rpbl* de *S. cerevisae,* et intégration de la séquence codant pour la protéine de fusion Gag-eGFP (SEQ ID NO : 3 - SEQ ID NO : 27) sous l'influence d'un promoteur galactose (GAL) [Milieu sélectif URA] | I-5173 | 24 février 2017 |
| Souche « TB21 » : *S. paradoxus* avec délétion du gène *Srb2* et avec remplacement du gène *Rpbl* par une version mutée (H80Y) du gène *Rpbl* de *S. cerevisae,* et intégration de la séquence codant pour la protéine de fusion Gag-eGFP (SEQ ID NO : 3 - SEQ ID NO : 27) sous l'influence d'un promoteur galactose (GAL) [Milieu sélectif URA] | I-5174 | 24 février 2017 |
| Souche « TB32 » : *S. paradoxus* avec délétion du gène *Srb2,* et intégration dans le génome de séquence codant pour la protéine de fusion Gag-eGP-6His-3MS2 (SEQ ID NO : 3 - SEQ ID NO : 27- SEQ ID NO : 6) Gag-eGFP-6His-3MS2 sous l'influence d'un promoteur galactose (GAL) [Milieu sélectif URA] | I-5175 | 24 février 2017 |
| Souche « TB53 » : *S. paradoxus* avec délétion du gène *Srb2* ; intégration de la séquence codant pour la protéine de fusion Gag-eGFP (SEQ ID NO : 3 - SEQ ID NO : 27) sous l'influence d'un promoteur galactose (GAL) [Milieu sélectif URA] ; et transformation avec plasmide permettant l'expression d'un ARN modèle Luciférase (SEQ ID NO : 29) disposant en 3' d'une séquence de ciblage complète mutée (GAGm = SEQ ID NO : 14) de l'ARN dans les *T-bodies* sous l'influence d'un promoteur galactose (GAL) [Milieu sélectif LEU] | I-5293 | 15 mars 2018 |
| Souche « TB54 » : *S. paradoxus* avec délétion du gène *Srb2* ; intégration de la séquence codant pour la protéine de fusion Gag-eGFP (SEQ ID NO : 3 - SEQ ID NO : 27) sous l'influence d'un promoteur galactose (GAL) [Milieu sélectif URA] ; et transformation avec plasmide permettant l'expression d'un ARN modèle Luciférase (SEQ ID NO : 29) disposant d'une séquence de ciblage partielle (ou minimale) (SEQ ID NO 18) de l'ARN dans les *T-bodies* sous l'influence d'un promoteur galactose (GAL) [Milieu sélectif LEU] | I-5294 | 15 mars 2018 |

| | | |
|---|---|---|
| CNCM est la Collection Nationale de Culture de Microorganismes (Institut Pasteur ; 28, rue du Dr Roux ; 75724 Paris Cedex 15 ; France). | | |

### 7. Induction de l'expression et extraction des T-bodies

### 7.1. Induction galactose

Dans le but de produire des T-bodies et d'exprimer la protéine Gag (SEQ ID NO : 3) avec ou sans tag 6-His (SEQ ID NO : 8, SEQ ID NO : 10 et SEQ ID NO : 12), une induction en galactose est réalisée. Les cellules sont centrifugées 10 min à 3000 rpm puis rinces 2 fois à l'eau stérile. Les cellules sont ensuite réensemmencé dans le milieu sélectif approprié aux plasmides épisomiques et ou intégratifs transformés dépourvu de glucose et complémentée par 2% de galactose. L'induction s'est déroulée une nuit durant à 25°C. Différentes techniques d'extraction mécanique et chimique ont été testées pour optimiser les conditions d'obtention des rétrosomes T (*T-bodies* ou particules pseudo-virales).

### 7.2. Lyse mécanique par des billes

Les levures ont été centrifugées 3 min à 3 000 rpm puis lavées dans du tampon de lyse froid (50 mM Tris pH 7,6 ; 50 mM NaCl ; 5 mM MgCl2 ; 0,1 % NPA ; 1 mM β-mercaptoéthanol ; 1X PROTEASE INHIBITOR COMPLETE MINI EDTA FREE^{™} ; 0,4 U/µL RNASE INHIBITOR^{™}). Les cellules sont centrifugées Le culot est repris dans du tampon de lyse froid additionné de billes. Le mélange obtenu a été mélangé par vortex sur glace pendant 1 min. Une centrifugation de 2 min à 2000 g est faite et le surnageant a été collecté puis centrifugé 10 min à 10000 g. Le culot a été repris dans du tampon de lyse froid et incubé 30 min à température ambiante. Une centrifugation de 10 min à 10000 g est faite puis le culot a été repris dans du tampon de lyse froid.

### 7.3. Lyse mécanique par sonication

Les levures ont été centrifugées 10 min à 10000 g puis remises en suspension dans du BINDING BUFFER^{™} 1X contenant 80 U/mL de lyticase afin d'obtenir des sphéroplastes. La suspension a été incubée 10 min à 25°C. Suite à cette incubation, les levures ont été lysées par sonication sur la glace à raison de 10 x 15 s à 70 % avec 15 s de refroidissement entre chaque sonication. Une centrifugation de 15 min à 5000 g a été faite afin de collecter les corps d'inclusion et les débris cellulaires.

### 7.4. Lyse chimique en hydroxyde de sodium

Les levures ont été centrifugées et remises en suspension dans un mélange volume à volume de H₂O et NaOH 0,2M. Le mélange obtenu a été incubé à température ambiante pendant 5 min puis centrifugé 2 min à 14000 rpm. Le culot obtenu a été repris dans du LAEMMLI^{™} 2X pour être dénaturé 15 min à 95 °C. Après dénaturation, une centrifugation de 2 min à 8000 rpm a été faite et le surnageant a été récupéré dans un nouveau tube pour être conservé à 4 °C.

### 7.5. Lyse chimique Y-PERTM

Le kit Y-PERTM YEAST PROTEIN EXTRACTION REAGENT^{™} (THERMOSCIENTIFIC) a été utilisé suivant les recommandations du fournisseur.

### 7.6. Lyse mécanique par homogénéisation à haute pression

Les levures sont lysées par homogénéisation haute pression 3 passages à 2000 bar dans le tampon de lyse (50 mM Tris pH 7,6, 50 mM NaCl, 5 mM MgCl2, 0,1% NP-40, 1 mM β-mercaptoethanol, 1× protease inhibitor complete mini EDTA free, 0.4 U/µL RNase inhibitor) (V/V).

### 8. Dosage protéique

Le dosage des extraits protéiques a été réalisé par la méthode BCA (INTERCHIM).

### 9. Transfert de Western SDS-PAGE

Les protéines extraites ont été séparées sur gel SDS-PAGE dans un tampon de migration 1 X. Le gel de séparation utilisé contenait 12 % d'acrylamide. La migration a été faite pendant 1h à 180V. Le marqueur de taille, le PRECISION PLUS PROTEIN ^{™} KALEIDOSCOPE ^{™} (BIO-RAD), a été déposé à raison de 10µL/puit. Après migration, les protéines ont été transférées sur une membrane de polyfluorure de vinylidène (PVDF) par un transfert semi-sec. La membrane de PVDF a été préalablement activée en méthanol avant de réaliser le montage du transfert. Le montage a été fait comme suit, 5 papiers WHATMANN humidifiés dans du tampon de transfert 1X, la membrane de PVDF, le gel de séparation 12% d'acrylamide et 5 papiers WHATMANN humidifiés dans du tampon de transfert 1X. Le transfert a été fait à 2,5 A ; 25 V durant 7 min. La membrane a été récupérée et puis lavée deux fois pendant 10 min par du TBS + 0,1 % TWEEN (TBST). Une saturation a été faite durant 1 h en TBST 0,1% + 3-5% de lait. Après saturation, trois lavages de 15 min ont été faits en TBST 0,1% puis les anticorps primaires ont été ajoutés pour incubation une nuit durant à 4°C. Des anticorps primaires de souris anti-Ty1 et des anticorps de lapin anti-6-His ont été dilués au 1/2000 dans du TBST 0,1% + 3% de lait. Après l'incubation avec les anticorps primaires, la membrane a été lavée trois fois 15 min en TBST 0,1% et les anticorps secondaires ont été ajoutés pour incubation 1h à température ambiante sous agitation. Les anticorps secondaires anti-IgG de souris (HRP) et l'anticorps anti-IgG de lapin (HRP) ont été dilués au 1/20 000 dans du TBST 0,1% + 3% de lait. Après l'incubation avec les anticorps secondaires, les membranes ont été lavées 3 fois 15 min dans du TBST 0,1 %. Les membranes sont ensuite révélées par le CLARITY WESTERN ECL BLOTTING SUBSTRATE^{™} (BIO-RAD). Un mélange volume à volume (1:1) de CLARITY WESTERN PEROXIDE REAGENT^{™} et CLARITY WESTERN LUMINOL/ENHANCER ^{™} a été fait et déposé sur les membranes. La lecture a été faite au PIXI^{™} ou par film photographique.

### 10. Analyse de l'immunofluorescence au microscope confocal

Afin de vérifier que la présence du tag 6-His (SEQ ID NO : 5) n'interfère pas avec la formation des rétrosomes T (*T-bodies*)*,* un marquage de ces *T-bodies* a été fait à l'aide du couple d'anticorps anti-Tyl/FITC.

Avant le marquage, l'induction de l'expression des différentes protéines a été faite une nuit durant à 25°C en -LEU + 2% de galactose. Les levures ont ensuite été fixées avec 4% de formaldéhyde pendant 1 h à 25°C. Les cellules ont été récupérées après centrifugation de 3 min à 2000 rpm et lavées trois fois dans une solution de 0,1M KHPO₄; pH 6,5 et une fois dans une solution de 1,2M de sorbitol ; 0,1M KHPO₄; pH 6,5. Le culot a été replacé en suspension dans une solution de 1,2M de sorbitol ; 0,1M KHPO₄; pH 6,5, puis le culot a été ajouté volume à volume dans une solution composée de 1,2M de sorbitol ; 0,1 M KHPO₄; pH 6,5 additionnée de 1/100 β-mercaptoéthanol. Le mélange obtenu a été incubé 5 min à température ambiante. La lyticase a été ajoutée à raison de 50 U/mL final avant l'incubation à 25°C pendant 30 min. Les sphéroplastes obtenus ont été récupérés par une centrifugation de 2 min à 2000 rpm. Un lavage a été fait avec une solution de 1,2M de sorbitol ; 0,1M KHPO₄; pH 6,5. Du méthanol froid a été ajouté à volume équivalent durant 6 min puis de l'acétone froid durant 30 s. Un lavage au PBS complémenté avec 1% de BSA a été fait avant de saturer les levures durant 5-10 min dans cette même solution. La solution de saturation a été éliminée et l'anticorps primaire de souris anti-Ty1 dilué au 1/1000 dans du PBS a été mis en contact avec les levures une nuit durant à 4°C. Le lendemain, les levures ont été lavées 5 fois en PBS +1% BSA. L'anticorps secondaire anti-IgG de souris couplé à la fluorescéine (FITC) dilué au 1/2000 dans du PBS+BSA 1% a été incubé pendant 2h à température ambiante dans l'obscurité. Après incubation, 5 lavages en PBS + BSA 1% et un lavage avec une solution de 1,2M de sorbitol; 0,1 M KHPO₄; pH 6,5 ont été effectués. Les levures ont été remises en suspension dans cette dernière solution avant d'être déposées entre lames et lamelles.

### 11. Analyse de la fluorescence par cytométrie en flux

Pour déterminer quelle technique d'extraction permet de récupérer le plus de cellules positives pour Gag (SEQ ID NO : 3), un couple d'anticorps couplé à de la fluorescence a été utilisé puis la fluorescence a été analysée par cytométrie en flux.

Pour 100 µL d'extraits protéiques, il a été ajouté 400 µL de PBS complémentés avec 10 % de sérum de veau fœtal (SVF) et 0,1% de RNAsin^{®} (PROMEGA). Le tout a été incubé à 4°C durant 30 min. L'anticorps primaire de souris anti-Ty1 dilué au 1/100, a été incubé durant 1h à 4°C. L'anticorps secondaire anti-IgG de souris Cy5 dilué au 1/200 a été ajouté puis incubé 30 min à 4 °C. Les échantillons ont été analysés par cytométrie en flux.

### 12. Purification par chromatographie d'affinité au nickel

La purification a été faite à l'aide du kit HIS BIND^{™} RESIN CHROMATOGRAPHY (NOVAGEN). La purification a été basée sur l'immobilisation d'ions bivalents nickel (Ni²⁺) sur une matrice d'acide nitrilotriacétique (NTA). Le tag 6-His (SEQ ID NO : 6) ajouté en fusion de la protéine Gag (SEQ ID NO : 3) est constitué de plusieurs noyaux imidazoles qui sont des donneurs d'électrons permettant d'interagir fortement avec les ions Ni²⁺ immobilisés sur la matrice. La protéine Gag en fusion avec un tag 6-His en Nter et/ou Cter (SEQ ID NO : 8, SEQ ID NO : 10 et SEQ ID NO : 12) est retenue sur la colonne de nickel. Après rétention de la protéine sur la colonne, une importante concentration d'imidazole a été ajoutée pour jouer le rôle de compétiteur pour l'interaction avec le Ni²⁺. Cette compétition a induit une élution de la protéine Gag en fusion avec un tag 6-His en Nter et/ou Cter. La purification a été faite par le kit HIS BIND ^{™} RESIN CHROMATOGRAPHY (NOVAGEN), selon les recommandations du fournisseur, par deux méthodes distinctes :
- une méthode en lot (méthode en *batch*) pour de faibles concentrations en protéines, dans le but de mettre au point la condition d'élution optimale : cette méthode consiste à faire la purification dans un micro-tube contenant la matrice ;
- une méthode en colonne, avec une plus grande concentration de protéines : la colonne est branchée à une pompe péristaltique et à un spectrophotomètre UVICORDSII^{™} (AMERSHAM) qui permet d'obtenir un signal lorsqu'une protéine est détectée.

### 13. Extraction des ARNs

Après purification des protéines, une extraction des ARN a été réalisée pour quantifier la proportion d'ARN spécifique de Gag. Pour un volume minimum de 200 µL de *T-bodies* purifiées, a été ajouté un volume de phénol acide / chloroforme. Le mélange obtenu a été mélangé au vortex 1 min puis centrifugé 5 min à 14000 rpm. Le surnageant a été récupéré et un volume de chloroforme est ajouté. Le mélange obtenu a été mélangé au vortex 1 min puis centrifugé 5 min à 14000 rpm. Le surnageant a été récupéré et 1/10 volume 3 M sodium acetate pH 5,2 ainsi que 2,5 volumes (surnageant + sodium acetate) d'éthanol 100 %. Le mélange obtenu a été incubé à -20°C sur la nuit. Le lendemain, le mélange obtenu a été centrifugé 15 min à 14000 rpm à 4°C. Le culot est séché a été remis en suspension dans 20 µL d'eau sans nucléases (*Nuclease Free Water*)*.* Les ARN extraits ont été quantifiés au NANODROP^{™}.

### 14. qRT-PCR

Les ARN extraits ont été soumis à transcription inverse (*reverse transcription ;* RT) pour la production d'ADN complémentaire (ADNc) par le kit REVERTAID RT REVERSE TRANSCRIPTION^{™} (THERMOFISHER SCIENTIFIC), selon les recommandations du fournisseur, en utilisant 500 ng d'ARN par réaction avec les amorces hexamères aléatoires (*random hexamer primer*) du kit. Les ADNc obtenus sont quantifiés au NANODROP^{™}. La qPCR se fait à l'aide du kit QUANTIFFAST SYBR GREEN PCR^{™} (QUIAGEN), selon les recommandations du fournisseur. La gamme étalon a été faite en utilisant le plasmide pBDG1130 de 1µg/ µL à 0,1 pg/µL. Les amorces utilisées : qGAGF (SEQ ID NO : 23) et qGAGR (SEQ ID NO : 24) ont permis l'amplification de 277 pb de GAG. Le programme utilisé pour l'amplification a été le suivant : dans un premier temps une pré-incubation 50°C 2 min, puis une dénaturation de 95°C 10 min, s'ensuivent 40 cycles de 95 °C 15s, 60°C 30s, 72 °C 30 s. Une courbe de fusion a été produite à la fin de l'expérimentation pour vérifier l'absence de contaminants : 95°C 15 s puis 50°C 1 min et 95°C avec des acquisitions tous les 5°C.

### Résultats

### Sélection et modifications de souches de levure (pour une production robuste d'ARNm dans les rétrosomes T (T-bodies))

L'analyse de l'expression de la protéine Gag (49kDa ; SEQ ID NO : 3) sur des échantillons de lysat cellulaire de levures *S. cerevisiae* et *S.paradoxus* confirme que la souche *S. paradoxus* est dépourvue de l'élément de rétrotransposition Ty1 (*cf.* Figure 1A).

La transformation de la levure *S. paradoxus* avec un plasmide codant pour l'eGFP sous le contrôle d'un promoteur inductible au galactose montre que les mutations transcriptionnelles ΔSpt21et/ou ΔSrb2 permettent une augmentation significative du nombre de cellules eGFP positives ainsi que de l'intensité de la fluorescence. Les résultats sont illustrés par la figure 1B.

### Construction de vecteurs d'expression permettant la formation de T-bodies et le ciblage des ARNm EGFP dans les T-bodies

Les analyses par microscopie confocale de fluorescence de l'expression de l'eGFP et de la protéine de fusion Gag-eGFP chez la levure *S. paradoxus* et les mutants transcriptionnels ΔSpt21 et ΔSrb2 ont été réalisées. Les résultats sont illustrés par les figures 2A et 2B. Une augmentation du nombre de cellules positives en *T-bodies* (figure 2A) ainsi qu'une augmentation du nombre de *T-bodies* par cellules chez les mutants transcriptionnels ΔSpt21 et ΔSrb2 sont observées (Figure 2B).

L'analyse par cytométrie en flux de l'expression de *T-bodies* eGFP (protéine de fusion Gag-eGFP) chez le mutant transcriptionnel de la levure *S. paradoxus* ΔSrb2 après un traitement de 8 heures à la tunicamycine (TUNI) a été réalisée (la tunicamycine est un antibiotique produit par les bactéries du genre *Streptomyces* ayant une action sur la N-glycosylation et sur le cycle cellulaire). Les résultats sont illustrés par la figure 2C. Un traitement de 8 heures à la tunicamycine provoque une augmentation du nombre de *T-bodies* (11% vs 48%).

Des expériences d'hybridation *in situ* en fluorescence (*fluorescence in situ hybridization ; FISH*) ont été réalisées pour localiser les ARNm modèles codant la protéine eGFP, par l'utilisation des amorces ciblant l'ARNm eGFP marqué Cy5 sur la levure *S. paradoxus* ΔSrb2 transformée avec un plasmide de fusion Gag-eGFP ou avec un plasmide de fusion MiniGag-eGFP. Les résultats sont illustrés par les figures 3A et 3B.

Une parfaite colocalisation entre la protéine Gag et l'ARNm correspondant est observée (Figure 3A). Une étude a été réalisée pour rechercher la séquence nucléotidique Gag délétée qui permet un ciblage des ARNm dans des *T-bodies.* Cette séquence est dénommée MiniGag en Figure 3B.

### Purification des T-bodies

Le protocole d'extraction des *T-bodies* chez la levure *S. paradoxus* exprimant la protéine de fusion Gag-eGFP après induction au galactose, est basé sur une lyse mécanique des levures suivie par des centrifugations différentielles.

Deux techniques différentes d'extraction mécanique (méthode de lyse par les billes et de sonication) ont été testées. Les résultats sont illustrés par les figures 4A (billes de verre) et 4B (sonication). Après extraction à l'aide de billes de verre (Figure 4A), les *T-bodies* apparaissent comme des particules d'aspect sphérique et d'un diamètre de 200 nm. Par contre, la méthode de lyse par sonication induit une destruction des *T-bodies* avec des débris observables en microscopie électronique (Figure 4B). La méthode de lyse par les billes semble donc être la mieux adaptée pour obtenir le plus de *T-bodies* intègres.

La purification des *T-bodies* a été effectuée sur colonne de nickel grâce à la présence du motif 6 histidines (tag 6His). Trois constructions ont été réalisées, où la protéine Gag dispose d'un tag 6His : - en N-terminal (6His-GAG), - en C-terminal (GAG-6His), - en N et C-terminal (6His-GAG-6His). Ces constructions ont été transformées chez le mutant ΔSrb2 de la levure *S. paradoxus.* La formation des *T-bodies* a été observée par immunofluorescence (Figure 5A). L'expression de la protéine Gag a été vérifiée par transfert de Western (Figure 5B). La purification a été réalisée sur colonne de nickel par compétition avec imidazol. Les résultats sont illustrés par la Figure 5C (élution des *T-bodies* à 800 mM d'imidazole pour la construction GAG-6His).

### EXEMPLE 2 :

Des levures *Saccharomyces* d'espèces autres que *paradoxus* (en l'occurrence, des levures *S. cerevisiae*)*,* et des levures de genres autres que *Saccharomyces* (en l'occurrence, des levures de genre *Pichia*) ont été transformées par les constructions plasmidiques décrites en exemple 1 ci-dessus, à savoir par :
- une construction plasmidique codant la protéine Gag (sous le contrôle d'un promoteur inductible), éventuellement en fusion avec une étiquette de purification (tag 6His) ou avec un marqueur de détection (eGFP) [*cf.* Figure 6A construction i. ; *cf.* Figure 7A], et par
- une construction plasmidique codant un ARN hétérologue d'intérêt lié à une séquence d'adressage rétrosomal (sous le contrôle d'un promoteur inductible), par exemple une construction plasmidique contenant (en insert) un ADN codant un ARNm de eGFP (à titre d'ARN hétérologue) lié (en 3') à une séquence ADN GAG dépourvue du promoteur d'expression de la protéine Gag (à titre de séquence d'adressage rétrosomal) [*cf.* Figure 6A construction ii. ; *cf.* Figure 7B].

Les levures ainsi transformées produisent des rétrosomes contenant l'ARN hétérologue, sans rétro-transcrire cet ARN ni le rétro-intégrer.

Des échantillons de certaines de ces levures ont été déposées auprès de la CNCN en vertu du traité de Budapest.

**Tableau 2 :**

| **Identité de la souche** | **Numéro CNCM** | **Date de dépôt** |
|---|---|---|
| Souche « TBSc1 » : souche *S. cerevisiae,* vierge d'élément Ty (Ty0), et transformée par un plasmide épisomique permettant l'expression d'une protéine de fusion GAG (de Ty1) (SEQ ID NO : 3) et l'eGFP sous l'influence d'un promoteur galactose (SEQ ID NO : 27) (GAL) [Milieu sélectif URA] | I-5176 | 24 février 2017 |

| | | |
|---|---|---|
| CNCM est la Collection Nationale de Culture de Microorganismes (Institut Pasteur ; 28, rue du Dr Roux ; 75724 Paris Cedex 15 ; France). | | |

### EXEMPLE 3 : Transfection (et expression) d'ARN extraits des T-bodies (particules pseudo-virales) dans des cellules dendritiques de mammifère

Des ARN ont été produits comme décrits en exemple 1 et ont été transfectés (par voie liposomique) dans des cellules de mammifère, en l'occurrence des cellules dendritiques. Les expériences de transfection d'ARN ont été réalisées dans des cellules dendritiques de souris (lignée DC2.4, disponible auprès de THERMOFISHER SCIENTIFIC).

Ont été transfectés les ARNm eGFP qui ont été produits chez la levure *S. paradoxus* ΔSrb2 (*cf.* exemple 1 ci-dessus) transformée par :
une construction responsable de la formation de la structure des *T-bodies* (plasmide codant la protéine Gag de SEQ ID NO : 3 ; Figure 6A (i)), et
une construction permettant la transcription d'un ARN eGFP disposant en 3'd'une séquence miniGAG (« EndGAG » ; SEQ ID NO : 14) qui adresse cet ARN eGFP au sein des *T-bodies* (Figure 6A (ii)).

Les ARNm eGFP produits dans les *T-bodies* de cette levure ont été extraits et purifiés comme décrit en exemple 1 ci-dessus (extraction des *T-bodies* par voie mécanique -billes-, puis purification des ARNm eGFP contenus dans ces *T-bodies*)*,* puis vectorisés sur des liposomes cationiques (liposomes 100 ou Lip100). Les liposomes 100 sont composés à part égale, d'un lipide cationique (lipide 1), et d'un colipide neutre pH sensible (lipide 2). Le lipide 1 est un lipide cationique de charge permanente portée par un groupement N-methylimidazolium, pour la complexation des acides nucléiques. Le lipide 2 possède est protonable à pH acide ce qui favorise l'échappement endosomal après déstabilisation de la membrane en milieu acide. Dans ces lipides ce n'est pas un ammonium quaternaire mais un atome de phosphore qui fait le lien entre les chaînes carbonées et le noyau imidazole. Ces lipides sont des lipophosphoramidates dont la structure est inspirée de celles des lipides des membranes qui sont moins toxiques à la fois *in vitro* et *in vivo.* Les liposomes 100 ont notamment été décrits dans Perche *et al.* 2010, Perche *et al.* 2011, Mével *et al.* 2008a, et Mével *et al.* 2008b.

Les cellules dendritiques de souris ont été incubées 20 heures avec ces liposomes, ou bien avec de l'ARN synthétiquement produit *in vitro* à titre de témoin.

Les résultats sont illustrés par la figure 6B. L'ARNm synthétiquement produit *in vitro* à titre de témoin ne donne qu'un très faible taux de transfection 0 à 1,5%, avec une dose de 100 ng d'ARNm eGFP *in vitro* (Figure 6B ; cytogramme de gauche). Par contre, après transfection à l'aide des liposomes contenant l'ARN des *T-bodies,* 32.77% des cellules expriment la protéine fluorescente (Figure 6B ; cytogramme de droite).

Ces résultats illustrent le fait que les ARNm produits par les *T-bodies* de levure de la demande peuvent être transfectés dans une cellule de mammifère, plus particulièrement dans une cellule dendritique, et que la protéine que ces ARNm codent peut être correctement traduite dans cette cellule hôte. Ces résultats viennent soutenir les applications médicales, et notamment vaccinales des ARN produits par ces *T-bodies* (vaccination à ARN).

### EXEMPLE 4 : Etude par spectrométrie de masse des modifications post-transcriptionelle présentent sur des ARNs extraits des T-bodies (particules pseudo-virales)

Une étude en spectrométrie de masse des modifications chimiques présentes au niveau de l'ARN messager modèle luciférase bioproduit comme décrits en exemple 1 a été réalisée après « *pull out* » de l'ARNm luciférase comme suit.

Une première étape d'hybridation est réalisée entre 10 µg ARN extrait des complexes, granules, particules pseudo-virales et 1 µM d'oligonucléotide ADN simple brin biotinylé en 3', complémentaire de la séquence de l'ARN luciférase bioproduit (5'-TCCATCTTCCAGCGGATAGAATGGCGCCGGGCCTTTCTTTATGTTTTTGGCGTC TTCCATAAA 3') en tampon SSC 5X (Chlorure de sodium 750 mM et citrate trisodique 75 mM (ajusté à pH 7,0 avec HCl) dans un volume final 100 µL. Le volume réactionnel est incubé 3 min à 90°C, puis 10 min à 65°C. La réaction d'hybridation est ensuite conservée à température ambiante. Parallèlement des billes magnétiques MyOneT1 Dynabeads greffées avec de la streptavidine sont lavées. 100 µL de billes sont prélevées par pipetage et mélangées au vortex à mi-vitesse. Pour séparer les billes du surnageant les billes sont centrifugées brièvement, puis placées sur un support magnétique pendant 2 min, le surnageant est retiré délicatement (tube toujours sur le support magnétique). Les billes sont resuspendues dans un volume égal de tampon B&W 1X (5 mM Tris-HCl (pH 7,5) 0,5 mM EDTA 1 M NaCl). Cette étape est répétée 3 fois. Un dernier lavage est réalisé en tampon SSC 5X. La réaction d'hybridation est mise en présence des billes mélangées au vortex à mi-vitesse et agitées (600 tr/min) pendant 30 min à 25°C. Les billes sont lavées une fois dans 50 µL de tampon SSC 1X et 3 fois dans 25 µl de SSC 0,1X. Le surnageant est retiré et les billes sont remise en suspension dans 25 µL d'eau MilliQ. Les billes sont chauffées à 75°C pendant 3 min Les billes sont centrifugées brièvement, puis placées sur un support magnétique pendant 2 min, le surnageant est collecté (tube toujours sur le support magnétique) et transféré dans un nouveau tube. Pendant l'étape d'élution, une partie de l'oligonucléotide immobilisé est clivé des billes, il peut être éliminé par digestion par DNase1 (RNase-free, 3,5 U/µL final) pendant 2 heures à 37°C. L'échantillon peut être conservé à -20°C. Le volume de l'échantillon est ramené à 180 µL grâce à l'eau MiliQ, 18 µL de solution d'acétate de sodium 3 M et 2 µL de glycogen (10 mg/mL) sont ajoutés et mélangé au vortex. 600 µL d'éthanol 100% sont ajoutés. La réaction est mélangée au vortex puis incubée à -20°C au moins 1 heure. La réaction est ensuite centrifugée à 10,000 g pendant 30 min à 4°C. Le culot est rincé 2 fois par 200 µL d'éthanol 70% puis séché à température ambiante pendant 5 min. Le culot est ensuite repris en eau MiliQ.

Une étude en spectrométrie de masse des modifications chimiques présentes au niveau de l'ARN messager bioproduits a été réalisée sur cet échantillon. Cette technique repose sur une analyse par spectrométrie de masse (LC-MS) des fragments obtenus après hydrolyse complète par RNase T1 de l'ARN étudié. 1 µg d'ARN est digéré en nucléosides et l'étalon interne (SILIS) est ajouté.

Le chromatogramme résultant montrant des modifications communes de l'ARN eucaryote correspond à la Figure 8. Une vingtaine de modifications de ribonucléosides caractéristiques des eucaryotes sont criblées grâce à cette technique (*cf.* Figure 8A). De nombreuses modifications ne sont plus détectables entre l'ARN avant et après « *pull out* » dans l'ARN purifié (*cf.* Figure 8). Certaines modifications sont détectables après purification « *pull out* » :
- m5U, 5-méthyluridine ;
- m5C, 5-méthylcytidine ;
- m1A, 1-méthyladénosine ;
- Gm, 2'-O-méthylguanosine ;
- M7G, 7-méthylguanosine ;
- Am, 2'-O-méthyladénosine ;
- Cm, 2'-O-méthylcytidine ; et
- m3C, 3-méthylcytidine.

### EXEMPLE 5 : Stabilité de l'expression d'ARN extraits des T-bodies (particules pseudo-virales) dans des cellules dendritiques de mammifère

Des ARNs luciférase produits et modifiés *in vitro* (coiffe ARCA, queue PolyA64+ ou pseudouridine) ont été transfectés dans des cellules dendritiques murines (DC2.4) en parallèle d'un ARN luciférase bioproduit en levure (*cf.* Figure 9) :
▪ Les ARN coiffés sont produits par transcription *in vitro* par la polymérase T7 à partir de matrices d'ADN linéarisées. Le kit de transcription Ambion mMessage mMachine^{™} ULTRA a été utilisé suivant le protocole du fournisseur. Le mélange de transcription contient un analogue de la coiffe m7G(5')ppp(5')G des ARNm (*anti-reverse cap analog* (ARCA)) qui est incorporé par la T7 polymérase en 5' des transcrits.
▪ La queue PolyA64+ est ajoutée *in vitro* à l'aide du réactif E-PAP pour une polyadénylation supplémentaire.
▪ La pseudouridine (N1-Methylpseudouridine-5'-Triphosphate - 100 mM - Trilink) est incorporée au mix réactionnel (1,5 µL de pseudouridine pour 20 µL de volume réactionnel) afin d'incorporer *in vitro* 50% de pseudouridine dans la composition de l'ARN synthétisé.
▪ Les ARN bioproduits l'ont été comme décrit en exemple 1.

50 ng de chacun des ARNs a été vectorisé en utilisant la Lipofectamine Messenger Max et l'activité luciférase dans les cellules transfectées a été mesurée *in vitro.* On observe que l'ARN luciférase bioproduit dans des *T-bodies* (TB) de levures est plus efficace (3 logs de différence) que le plus efficace des ARNs synthétisés *in vitro* (*cf.* Figure 9).

### EXEMPLE 6 : Production pré-industrielle d'ARN extraits des T-bodies

Des ARNs bioproduits en levure ont été obtenus en phase pré-industrielle *via* la mise en œuvre du protocole suivant :
**1**/ 3 pré-cultures (PC) sont réalisées en milieu YNB (6,7g/L) + CSM -URA-LEU (0,79 g/L) + Glucose (20 g/L) stérilisé à l'autoclave à 25°C sous agitation (230 rpm) comme suit :
   a. PC1 : 5 mL à 25°C pendant 24h à partir d'un clone isolé ;
   b. PC2 : 100 mL (3,5 mL PC1 + 96,5 mL de milieu) à 25°C pendant 24h ; puis
   c. PC3 : 400 mL (inoculation à DO₆₀₀ₙₘ 0,1 à partir de la PC2) à 25°C pendant 24h.
**2**/ Un fermenteur (capacité 5 L) est inoculé à DO 0,2 (à partir de la PC3) en milieu YNB (6,7 g/L) + CSM -URA-LEU (0,79 g/L) + Glucose (20 g/L). Un anti-mousse type EX-CELL^{®} Antifoam est ajouté : pulse 0,5 % (v/v) en fin de journée. L'agitation du fermenteur est fixée à 1000 rpm en « local » puis passage en « remote » lorsque la valeur de la pO₂ est 10 % en dessous de la valeur de consigne. Le pH est maintenu à 5,4 et ajusté à partir de solutions d'acide phosphorique (1,2 M) et d'ammoniac 20 % stériles.
**3**/ En parallèle, un *feeding* galactose est réalisé. Le déclenchement de ce *feeding* galactose est programmé sur le pic pO₂ avec comme consigne pO₂ : 40 %. La remontée en pO₂ étant dépendante de la consommation totale du glucose. Ce *feeding* en galactose est réalisé à partir d'une solution à 20 % galactose stérile pour une concentration finale à 2 %. La durée de l'induction est fixée à 24h.
4/ Passé ces 24h, le volume de culture est collecté et centrifugé à 4000 rpm pendant 15 min.
5/ Les *T-bodies* sont extraits décrit l'exemple 1.
6/ Les ARNs bioproduits en levure sont extraits des T-bodies comme décrit dans l'exemple 1.

### RÉFÉRENCES BIBLIOGRAPHIQUES

Mével et al. 2008a Synthesis and transfection activity of new cationic phosporamidate lipids : high efficiency of an imidazolium derivative. ChemBioChem 6: 1462-1471

Mével et al. 2008b Novel neutral imidazole-lipophosphoramides for transfection assays. Chem Commun. 3124-316.

Perche et al. 2010 Selective gene delivery in dendritic cells with mannosylated and histidylated lipopolyplexes. Journal of Drug Targeting 1-11

Perche et al. 2011 Enhancement of dendritic cells transfection in vivo and of vaccination against B16F10 melanoma with mannosylated hitidylated lipopolyplexes loaded with tumor antigen messenger RNA. Nanomedicine 7(4): 445-453.

Rothstein et al. 1983 One-step gene disruption in yeast. Methods Enzymol. 101:202-211

Thomas et al. 1989 Elevated recombination rates in transcriptionally active DNA. Cell 56 :619-630

WO 2010/084371 au nom de MITOPROD

WO 2011/128444 au nom de EUKARYS

## Revendications

1. Un **procédé** de production recombinante d'ARN messager (ARNm) ou d'ARN long non-codant (ARNlnc), qui comprend :
- le fait de cultiver des cellules de levure, qui ont été modifiées génétiquement pour produire des particules pseudo-virales recombinantes qui comprennent ledit ARNm ou ARNlnc, et
- le fait de collecter l'ARNm ou ARNlnc qui est contenu dans les particules pseudo-virales ainsi formées,
dans lequel les cellules de levure sont des cellules d'une levure qui est naturellement dépourvue de rétrotransposon Ty, ou qui a été modifiée génétiquement pour ne pas produire de rétrosome T,
dans lequel les modifications génétiques apportées à ces cellules de levure pour qu'elles produisent lesdites particules pseudo-virales comprennent la transfection de ces cellules de levure par
i. un acide nucléique comprenant une première séquence nucléotidique, et
ii. un acide nucléique comprenant une deuxième séquence nucléotidique,
dans lequel :
- l'acide nucléique de i. et l'acide nucléique de ii. sont une seule et même molécule, ou bien deux molécules distinctes ou séparées,
- la première séquence nucléotidique comprend une séquence ADN qui code l'expression de la protéine Gag d'un rétrotransposon Ty de levure,
- la deuxième séquence nucléotidique comprend une séquence ADN dont le transcrit ARN est :
a. la séquence dudit ARNm ou ARNlnc, liée à
b. une séquence d'adressage qui comprend un fragment de la séquence ARN dont la traduction en acides aminés est la séquence de ladite protéine Gag de rétrotransposon Ty de levure, ce fragment étant d'au moins 150 nucléotides et ne comprenant pas le codon départ de traduction de cette séquence ARN, et
la séquence ADN de cette deuxième séquence nucléotidique ne présentant pas de cadre de lecture dont le premier codon serait le codon ATG,
- l'acide nucléique de ii. ne comprend pas de séquence ADN dont le transcrit ARN serait la séquence ARN complète de ladite protéine Gag de rétrotransposon Ty de levure,
- la protéine Gag de rétrotransposon Ty de levure est une protéine Gag de rétrotransposon Ty1, Ty2 ou Ty3 de levure, et la séquence de cette protéine Gag est la séquence de SEQ ID NO : 3 ou une séquence qui est identique à au moins 90% à la séquence de SEQ ID NO : 3, et
- l'acide nucléique de i. et l'acide nucléique de ii. ne comprennent pas de séquence codant la transcriptase inverse d'un rétrotransposon Ty1 de levure,
l'acide nucléique de i. et l'acide nucléique de ii. codant ainsi la formation, dans lesdites cellules de levure, de particules pseudo-virales qui sont formées par la protéine Gag traduite à partir de la première séquence nucléotidique, et qui encapsulent ledit ARNm ou ARNlnc transcrit à partir de la deuxième séquence nucléotidique.

2. Le procédé de la revendication 1, dans lequel ledit fragment de séquence ARN, qui est compris dans ladite séquence d'adressage b., est la séquence de SEQ ID NO : 16 ou un fragment d'au moins 150 nucléotides de la séquence de SEQ ID NO : 16.

3. Le procédé de la revendication 1 ou 2, dans lequel la levure est une levure de genre *Saccharomyces* ou *Pichia,* plus particulièrement une levure de l'espèce *Saccharomyces paradoxus, Saccharomyces cerevisiae,* ou *Pichia pastoris.*

4. Le procédé de l'une quelconque des revendications 1 à 3, dans lequel la levure est une levure dont un ou plusieurs des gènes *rpb1, spt21, srb2* et *srb5* ont été mutés par une ou plusieurs mutations qui comprennent une ou plusieurs mutations choisies parmi :
- le remplacement d'un ou plusieurs codons chacun par un autre codon ou par un autre triplet de nucléotides, et
- la délétion d'un ou plusieurs nucléotides,
et qui stimulent ou augmentent la production de particules pseudo-virales.

5. Le procédé de l'une quelconque des revendications 1 à 4, dans lequel l'ARNm ou ARNlnc comprend au moins 120 nucléotides et est hétérologue à ladite levure, et dans lequel l'ARNm ou ARNlnc optionnellement code :
- une protéine ou un polypeptide de bactérie,
- une enzyme d'édition génomique,
- une protéine ou un polypeptide de virus,
- un facteur de transcription humain,
- un facteur de croissance humain,
- une protéine CFTR humaine,
- un anticorps ou fragment d'anticorps,
- un polypeptide comprenant au moins un épitope d'antigènes.

6. **Un kit** qui est spécialement adaptée à la mise en œuvre du procédé selon l'une quelconque des revendications 1 à 5, **caractérisé en ce que** le kit comprend au moins un acide nucléique comprenant une première séquence nucléotidique et au moins un acide nucléique comprenant une deuxième séquence nucléotidique, dans laquelle :
- la première séquence nucléotidique comprend une séquence ADN qui code l'expression de la protéine Gag d'un rétrotransposon Ty de levure,
- la protéine Gag de rétrotransposon Ty de levure est une protéine Gag de rétrotransposon Ty1, Ty2 ou Ty3 de levure, et la séquence de cette protéine Gag est la séquence de SEQ ID NO : 3 ou une séquence qui est identique à au moins 90% à la séquence de SEQ ID NO : 3, et
- la deuxième séquence nucléotidique comprend une séquence ADN qui comprend la séquence de SEQ ID NO : 16 ou un fragment d'au moins 150 nucléotides de la séquence de SEQ ID NO : 16, la séquence ADN de cette deuxième séquence nucléotidique ne comprenant pas de cadre de lecture dont le premier codon serait le codon ATG,
- l'acide nucléique qui comprend la deuxième séquence nucléotidique ne comprend pas de séquence ADN dont le transcrit ARN serait la séquence ARN complète de ladite protéine Gag de rétrotransposon Ty de levure, et
- l'acide nucléique qui comprend la première séquence nucléotidique et l'acide nucléique qui comprend la deuxième séquence nucléotidique sont une seule et même molécule, ou bien deux molécules distinctes ou séparées,
ledit kit ne comprenant pas de transcriptase inverse de rétrotransposon Ty de levure, ni d'acide nucléique codant une telle transcriptase inverse, ni d'acide nucléique comprenant une séquence codant une telle transcriptase inverse, et
ledit kit pouvant en outre optionnellement comprendre des cellules d'une levure qui est naturellement dépourvue de rétrotransposon Ty, ou qui a été modifiée génétiquement pour ne pas produire de rétrosome T.

7. **Une souche recombinante de levure** qui est spécialement adaptée à la mise en œuvre du procédé selon l'une quelconque des revendications 1 à 5, **caractérisée en ce que** :
- la souche de levure est dépourvue de rétrotransposon Ty1, Ty2 et Ty3, et
- la souche de levure ne comprend pas de transcriptase inverse de rétrotransposon Ty de levure, ni d'acide nucléique codant une telle transcriptase inverse, ni d'acide nucléique comprenant une séquence codant une telle transcriptase inverse,
- la souche de levure a été génétiquement modifiée pour comprendre au moins un acide nucléique comprenant une première séquence nucléotidique et au moins un acide nucléique comprenant une deuxième séquence nucléotidique, dans laquelle :
- la première séquence nucléotidique comprend une séquence ADN qui code l'expression de la protéine Gag d'un rétrotransposon Ty de levure,
- la protéine Gag de rétrotransposon Ty de levure est une protéine Gag de rétrotransposon Ty1, Ty2 ou Ty3 de levure, et la séquence de cette protéine Gag est la séquence de SEQ ID NO : 3 ou une séquence qui est identique à au moins 90% à la séquence de SEQ ID NO : 3,
- la deuxième séquence nucléotidique comprend une séquence ADN dont le transcrit ARN comprend la séquence de SEQ ID NO : 16 ou un fragment d'au moins 150 nucléotides de la séquence de SEQ ID NO : 16, et la séquence ADN de cette deuxième séquence nucléotidique ne comprenant pas de cadre de lecture dont le premier codon serait le codon ATG,
- l'acide nucléique qui comprend la deuxième séquence nucléotidique ne comprend pas de séquence ADN dont le transcrit ARN serait la séquence ARN complète de ladite protéine Gag de rétrotransposon Ty de levure, et
- l'acide nucléique qui comprend la première séquence nucléotidique et l'acide nucléique qui comprend la deuxième séquence nucléotidique sont une seule et même molécule, ou bien deux molécules distinctes ou séparées.

8. **Une particule pseudo-virale** recombinante, qui est susceptible d'être obtenue par collecte d'une particule pseudo-virale produite lors de la mise en œuvre du procédé de l'une quelconque des revendications 1 à 5, **caractérisée en ce que** la particule pseudo-virale recombinante comprend un ARN encapsulé par un polymère protéique, dans laquelle :
- le polymère protéique comprend la protéine Gag d'un rétrotransposon Ty de levure,
- la protéine Gag de rétrotransposon Ty de levure est une protéine Gag de rétrotransposon Ty1, Ty2 ou Ty3 de levure, et la séquence de cette protéine Gag est la séquence de SEQ ID NO : 3 ou une séquence qui est identique à au moins 90% à la séquence de SEQ ID NO : 3,
- la séquence nucléotidique de l'ARN encapsulé comprend la séquence de SEQ ID NO : 16 ou un fragment d'au moins 150 nucléotides de la séquence de SEQ ID NO : 16, mais ne comprend pas de cadre de lecture dont le premier codon serait le codon ATG, et ne comprend pas la séquence ARN qui est le transcrit ARN de la SEQ ID NO : 2, et
- la particule pseudo-virale VLP ne comprend ni transcriptase inverse, ni acide nucléique codant une transcriptase inverse, ni acide nucléique comprenant une séquence codant une telle transcriptase inverse.

9. **Utilisation *in vitro*** du kit de la revendication 6, ou de la souche recombinante de levure de la revendication 7, ou de la particule pseudo-virale recombinante de la revendication 8, pour la production d'ARNm ou ARNlnc présentant une queue polyA en 3' et/ou une coiffe 7-méthylguanosine en 5'.

10. Un procédé pour la production d'une composition pharmaceutique qui comprend au moins un ARNm ou ARNlnc, ledit procédé comprenant le fait de produire un ARNm ou ARNlnc par le procédé de l'une quelconque des revendications 1 à 5, et le fait de placer cet ARNm ou ARNlnc dans un véhicule pharmaceutiquement acceptable pour produire une composition pharmaceutique, cette composition pharmaceutique étant optionnellement destinée à :
- la prévention ou au traitement d'une infection microbiologique,
- la prévention ou au traitement d'une prolifération tumorale,
- la prévention ou au traitement d'une maladie chronique,
- une thérapie par régénération tissulaire ou cellulaire, ou
- une thérapie génique.

## Patentansprüche

1. Verfahren zur rekombinanten Herstellung von Messenger-RNA (mRNA) oder von langer nicht-codierender RNA (lncRNA), das umfasst:
- Kultivieren von Hefezellen, die genetisch verändert wurden, um rekombinante pseudovirale Partikel zu produzieren, die die mRNA oder lncRMA umfassen, und
- Sammeln der mRNA oder lncRNA, die in den so gebildeten pseudoviralen Partikeln enthalten ist,
wobei die Hefezellen Zellen einer Hefe sind, die natürlich frei von Retrotransposon Ty ist, oder die genetisch verändert wurde, um kein Retrosom T zu produzieren,
wobei die genetischen Veränderungen, die an diesen Hefezellen vorgenommen werden, damit sie die pseudoviralen Partikel produzieren, die Transfektion dieser Hefezellen umfassen durch:
i. eine Nukleinsäure, die eine erste Nukleotidsequenz umfasst, und
ii. eine Nukleinsäure, die eine zweite Nukleotidsequenz umfasst,
wobei:
- die Nukleinsäure von i. und die Nukleinsäure von ii. ein und dasselbe Molekül oder zwei verschiedene oder getrennte Moleküle sind,
- die erste Nukleotidsequenz eine DNA-Sequenz umfasst, die die Expression des Proteins Gag eines Hefe-Retrotransposons Ty codiert,
- die zweite Nukleotidsequenz eine DNA-Sequenz umfasst, deren RNA-Transkript ist:
a. die Sequenz der mRNA oder lncRNA, verbunden mit
b. eine Adressierungssequenz, die ein Fragment der RNA-Sequenz umfasst, deren Übersetzung in Aminosäuren die Sequenz des Proteins Gag des Hefe-Retrotransposons Ty ist, wobei dieses Fragment mindestens 150 Nukleotide aufweist und kein Übersetzungs-Startcodon dieser RNA-Sequenz umfasst, und
die DNA-Sequenz dieser zweiten Nukleotidsequenz keinen Leserahmen aufweist, dessen erstes Codon das Codon ATG ist,
- die Nukleinsäure von ii. keine DNA-Sequenz umfasst, deren RNA-Transkript die vollständige RNA-Sequenz des Proteins Gag des Hefe-Retrotransposons Ty ist,
- das Protein Gag des Hefe-Retrotransposons Ty ein Protein Gag des Hefe-Retrotransposons Ty1, Ty2 oder Ty3 ist und die Sequenz dieses Proteins Gag die Sequenz mit der SEQ ID NR: 3 oder eine Sequenz ist, die zu mindestens 90 % identisch mit der SEQ ID NR: 3 ist, und
- die Nukleinsäure von i. und die Nukleinsäure von ii. keine Sequenz umfassen, die die reverse Transkriptase eines Hefe-Retrotransposons Ty1 codiert, wobei die Nukleinsäure von i. und die Nukleinsäure von ii. somit die Bildung von pseudoviralen Partikeln, die aus dem ausgehend von der ersten Nukleotidsequenz übersetzten Protein Gag gebildet sind und die die ausgehend von der zweiten Nukleotidsequenz transkribierte mRNA oder lncRNA einkapseln, in den Hefezellen codieren.

2. Verfahren nach Anspruch 1, wobei das Fragment der RNA-Sequenz, das in der Adressierungssequenz b. enthalten ist, die Sequenz mit der SEQ ID NR: 16 oder ein Fragment von mindestens 150 Nukleotiden der Sequenz mit der SEQ ID NR: 16 ist.

3. Verfahren nach Anspruch 1 oder 2, wobei die Hefe eine Hefe der Gattung *Saccharomyces* oder *Pichia,* insbesondere eine Hefe der Art Saccharomyces paradoxus, *Saccharomyces cerevisiae* oder *Pichia pastoris* ist.

4. Verfahren nach einem der Ansprüche 1 bis 3, wobei die Hefe eine Hefe ist, von der eines oder mehrere der Gene *rpb1, spt21, srb2* und *srb5* durch eine oder mehrere Mutationen mutiert wurden, die eine oder mehrere Mutationen umfassen, die ausgewählt sind aus:
- der Ersetzung von einem oder mehreren Codons, jeweils durch ein anderes Codon oder durch ein anderes Nukleotid-Triplett, und
- der Löschung von einem oder mehreren Nukleotiden, und die die Produktion pseudoviraler Partikel stimulieren oder erhöhen.

5. Verfahren nach einem der Ansprüche 1 bis 4, wobei die mRNA oder lncRNA mindestens 120 Nukleotide umfasst und heterolog zu der Hefe ist, und wobei die mRNA oder die lncRNA wahlweise codiert:
- ein Bakterienprotein oder -polypeptid,
- ein Enzym zur Genomeditierung,
- ein Virenprotein oder -polypeptid,
- einen humanen Transkriptionsfaktor,
- einen humanen Wachstumsfaktor,
- ein humanes CFTR-Protein,
- einen Antikörper oder ein Antikörperfragment,
- ein Polypeptid, das mindestens ein Epitop von Antigenen umfasst.

6. Kit, das eigens an die Durchführung des Verfahrens nach einem der Ansprüche 1 bis 5 angepasst ist, **dadurch gekennzeichnet, dass** das Kit mindestens eine Nukleinsäure, die eine erste Nukleotidsequenz umfasst, und mindestens eine Nukleinsäure umfasst, die eine zweite Nukleotidsequenz umfasst, wobei:
- die erste Nukleotidsequenz eine DNA-Sequenz umfasst, die die Expression des Proteins Gag eines Hefe-Retrotransposons Ty codiert,
- das Protein Gag des Hefe-Retrotransposons Ty ein Protein Gag des Hefe-Retrotransposons Ty1, Ty2 oder Ty3 ist und die Sequenz dieses Proteins Gag die Sequenz mit der SEQ ID NR: 3 oder eine Sequenz ist, die zu mindestens 90 % identisch mit der SEQ ID NR: 3 ist, und
- die zweite Nukleotidsequenz eine DNA-Sequenz umfasst, die die Sequenz mit der SEQ ID NR: 16 oder ein Fragment von mindestens 150 Nukleotiden der Sequenz mit der SEQ ID NR: 16 umfasst, wobei die DNA-Sequenz dieser zweiten Nukleotidsequenz keinen Leserahmen umfasst, dessen erstes Codon das Codon ATG ist,
- die Nukleinsäure, die die zweite Nukleotidsequenz umfasst, keine DNA-Sequenz umfasst, deren RNA-Transkript die vollständige RNA-Sequenz des Proteins Gag des Hefe-Retrotransposons Ty ist, und
- die Nukleinsäure, die die erste Nukleotidsequenz umfasst, und die Nukleinsäure, die die zweite Nukleotidsequenz umfasst, ein und dasselbe Molekül oder zwei verschiedene oder getrennte Moleküle sind,
wobei das Kit weder reverse Transkriptase des Hefe-Retrotransposons Ty noch Nukleinsäure, die eine solche reverse Transkriptase codiert, noch Nukleinsäure umfasst, die eine Sequenz umfasst, die eine solche reverse Transkriptase codiert, und
das Kit ferner wahlweise Zellen einer Hefe umfassen kann, die natürlich frei von Retrotransposon Ty ist, oder die genetisch verändert wurde, um kein Retrosom T zu produzieren.

7. Rekombinanter Hefestamm, der eigens an die Durchführung des Verfahrens nach einem der Ansprüche 1 bis 5 angepasst ist, **dadurch gekennzeichnet, dass**:
- der Hefestamm kein Retrotransposon Ty1, Ty2 und Ty3 aufweist, und
- der Hefestamm weder reverse Transkriptase des Hefe-Retrotransposons Ty noch Nukleinsäure, die eine solche reverse Transkriptase codiert, noch Nukleinsäure umfasst, die eine Sequenz umfasst, die eine solche reverse Transkriptase codiert,
- der Hefestamm genetisch verändert wurde, um mindestens eine Nukleinsäure, die eine erste Nukleotidsequenz umfasst, und mindestens eine Nukleinsäure zu umfassen, die eine zweite Nukleotidsequenz umfasst, wobei:
- die erste Nukleotidsequenz eine DNA-Sequenz umfasst, die die Expression des Proteins Gag eines Hefe-Retrotransposons Ty codiert,
- das Protein Gag des Hefe-Retrotransposons Ty ein Protein Gag des Hefe-Retrotransposons Ty1, Ty2 oder Ty3 ist und die Sequenz dieses Proteins Gag die Sequenz mit der SEQ ID NR: 3 oder eine Sequenz ist, die zu mindestens 90 % identisch mit der SEQ ID NR: 3 ist,
- die zweite Nukleotidsequenz eine DNA-Sequenz umfasst, deren RNA-Transkript die Sequenz mit der SEQ ID NR: 16 oder ein Fragment von mindestens 150 Nukleotiden der Sequenz mit der SEQ ID NR: 16 umfasst, und die DNA-Sequenz dieser zweiten Nukleotidsequenz keinen Leserahmen umfasst, dessen erstes Codon das Codon ATG ist,
- die Nukleinsäure, die die zweite Nukleotidsequenz umfasst, keine DNA-Sequenz umfasst, deren RNA-Transkript die vollständige RNA-Sequenz des Proteins Gag des Hefe-Retrotransposons Ty ist, und
- die Nukleinsäure, die die erste Nukleotidsequenz umfasst, und die Nukleinsäure, die die zweite Nukleotidsequenz umfasst, ein und dasselbe Molekül oder zwei verschiedene oder getrennte Moleküle sind.

8. Rekombinantes pseudovirales Partikel, das durch Sammeln eines pseudoviralen Partikels erhalten werden kann, das bei der Durchführung des Verfahrens nach einem der Ansprüche 1 bis 5 hergestellt wird, **dadurch gekennzeichnet, dass** das rekombinante pseudovirale Partikel eine RNA umfasst, die durch ein Proteinpolymer eingekapselt ist, wobei:
- das Proteinpolymer das Protein Gag eines Hefe-Retrotransposons Ty umfasst,
- das Protein Gag des Hefe-Retrotransposons Ty ein Protein Gag des Hefe-Retrotransposons Ty1, Ty2 oder Ty3 ist und die Sequenz dieses Proteins Gag die Sequenz mit der SEQ ID NR: 3 oder eine Sequenz ist, die zu mindestens 90 % identisch mit der SEQ ID NR: 3 ist,
- die Nukleotidsequenz der eingekapselten RNA die Sequenz mit der SEQ ID NR: 16 oder ein Fragment von mindestens 150 Nukleotiden der Sequenz mit der SEQ ID NR: 16 umfasst, aber keinen Leserahmen umfasst, dessen erstes Codon das Codon ATG ist, und nicht die RNA-Sequenz umfasst, die das RNA-Transkript mit der SEQ ID NR: 2 ist, und
- das pseudovirale Partikel VLP weder reverse Transkriptase, noch Nukleinsäure, die eine reverse Transkriptase codiert, noch Nukleinsäure umfasst, die eine Sequenz umfasst, die eine solche reverse Transkriptase codiert.

9. In-vitro-Nutzung des Kits nach Anspruch 6 oder des rekombinanten Hefestamms nach Anspruch 7 oder des rekombinanten pseudoviralen Partikels nach Anspruch 8 für die Herstellung von mRNA oder lncRNA, die einen polyA-Schwanz am 3'-Ende und/oder eine 7-Methylguanosin-Kappe am 5'-Ende aufweist.

10. Verfahren zur Herstellung einer pharmazeutischen Zusammensetzung, die mindestens eine mRNA oder lncRNA umfasst, wobei das Verfahren das Herstellen einer mRNA oder lncRNA durch das Verfahren nach einem der Ansprüche 1 bis 5 und das Platzieren dieser mRNA oder lncRNA in einem pharmazeutisch annehmbaren Vehikel zur Herstellung einer pharmazeutischen Zusammensetzung umfasst, wobei diese pharmazeutische Zusammensetzung wahlweise bestimmt ist:
- zur Prävention oder zur Behandlung einer mikrobiologischen Infektion,
- zur Prävention oder zur Behandlung einer Tumorproliferation,
- zur Prävention oder zur Behandlung einer chronischen Erkrankung,
- für eine Therapie durch Gewebe- oder Zellregeneration, oder
- für eine Gentherapie.

## Claims

1. A method for the recombinant production of messenger RNA (mRNA) or non-coding long-chain RNA (lncRNA), which comprises:
- culturing yeast cells, which have been genetically modified to produce recombinant virus-like particles which comprise said mRNA or lncRNA, and
- collecting the mRNA or lncRNA which is contained in the virus-like particles thus formed,
wherein the yeast cells are cells of a yeast which is naturally free of Ty retrotransposon or which has been genetically modified not to produce a T-retrosome,
wherein the genetic modifications made to these yeast cells to produce said virus-like particles comprise transfection of these said yeast cells by
i. a nucleic acid comprising a first nucleotide sequence, and
ii. a nucleic acid comprising a second nucleotide sequence,
wherein:
- the nucleic acid of i. and the nucleic acid of ii. are the same molecule, or are two distinct or separate molecules,
- the first nucleotide sequence comprises a DNA sequence which encodes the expression of the Gag protein of a yeast Ty retrotransposon,
- the second nucleotide sequence comprises a DNA sequence whose RNA transcript is:
a. the sequence of said mRNA or lncRNA linked to
b. an addressing sequence which comprises a fragment of the RNA sequence whose translation into amino acids is the sequence of said Gag protein of a yeast Ty retrotransposon, this fragment being at least 150 nucleotides and comprising no translation start codon of this RNA sequence, and
the DNA sequence of this second nucleotide sequence having no reading frame, the first codon of which would be the ATG codon,
- the nucleic acid of ii. does not comprise a DNA sequence whose RNA transcript would be the complete RNA sequence of said Gag protein of a yeast Ty retrotransposon,
- the Gag protein of a yeast Ty retrotransposon is a Gag protein of a yeast Ty1, Ty2 or Ty3 retrotransposon, and the sequence of this Gag protein is the sequence of SEQ ID NO: 3 or a sequence which is at least 90% identical to the sequence of SEQ ID NO: 3, and
- the nucleic acid of i. and the nucleic acid of ii. do not comprise a sequence encoding the reverse transcriptase of a yeast Ty1 retrotransposon,
the nucleic acid of i. and the nucleic acid of ii. thereby encoding the formation, in said yeast cells, of virus-like particles which are formed by the Gag protein translated from the first nucleotide sequence, and which encapsulate said mRNA or lncRNA transcribed from the second nucleotide sequence.

2. The method of claim 1, wherein said RNA sequence fragment, which is comprised in said b. addressing sequence, is the sequence of SEQ ID NO: 16 or a fragment of at least 150 nucleotides of the sequence of SEQ ID NO: 16.

3. The method of claim 1 or 2, wherein the yeast is a yeast of the genus *Saccharomyces* or *Pichia,* more particularly a yeast of the species *Saccharomyces paradoxus, Saccharomyces cerevisiae,* or *Pichia pastoris.*

4. The method of any one of claims 1 to 3, wherein the yeast is a yeast of which one or more of the *rpb1, spt21, srb2* and *srb5* genes have been mutated by one or more mutations that comprise one or more mutations selected from:
- replacing one or more codons each with another codon or another nucleotide triplet, and
- deleting one or more nucleotides,
and which stimulate or increase the production of virus-like particles.

5. The method of any one of claims 1 to 4, wherein the mRNA or lncRNA comprises at least 120 nucleotides and is heterologous to said yeast, and wherein the mRNA or lncRNA optionally encodes:
- a bacterial protein or polypeptide,
- a genomic editing enzyme,
- a virus protein or polypeptide,
- a human transcription factor,
- a human growth factor,
- a human CFTR protein,
- an antibody or antibody fragment,
- a polypeptide comprising at least one antigen epitope.

6. A kit which is specially adapted for carrying out the method according to any one of claims 1 to 5, **characterized in that** the kit comprises at least one nucleic acid comprising a first nucleotide sequence and at least one nucleic acid comprising a second nucleotide sequence, wherein:
- the first nucleotide sequence comprises a DNA sequence which encodes the expression of the Gag protein of a yeast Ty retrotransposon,
- the Gag protein of a yeast Ty retrotransposon is a Gag protein of a yeast Ty1, Ty2 or Ty3 retrotransposon, and the sequence of this Gag protein is the sequence of SEQ ID NO: 3 or a sequence which is at least 90% identical to the sequence of SEQ ID NO: 3, and
- the second nucleotide sequence comprises a DNA sequence which comprises the sequence of SEQ ID NO: 16 or a fragment of at least 150 nucleotides of the sequence of SEQ ID NO: 16, wherein the DNA sequence of this second nucleotide sequence does not comprise a reading frame, the first codon of which is the ATG codon,
- the nucleic acid which comprises the second nucleotide sequence does not comprise a DNA sequence whose RNA transcript would be the complete RNA sequence of said Gag protein of a yeast Ty retrotransposon, and
- the nucleic acid which comprises the first nucleotide sequence and the nucleic acid which comprises the second nucleotide sequence are the same molecule, or are two distinct or separate molecules,
said kit comprising no reverse transcriptase of a yeast Ty retrotransposon, nor a nucleic acid encoding such a reverse transcriptase, nor a nucleic acid comprising a sequence encoding such a reverse transcriptase, and
said kit may further optionally comprise cells of a yeast which is naturally free of a Ty retrotransposon, or that has been genetically modified not to produce a T-retrosome.

7. A recombinant yeast strain which is specially adapted for carrying out the method according to any one of claims 1 to 5, **characterized in that**:
- the yeast strain is devoid of Ty1, Ty2 and Ty3 retrotransposon, and
- the yeast strain comprises no reverse transcriptase of a yeast Ty retrotransposon, nor a nucleic acid encoding such a reverse transcriptase, nor a nucleic acid comprising a sequence encoding such a reverse transcriptase,
- the yeast strain has been genetically modified to comprise at least one nucleic acid comprising a first nucleotide sequence and at least one nucleic acid comprising a second nucleotide sequence, wherein:
- the first nucleotide sequence comprises a DNA sequence which encodes the expression of the Gag protein of a yeast Ty retrotransposon,
- the Gag protein of a yeast Ty retrotransposon is a Gag protein of a yeast Ty1, Ty2 or Ty3 retrotransposon, and the sequence of this Gag protein is the sequence of SEQ ID NO: 3 or a sequence which is at least 90% identical to the sequence of SEQ ID NO: 3,
- the second nucleotide sequence comprises a DNA sequence whose RNA transcript comprises the sequence of SEQ ID NO: 16 or a fragment of at least 150 nucleotides of the sequence of SEQ ID NO: 16, and the DNA sequence of this second nucleotide sequence does not comprise a reading frame, the first codon of which would be the ATG codon,
- the nucleic acid which comprises the second nucleotide sequence does not comprise a DNA sequence whose RNA transcript would be the complete RNA sequence of said Gag protein of a yeast Ty retrotransposon, and
- the nucleic acid which comprises the first nucleotide sequence and the nucleic acid which comprises the second nucleotide sequence are the same molecule, or are two distinct or separate molecules.

8. A recombinant virus-like particle, which can be obtained by collecting a virus-like particle produced during the implementation of the method of any one of claims 1 to 5, **characterized in that** the recombinant virus-like particle comprises an RNA encapsulated by a protein polymer, wherein:
- the protein polymer comprises the Gag protein of a yeast Ty retrotransposon,
- the Gag protein of a yeast Ty retrotransposon is a Gag protein of a yeast Ty1, Ty2 or Ty3 retrotransposon, and the sequence of this Gag protein is the sequence of SEQ ID NO: 3 or a sequence which is at least 90% identical to the sequence of SEQ ID NO: 3,
- the nucleotide sequence of the encapsulated RNA comprises the sequence of SEQ ID NO: 16 or a fragment of at least 150 nucleotides of the sequence of SEQ ID NO: 16, but does not comprise a reading frame whose first codon would be the ATG codon, and does not comprise the sequence of this RNA, which is the RNA transcript of SEQ ID NO: 2, and
- the virus-like particle VLP comprises neither a reverse transcriptase, nor a nucleic acid encoding a reverse transcriptase, nor a nucleic acid comprising a sequence encoding such a reverse transcriptase.

9. The *in vitro* use of the kit of claim 6, or of the recombinant yeast strain of claim 7, or of the recombinant virus-like particle of claim 8, for the production of mRNA or lncRNA having a 3' polyA tail and/or a 5' 7-methylguanosine cap.

10. A method for producing a pharmaceutical composition which comprises at least one mRNA or lncRNA, said method comprising producing an mRNA or lncRNA by the method of any one of claims 1 to 5, and placing this mRNA or lncRNA in a pharmaceutically acceptable carrier to produce a pharmaceutical composition, this pharmaceutical composition optionally being intended for:
- the prevention or treatment of a microbiological infection,
- the prevention or treatment of tumor growth,
- the prevention or treatment of a chronic disease,
- tissue or cell regeneration therapy, or
- a gene therapy.


### SEQUENCE LISTING

<110> Centre National de la Recherche scientifique université d'Orléans
<120> production d'ARN par des levures à particules pseudo-virales recombinantes
<130> WOB TBOD
<150> FR/1752309
   <151> 2017-03-21
<160> 44
<170> PatentIn version 3.5
<210> 1
   <211> 1356
   <212> DNA
   <213> Saccharomyces cerevisiae
<400> 1
   tgatagatct gactagtatg gaatcccaac aattatctaa ttacccacat atatctcatg      60
   gtagcgcctg tgcttcggtt acttctaagg aagtccacac aaatcaagat ccgttagacg     120
   tttcagcttc caaaattcaa gaatatgata aggcttccac taaggctaac tctcaacaga     180
   caacaacacc tgcttcatca gctgttccag agaaccccca tcatgcctct cctcaacctg     240
   cttcagtacc acctccacag aatgggccgt acccacagca gtgcatgatg acccaaaacc     300
   aagccaatcc atctggttgg tcattttacg gacacccatc tatgattccg tatacacctt     360
   atcaaatgtc gcctatgtac tttccacctg ggccacaatc acagtttccg cagtatccat     420
   catcagttgg aacgcctctg agcactccat cacctgagtc aggtaataca tttactgatt     480
   catcctcagc ggactctgat atgacatcca ctaaaaaata tgtcagacca ccaccaatgt     540
   taacctcacc taatgacttt ccaaattggg ttaaaacata catcaaattt ttacaaaact     600
   cgaatctcgg tggtattatt ccgacagtaa acggaaaacc cgtacgtccg atcactgatg     660
   atgaactcac cttcttgtat aacgcttttc aaatatttgc tccctctcaa ttcctaccta     720
   cctgggtcaa agacatccta tccgttgatt atacggatat catgaaaatt ctttccaaaa     780
   gtattgaaaa aatgcaatct gatacccaag aggcaaacga cattgtgacc ctggcaaatt     840
   tgcaatataa tggcagtaca cctgcagatg catttgaaac aaaagtcaca aacattatcg     900
   acagactgaa caataatggc attcatatca ataacaaggt cgcatgtcaa ttaattatga     960
   gaggtctatc tggcgaatat aaatttttac gctacacacg tcatcgacat ctaaatatga    1020
   cagtcgctga actgttctta gatatccatg ctatttatga agaacaacag ggatcgagaa    1080
   acagcaaacc taattacagg agaaatccga gtgatgagaa gaatgattct cgcagctata    1140
   cgaatacaac caaacccaaa gttatagctc ggaatcctca aaaaacaaat aattcgaaat    1200
   cgaaaacagc cagggctcac aatgtatcca catctaataa ctctcccagc acggacaacg    1260
   attccatcag taaatcaact actgaaccga ttcaattgaa caataagcac gaccttcatc    1320
   ttaggccaga aacttactga gtcgacggta cctgat                              1356
<210> 2
   <211> 1323
   <212> DNA
   <213> Saccharomyces cerevisiae
<400> 2
   atggaatccc aacaattatc taattaccca catatatctc atggtagcgc ctgtgcttcg      60
   gttacttcta aggaagtcca cacaaatcaa gatccgttag acgtttcagc ttccaaaatt     120
   caagaatatg ataaggcttc cactaaggct aactctcaac agacaacaac acctgcttca     180
   tcagctgttc cagagaaccc ccatcatgcc tctcctcaac ctgcttcagt accacctcca     240
   cagaatgggc cgtacccaca gcagtgcatg atgacccaaa accaagccaa tccatctggt     300
   tggtcatttt acggacaccc atctatgatt ccgtatacac cttatcaaat gtcgcctatg     360
   tactttccac ctgggccaca atcacagttt ccgcagtatc catcatcagt tggaacgcct     420
   ctgagcactc catcacctga gtcaggtaat acatttactg attcatcctc agcggactct     480
   gatatgacat ccactaaaaa atatgtcaga ccaccaccaa tgttaacctc acctaatgac     540
   tttccaaatt gggttaaaac atacatcaaa tttttacaaa actcgaatct cggtggtatt     600
   attccgacag taaacggaaa acccgtacgt ccgatcactg atgatgaact caccttcttg     660
   tataacgctt ttcaaatatt tgctccctct caattcctac ctacctgggt caaagacatc     720
   ctatccgttg attatacgga tatcatgaaa attctttcca aaagtattga aaaaatgcaa     780
   tctgataccc aagaggcaaa cgacattgtg accctggcaa atttgcaata taatggcagt     840
   acacctgcag atgcatttga aacaaaagtc acaaacatta tcgacagact gaacaataat     900
   ggcattcata tcaataacaa ggtcgcatgt caattaatta tgagaggtct atctggcgaa     960
   tataaatttt tacgctacac acgtcatcga catctaaata tgacagtcgc tgaactgttc    1020
   ttagatatcc atgctattta tgaagaacaa cagggatcga gaaacagcaa acctaattac    1080
   aggagaaatc cgagtgatga gaagaatgat tctcgcagct atacgaatac aaccaaaccc    1140
   aaagttatag ctcggaatcc tcaaaaaaca aataattcga aatcgaaaac agccagggct    1200
   cacaatgtat ccacatctaa taactctccc agcacggaca acgattccat cagtaaatca    1260
   actactgaac cgattcaatt gaacaataag cacgaccttc atcttaggcc agaaacttac    1320
   tga                                                                  1323
<210> 3
   <211> 440
   <212> PRT
   <213> Saccharomyces cerevisiae
<400> 3
   Met Glu Ser Gln Gln Leu Ser Asn Tyr Pro His Ile Ser His Gly Ser
   1               5                   10                  15
   Ala Cys Ala Ser Val Thr Ser Lys Glu Val His Thr Asn Gln Asp Pro
               20                  25                  30
   Leu Asp Val Ser Ala Ser Lys Ile Gln Glu Tyr Asp Lys Ala Ser Thr
           35                  40                  45
   Lys Ala Asn Ser Gln Gln Thr Thr Thr Pro Ala Ser Ser Ala Val Pro
       50                  55                  60
   Glu Asn Pro His His Ala Ser Pro Gln Pro Ala Ser Val Pro Pro Pro
   65                  70                  75                  80
   Gln Asn Gly Pro Tyr Pro Gln Gln Cys Met Met Thr Gln Asn Gln Ala
                   85                  90                  95
   Asn Pro Ser Gly Trp Ser Phe Tyr Gly His Pro Ser Met Ile Pro Tyr
               100                 105                 110
   Thr Pro Tyr Gln Met Ser Pro Met Tyr Phe Pro Pro Gly Pro Gln Ser
           115                 120                 125
   Gln Phe Pro Gln Tyr Pro Ser Ser Val Gly Thr Pro Leu Ser Thr Pro
       130                 135                 140
   Ser Pro Glu Ser Gly Asn Thr Phe Thr Asp Ser Ser Ser Ala Asp Ser
   145                 150                 155                 160
   Asp Met Thr Ser Thr Lys Lys Tyr Val Arg Pro Pro Pro Met Leu Thr
                   165                 170                 175
   Ser Pro Asn Asp Phe Pro Asn Trp Val Lys Thr Tyr Ile Lys Phe Leu
               180                 185                 190
   Gln Asn Ser Asn Leu Gly Gly Ile Ile Pro Thr Val Asn Gly Lys Pro
           195                 200                 205
   Val Arg Pro Ile Thr Asp Asp Glu Leu Thr Phe Leu Tyr Asn Ala Phe
       210                 215                 220
   Gln Ile Phe Ala Pro Ser Gln Phe Leu Pro Thr Trp Val Lys Asp Ile
   225                 230                 235                 240
   Leu Ser Val Asp Tyr Thr Asp Ile Met Lys Ile Leu Ser Lys Ser Ile
                   245                 250                 255
   Glu Lys Met Gln Ser Asp Thr Gln Glu Ala Asn Asp Ile Val Thr Leu
               260                 265                 270
   Ala Asn Leu Gln Tyr Asn Gly Ser Thr Pro Ala Asp Ala Phe Glu Thr
           275                 280                 285
   Lys Val Thr Asn Ile Ile Asp Arg Leu Asn Asn Asn Gly Ile His Ile
       290                 295                 300
   Asn Asn Lys Val Ala Cys Gln Leu Ile Met Arg Gly Leu Ser Gly Glu
   305                 310                 315                 320
   Tyr Lys Phe Leu Arg Tyr Thr Arg His Arg His Leu Asn Met Thr Val
                   325                 330                 335
   Ala Glu Leu Phe Leu Asp Ile His Ala Ile Tyr Glu Glu Gln Gln Gly
               340                 345                 350
   Ser Arg Asn Ser Lys Pro Asn Tyr Arg Arg Asn Pro Ser Asp Glu Lys
           355                 360                 365
   Asn Asp Ser Arg Ser Tyr Thr Asn Thr Thr Lys Pro Lys Val Ile Ala
       370                 375                 380
   Arg Asn Pro Gln Lys Thr Asn Asn Ser Lys Ser Lys Thr Ala Arg Ala
   385                 390                 395                 400
   His Asn Val Ser Thr Ser Asn Asn Ser Pro Ser Thr Asp Asn Asp Ser
                   405                 410                 415
   Ile Ser Lys Ser Thr Thr Glu Pro Ile Gln Leu Asn Asn Lys His Asp
               420                 425                 430
   Leu His Leu Arg Pro Glu Thr Tyr
           435                 440
<210> 4
   <211> 18
   <212> DNA
   <213> Artificial sequence
<220>
   <223> étiquette 6His
<400> 4
   caccaccacc accaccac 18
<210> 5
   <211> 18
   <212> RNA
   <213> Artificial sequence
<220>
   <223> étiquette 6His
<400> 5
   caccaccacc accaccac 18
<210> 6
   <211> 6
   <212> PRT
   <213> Artificial sequence
<220>
   <223> étiquette 6His
<400> 6
   His His His His His His
   1               5
<210> 7
   <211> 1359
   <212> DNA
   <213> Artificial sequence
<220>
   <223> 6His-Gag de Ty1
<400> 7
   atgcaccacc accaccacca cggcagcggc agcggcatgg aatcccaaca attatctaat      60
   tacccacata tatctcatgg tagcgcctgt gcttcggtta cttctaagga agtccacaca     120
   aatcaagatc cgttagacgt ttcagcttcc aaaattcaag aatatgataa ggcttccact     180
   aaggctaact ctcaacagac aacaacacct gcttcatcag ctgttccaga gaacccccat     240
   catgcctctc ctcaacctgc ttcagtacca cctccacaga atgggccgta cccacagcag     300
   tgcatgatga cccaaaacca agccaatcca tctggttggt cattttacgg acacccatct     360
   atgattccgt atacacctta tcaaatgtcg cctatgtact ttccacctgg gccacaatca     420
   cagtttccgc agtatccatc atcagttgga acgcctctga gcactccatc acctgagtca     480
   ggtaatacat ttactgattc atcctcagcg gactctgata tgacatccac taaaaaatat     540
   gtcagaccac caccaatgtt aacctcacct aatgactttc caaattgggt taaaacatac     600
   atcaaatttt tacaaaactc gaatctcggt ggtattattc cgacagtaaa cggaaaaccc     660
   gtacgtccga tcactgatga tgaactcacc ttcttgtata acgcttttca aatatttgct     720
   ccctctcaat tcctacctac ctgggtcaaa gacatcctat ccgttgatta tacggatatc     780
   atgaaaattc tttccaaaag tattgaaaaa atgcaatctg atacccaaga ggcaaacgac     840
   attgtgaccc tggcaaattt gcaatataat ggcagtacac ctgcagatgc atttgaaaca     900
   aaagtcacaa acattatcga cagactgaac aataatggca ttcatatcaa taacaaggtc     960
   gcatgtcaat taattatgag aggtctatct ggcgaatata aatttttacg ctacacacgt    1020
   catcgacatc taaatatgac agtcgctgaa ctgttcttag atatccatgc tatttatgaa    1080
   gaacaacagg gatcgagaaa cagcaaacct aattacagga gaaatccgag tgatgagaag    1140
   aatgattctc gcagctatac gaatacaacc aaacccaaag ttatagctcg gaatcctcaa    1200
   aaaacaaata attcgaaatc gaaaacagcc agggctcaca atgtatccac atctaataac    1260
   tctcccagca cggacaacga ttccatcagt aaatcaacta ctgaaccgat tcaattgaac    1320
   aataagcacg accttcatct taggccagaa acttactga                            1359
<210> 8
   <211> 452
   <212> PRT
   <213> Artificial sequence
<220>
   <223> 6His-Gag de Ty1
<400> 8
   Met His His His His His His Gly Ser Gly Ser Gly Met Glu Ser Gln
   1               5                   10                  15
   Gln Leu Ser Asn Tyr Pro His Ile Ser His Gly Ser Ala Cys Ala Ser
               20                  25                  30
   Val Thr Ser Lys Glu Val His Thr Asn Gln Asp Pro Leu Asp Val Ser
           35                  40                  45
   Ala Ser Lys Ile Gln Glu Tyr Asp Lys Ala Ser Thr Lys Ala Asn Ser
       50                  55                  60
   Gln Gln Thr Thr Thr Pro Ala Ser Ser Ala Val Pro Glu Asn Pro His
   65                  70                  75                  80
   His Ala Ser Pro Gln Pro Ala Ser Val Pro Pro Pro Gln Asn Gly Pro
                   85                  90                  95
   Tyr Pro Gln Gln Cys Met Met Thr Gln Asn Gln Ala Asn Pro Ser Gly
               100                 105                 110
   Trp Ser Phe Tyr Gly His Pro Ser Met Ile Pro Tyr Thr Pro Tyr Gln
           115                 120                 125
   Met Ser Pro Met Tyr Phe Pro Pro Gly Pro Gln Ser Gln Phe Pro Gln
       130                 135                 140
   Tyr Pro Ser Ser Val Gly Thr Pro Leu Ser Thr Pro Ser Pro Glu Ser
   145                 150                 155                 160
   Gly Asn Thr Phe Thr Asp Ser Ser Ser Ala Asp Ser Asp Met Thr Ser
                   165                 170                 175
   Thr Lys Lys Tyr Val Arg Pro Pro Pro Met Leu Thr Ser Pro Asn Asp
               180                 185                 190
   Phe Pro Asn Trp Val Lys Thr Tyr Ile Lys Phe Leu Gln Asn Ser Asn
           195                 200                 205
   Leu Gly Gly Ile Ile Pro Thr Val Asn Gly Lys Pro Val Arg Pro Ile
       210                 215                 220
   Thr Asp Asp Glu Leu Thr Phe Leu Tyr Asn Ala Phe Gln Ile Phe Ala
   225                 230                 235                 240
   Pro Ser Gln Phe Leu Pro Thr Trp Val Lys Asp Ile Leu Ser Val Asp
                   245                 250                 255
   Tyr Thr Asp Ile Met Lys Ile Leu Ser Lys Ser Ile Glu Lys Met Gln
               260                 265                 270
   Ser Asp Thr Gln Glu Ala Asn Asp Ile Val Thr Leu Ala Asn Leu Gln
           275                 280                 285
   Tyr Asn Gly Ser Thr Pro Ala Asp Ala Phe Glu Thr Lys Val Thr Asn
       290                 295                 300
   Ile Ile Asp Arg Leu Asn Asn Asn Gly Ile His Ile Asn Asn Lys Val
   305                 310                 315                 320
   Ala Cys Gln Leu Ile Met Arg Gly Leu Ser Gly Glu Tyr Lys Phe Leu
                   325                 330                 335
   Arg Tyr Thr Arg His Arg His Leu Asn Met Thr Val Ala Glu Leu Phe
               340                 345                 350
   Leu Asp Ile His Ala Ile Tyr Glu Glu Gln Gln Gly Ser Arg Asn Ser
           355                 360                 365
   Lys Pro Asn Tyr Arg Arg Asn Pro Ser Asp Glu Lys Asn Asp Ser Arg
       370                 375                 380
   Ser Tyr Thr Asn Thr Thr Lys Pro Lys Val Ile Ala Arg Asn Pro Gln
   385                 390                 395                 400
   Lys Thr Asn Asn Ser Lys Ser Lys Thr Ala Arg Ala His Asn Val Ser
                   405                 410                 415
   Thr Ser Asn Asn Ser Pro Ser Thr Asp Asn Asp Ser Ile Ser Lys Ser
               420                 425                 430
   Thr Thr Glu Pro Ile Gln Leu Asn Asn Lys His Asp Leu His Leu Arg
           435                 440                 445
   Pro Glu Thr Tyr
       450
<210> 9
   <211> 1356
   <212> DNA
   <213> Artificial sequence
<220>
   <223> Gag de Ty1 - 6His
<400> 9
   atggaatccc aacaattatc taattaccca catatatctc atggtagcgc ctgtgcttcg      60
   gttacttcta aggaagtcca cacaaatcaa gatccgttag acgtttcagc ttccaaaatt     120
   caagaatatg ataaggcttc cactaaggct aactctcaac agacaacaac acctgcttca     180
   tcagctgttc cagagaaccc ccatcatgcc tctcctcaac ctgcttcagt accacctcca     240
   cagaatgggc cgtacccaca gcagtgcatg atgacccaaa accaagccaa tccatctggt     300
   tggtcatttt acggacaccc atctatgatt ccgtatacac cttatcaaat gtcgcctatg     360
   tactttccac ctgggccaca atcacagttt ccgcagtatc catcatcagt tggaacgcct     420
   ctgagcactc catcacctga gtcaggtaat acatttactg attcatcctc agcggactct     480
   gatatgacat ccactaaaaa atatgtcaga ccaccaccaa tgttaacctc acctaatgac     540
   tttccaaatt gggttaaaac atacatcaaa tttttacaaa actcgaatct cggtggtatt     600
   attccgacag taaacggaaa acccgtacgt ccgatcactg atgatgaact caccttcttg     660
   tataacgctt ttcaaatatt tgctccctct caattcctac ctacctgggt caaagacatc     720
   ctatccgttg attatacgga tatcatgaaa attctttcca aaagtattga aaaaatgcaa     780
   tctgataccc aagaggcaaa cgacattgtg accctggcaa atttgcaata taatggcagt     840
   acacctgcag atgcatttga aacaaaagtc acaaacatta tcgacagact gaacaataat     900
   ggcattcata tcaataacaa ggtcgcatgt caattaatta tgagaggtct atctggcgaa     960
   tataaatttt tacgctacac acgtcatcga catctaaata tgacagtcgc tgaactgttc    1020
   ttagatatcc atgctattta tgaagaacaa cagggatcga gaaacagcaa acctaattac    1080
   aggagaaatc cgagtgatga gaagaatgat tctcgcagct atacgaatac aaccaaaccc    1140
   aaagttatag ctcggaatcc tcaaaaaaca aataattcga aatcgaaaac agccagggct    1200
   cacaatgtat ccacatctaa taactctccc agcacggaca acgattccat cagtaaatca    1260
   actactgaac cgattcaatt gaacaataag cacgaccttc atcttaggcc agaaacttac    1320
   ggcagcggca gcggccacca ccaccaccac cactga                              1356
<210> 10
   <211> 451
   <212> PRT
   <213> Artificial sequence
<220>
   <223> Gag de Ty1 - 6His
<400> 10
   Met Glu Ser Gln Gln Leu Ser Asn Tyr Pro His Ile Ser His Gly Ser
   1               5                   10                  15
   Ala Cys Ala Ser Val Thr Ser Lys Glu Val His Thr Asn Gln Asp Pro
               20                  25                  30
   Leu Asp Val Ser Ala Ser Lys Ile Gln Glu Tyr Asp Lys Ala Ser Thr
           35                  40                  45
   Lys Ala Asn Ser Gln Gln Thr Thr Thr Pro Ala Ser Ser Ala Val Pro
       50                  55                  60
   Glu Asn Pro His His Ala Ser Pro Gln Pro Ala Ser Val Pro Pro Pro
   65                  70                  75                  80
   Gln Asn Gly Pro Tyr Pro Gln Gln Cys Met Met Thr Gln Asn Gln Ala
                   85                  90                  95
   Asn Pro Ser Gly Trp Ser Phe Tyr Gly His Pro Ser Met Ile Pro Tyr
               100                 105                 110
   Thr Pro Tyr Gln Met Ser Pro Met Tyr Phe Pro Pro Gly Pro Gln Ser
           115                 120                 125
   Gln Phe Pro Gln Tyr Pro Ser Ser Val Gly Thr Pro Leu Ser Thr Pro
       130                 135                 140
   Ser Pro Glu Ser Gly Asn Thr Phe Thr Asp Ser Ser Ser Ala Asp Ser
   145                 150                 155                 160
   Asp Met Thr Ser Thr Lys Lys Tyr Val Arg Pro Pro Pro Met Leu Thr
                   165                 170                 175
   Ser Pro Asn Asp Phe Pro Asn Trp Val Lys Thr Tyr Ile Lys Phe Leu
               180                 185                 190
   Gln Asn Ser Asn Leu Gly Gly Ile Ile Pro Thr Val Asn Gly Lys Pro
           195                 200                 205
   Val Arg Pro Ile Thr Asp Asp Glu Leu Thr Phe Leu Tyr Asn Ala Phe
       210                 215                 220
   Gln Ile Phe Ala Pro Ser Gln Phe Leu Pro Thr Trp Val Lys Asp Ile
   225                 230                 235                 240
   Leu Ser Val Asp Tyr Thr Asp Ile Met Lys Ile Leu Ser Lys Ser Ile
                   245                 250                 255
   Glu Lys Met Gln Ser Asp Thr Gln Glu Ala Asn Asp Ile Val Thr Leu
               260                 265                 270
   Ala Asn Leu Gln Tyr Asn Gly Ser Thr Pro Ala Asp Ala Phe Glu Thr
           275                 280                 285
   Lys Val Thr Asn Ile Ile Asp Arg Leu Asn Asn Asn Gly Ile His Ile
       290                 295                 300
   Asn Asn Lys Val Ala Cys Gln Leu Ile Met Arg Gly Leu Ser Gly Glu
   305                 310                 315                 320
   Tyr Lys Phe Leu Arg Tyr Thr Arg His Arg His Leu Asn Met Thr Val
                   325                 330                 335
   Ala Glu Leu Phe Leu Asp Ile His Ala Ile Tyr Glu Glu Gln Gln Gly
               340                 345                 350
   Ser Arg Asn Ser Lys Pro Asn Tyr Arg Arg Asn Pro Ser Asp Glu Lys
           355                 360                 365
   Asn Asp Ser Arg Ser Tyr Thr Asn Thr Thr Lys Pro Lys Val Ile Ala
       370                 375                 380
   Arg Asn Pro Gln Lys Thr Asn Asn Ser Lys Ser Lys Thr Ala Arg Ala
   385                 390                 395                 400
   His Asn Val Ser Thr Ser Asn Asn Ser Pro Ser Thr Asp Asn Asp Ser
                   405                 410                 415
   Ile Ser Lys Ser Thr Thr Glu Pro Ile Gln Leu Asn Asn Lys His Asp
               420                 425                 430
   Leu His Leu Arg Pro Glu Thr Tyr Gly Ser Gly Ser Gly His His His
           435                 440                 445
   His His His
       450
<210> 11
   <211> 1392
   <212> DNA
   <213> Artificial sequence
<220>
   <223> 6His-Gag de Ty1-6His
<400> 11
   atgcaccacc accaccacca cggcagcggc agcggcatgg aatcccaaca attatctaat      60
   tacccacata tatctcatgg tagcgcctgt gcttcggtta cttctaagga agtccacaca     120
   aatcaagatc cgttagacgt ttcagcttcc aaaattcaag aatatgataa ggcttccact     180
   aaggctaact ctcaacagac aacaacacct gcttcatcag ctgttccaga gaacccccat     240
   catgcctctc ctcaacctgc ttcagtacca cctccacaga atgggccgta cccacagcag     300
   tgcatgatga cccaaaacca agccaatcca tctggttggt cattttacgg acacccatct     360
   atgattccgt atacacctta tcaaatgtcg cctatgtact ttccacctgg gccacaatca     420
   cagtttccgc agtatccatc atcagttgga acgcctctga gcactccatc acctgagtca     480
   ggtaatacat ttactgattc atcctcagcg gactctgata tgacatccac taaaaaatat     540
   gtcagaccac caccaatgtt aacctcacct aatgactttc caaattgggt taaaacatac     600
   atcaaatttt tacaaaactc gaatctcggt ggtattattc cgacagtaaa cggaaaaccc     660
   gtacgtccga tcactgatga tgaactcacc ttcttgtata acgcttttca aatatttgct     720
   ccctctcaat tcctacctac ctgggtcaaa gacatcctat ccgttgatta tacggatatc     780
   atgaaaattc tttccaaaag tattgaaaaa atgcaatctg atacccaaga ggcaaacgac     840
   attgtgaccc tggcaaattt gcaatataat ggcagtacac ctgcagatgc atttgaaaca     900
   aaagtcacaa acattatcga cagactgaac aataatggca ttcatatcaa taacaaggtc     960
   gcatgtcaat taattatgag aggtctatct ggcgaatata aatttttacg ctacacacgt    1020
   catcgacatc taaatatgac agtcgctgaa ctgttcttag atatccatgc tatttatgaa    1080
   gaacaacagg gatcgagaaa cagcaaacct aattacagga gaaatccgag tgatgagaag    1140
   aatgattctc gcagctatac gaatacaacc aaacccaaag ttatagctcg gaatcctcaa    1200
   aaaacaaata attcgaaatc gaaaacagcc agggctcaca atgtatccac atctaataac    1260
   tctcccagca cggacaacga ttccatcagt aaatcaacta ctgaaccgat tcaattgaac    1320
   aataagcacg accttcatct taggccagaa acttacggca gcggcagcgg ccaccaccac    1380
   caccaccact ga                                                        1392
<210> 12
   <211> 463
   <212> PRT
   <213> Artificial sequence
<220>
   <223> 6His-Gag de Ty1-6His
<400> 12
   Met His His His His His His Gly Ser Gly Ser Gly Met Glu Ser Gln
   1               5                   10                  15
   Gln Leu Ser Asn Tyr Pro His Ile Ser His Gly Ser Ala Cys Ala Ser
               20                  25                  30
   Val Thr Ser Lys Glu Val His Thr Asn Gln Asp Pro Leu Asp Val Ser
           35                  40                  45
   Ala Ser Lys Ile Gln Glu Tyr Asp Lys Ala Ser Thr Lys Ala Asn Ser
       50                  55                  60
   Gln Gln Thr Thr Thr Pro Ala Ser Ser Ala Val Pro Glu Asn Pro His
   65                  70                  75                  80
   His Ala Ser Pro Gln Pro Ala Ser Val Pro Pro Pro Gln Asn Gly Pro
                   85                  90                  95
   Tyr Pro Gln Gln Cys Met Met Thr Gln Asn Gln Ala Asn Pro Ser Gly
               100                 105                 110
   Trp Ser Phe Tyr Gly His Pro Ser Met Ile Pro Tyr Thr Pro Tyr Gln
           115                 120                 125
   Met Ser Pro Met Tyr Phe Pro Pro Gly Pro Gln Ser Gln Phe Pro Gln
       130                 135                 140
   Tyr Pro Ser Ser Val Gly Thr Pro Leu Ser Thr Pro Ser Pro Glu Ser
   145                 150                 155                 160
   Gly Asn Thr Phe Thr Asp Ser Ser Ser Ala Asp Ser Asp Met Thr Ser
                   165                 170                 175
   Thr Lys Lys Tyr Val Arg Pro Pro Pro Met Leu Thr Ser Pro Asn Asp
               180                 185                 190
   Phe Pro Asn Trp Val Lys Thr Tyr Ile Lys Phe Leu Gln Asn Ser Asn
           195                 200                 205
   Leu Gly Gly Ile Ile Pro Thr Val Asn Gly Lys Pro Val Arg Pro Ile
       210                 215                 220
   Thr Asp Asp Glu Leu Thr Phe Leu Tyr Asn Ala Phe Gln Ile Phe Ala
   225                 230                 235                 240
   Pro Ser Gln Phe Leu Pro Thr Trp Val Lys Asp Ile Leu Ser Val Asp
                   245                 250                 255
   Tyr Thr Asp Ile Met Lys Ile Leu Ser Lys Ser Ile Glu Lys Met Gln
               260                 265                 270
   Ser Asp Thr Gln Glu Ala Asn Asp Ile Val Thr Leu Ala Asn Leu Gln
           275                 280                 285
   Tyr Asn Gly Ser Thr Pro Ala Asp Ala Phe Glu Thr Lys Val Thr Asn
       290                 295                 300
   Ile Ile Asp Arg Leu Asn Asn Asn Gly Ile His Ile Asn Asn Lys Val
   305                 310                 315                 320
   Ala Cys Gln Leu Ile Met Arg Gly Leu Ser Gly Glu Tyr Lys Phe Leu
                   325                 330                 335
   Arg Tyr Thr Arg His Arg His Leu Asn Met Thr Val Ala Glu Leu Phe
               340                 345                 350
   Leu Asp Ile His Ala Ile Tyr Glu Glu Gln Gln Gly Ser Arg Asn Ser
           355                 360                 365
   Lys Pro Asn Tyr Arg Arg Asn Pro Ser Asp Glu Lys Asn Asp Ser Arg
       370                 375                 380
   Ser Tyr Thr Asn Thr Thr Lys Pro Lys Val Ile Ala Arg Asn Pro Gln
   385                 390                 395                 400
   Lys Thr Asn Asn Ser Lys Ser Lys Thr Ala Arg Ala His Asn Val Ser
                   405                 410                 415
   Thr Ser Asn Asn Ser Pro Ser Thr Asp Asn Asp Ser Ile Ser Lys Ser
               420                 425                 430
   Thr Thr Glu Pro Ile Gln Leu Asn Asn Lys His Asp Leu His Leu Arg
           435                 440                 445
   Pro Glu Thr Tyr Gly Ser Gly Ser Gly His His His His His His
       450                 455                 460
<210> 13
   <211> 1329
   <212> DNA
   <213> Artificial sequence
<220>
   <223> séquence d'adressage (Gag modifiée)
<400> 13
   aagcttgatg gatcccaaca attatctaat tacccacata tatctcatgg tagcgcctgt      60
   gcttcggtta cttctaagga agtccacaca aatcaagatc cgttagacgt ttcagcttcc     120
   aaaattcaag aatatgataa ggcttccact aaggctaact ctcaacagac aacaacacct     180
   gcttcatcag ctgttccaga gaacccccat catgcctctc ctcaacctgc ttcagtacca     240
   cctccacaga atgggccgta cccacagcag tgcatgatga cccaaaacca agccaatcca     300
   tctggttggt cattttacgg acacccatct atgattccgt atacacctta tcaaatgtcg     360
   cctatgtact ttccacctgg gccacaatca cagtttccgc agtatccatc atcagttgga     420
   acgcctctga gcactccatc acctgagtca ggtaatacat ttactgattc atcctcagcg     480
   gactctgata tgacatccac taaaaaatat gtcagaccac caccaatgtt aacctcacct     540
   aatgactttc caaattgggt taaaacatac atcaaatttt tacaaaactc gaatctcggt     600
   ggtattattc cgacagtaaa cggaaaaccc gtacgtccga tcactgatga tgaactcacc     660
   ttcttgtata acgcttttca aatatttgct ccctctcaat tcctacctac ctgggtcaaa     720
   gacatcctat ccgttgatta tacggatatc atgaaaattc tttccaaaag tattgaaaaa     780
   atgcaatctg atacccaaga ggcaaacgac attgtgaccc tggcaaattt gcaatataat     840
   ggcagtacac ctgcagatgc atttgaaaca aaagtcacaa acattatcga cagactgaac     900
   aataatggca ttcatatcaa taacaaggtc gcatgtcaat taattatgag aggtctatct     960
   ggcgaatata aatttttacg ctacacacgt catcgacatc taaatatgac agtcgctgaa    1020
   ctgttcttag atatccatgc tatttatgaa gaacaacagg gatcgagaaa cagcaaacct    1080
   aattacagga gaaatccgag tgatgagaag aatgattctc gcagctatac gaatacaacc    1140
   aaacccaaag ttatagctcg gaatcctcaa aaaacaaata attcgaaatc gaaaacagcc    1200
   agggctcaca atgtatccac atctaataac tctcccagca cggacaacga ttccatcagt    1260
   aaatcaacta ctgaaccgat tcaattgaac aataagcacg accttcatct taggccagaa    1320
   acttactga                                                            1329
<210> 14
   <211> 1329
   <212> RNA
   <213> Artificial sequence
<220>
   <223> séquence d'adressage (Gag modifiée)
<400> 14
   aagcuugaug gaucccaaca auuaucuaau uacccacaua uaucucaugg uagcgccugu 60
   gcuucgguua cuucuaagga aguccacaca aaucaagauc cguuagacgu uucagcuucc 120
   aaaauucaag aauaugauaa ggcuuccacu aaggcuaacu cucaacagac aacaacaccu 180
   gcuucaucag cuguuccaga gaacccccau caugccucuc cucaaccugc uucaguacca 240
   ccuccacaga augggccgua cccacagcag ugcaugauga cccaaaacca agccaaucca 300
   ucugguuggu cauuuuacgg acacccaucu augauuccgu auacaccuua ucaaaugucg 360
   ccuauguacu uuccaccugg gccacaauca caguuuccgc aguauccauc aucaguugga 420
   acgccucuga gcacuccauc accugaguca gguaauacau uuacugauuc auccucagcg 480
   gacucugaua ugacauccac uaaaaaauau gucagaccac caccaauguu aaccucaccu 540
   aaugacuuuc caaauugggu uaaaacauac aucaaauuuu uacaaaacuc gaaucucggu 600
   gguauuauuc cgacaguaaa cggaaaaccc guacguccga ucacugauga ugaacucacc 660
   uucuuguaua acgcuuuuca aauauuugcu cccucucaau uccuaccuac cugggucaaa 720
   gacauccuau ccguugauua uacggauauc augaaaauuc uuuccaaaag uauugaaaaa 780
   augcaaucug auacccaaga ggcaaacgac auugugaccc uggcaaauuu gcaauauaau 840
   ggcaguacac cugcagaugc auuugaaaca aaagucacaa acauuaucga cagacugaac 900
   aauaauggca uucauaucaa uaacaagguc gcaugucaau uaauuaugag aggucuaucu 960
   ggcgaauaua aauuuuuacg cuacacacgu caucgacauc uaaauaugac agucgcugaa 1020
   cuguucuuag auauccaugc uauuuaugaa gaacaacagg gaucgagaaa cagcaaaccu 1080
   aauuacagga gaaauccgag ugaugagaag aaugauucuc gcagcuauac gaauacaacc 1140
   aaacccaaag uuauagcucg gaauccucaa aaaacaaaua auucgaaauc gaaaacagcc 1200
   agggcucaca auguauccac aucuaauaac ucucccagca cggacaacga uuccaucagu 1260
   aaaucaacua cugaaccgau ucaauugaac aauaagcacg accuucaucu uaggccagaa 1320
   acuuacuga 1329
<210> 15
   <211> 398
   <212> DNA
   <213> Artificial sequence
<220>
   <223> séquence d'adressage (fragment de Gag)
<400> 15
   ccacagcagt gcatgatgac ccaaaaccaa gccaatccat ctggttggtc attttacgga 60
   cacccatcta tgattccgta tacaccttat caaatgtcgc ctatgtactt tccacctggg 120
   ccacaatcac agtttccgca gtatccatca tcagttggaa cgcctctgag cactccatca 180
   cctgagtcag gtaatacatt tactgattca tcctcagcgg actctgatat gacatccact 240
   aaaaaatatg tcagaccacc accaatgtta acctcaccta atgactttcc aaattgggtt 300
   aaaacataca tcaaattttt acaaaactcg aatctcggtg gtattattcc gacagtaaac 360
   ggaaaacccg tacgtccgat cactgatgat gaactcac 398
<210> 16
   <211> 398
   <212> RNA
   <213> Artificial sequence
<220>
   <223> séquence d'adressage (fragment de Gag)
<400> 16
   ccacagcagu gcaugaugac ccaaaaccaa gccaauccau cugguugguc auuuuacgga      60
   cacccaucua ugauuccgua uacaccuuau caaaugucgc cuauguacuu uccaccuggg     120
   ccacaaucac aguuuccgca guauccauca ucaguuggaa cgccucugag cacuccauca     180
   ccugagucag guaauacauu uacugauuca uccucagcgg acucugauau gacauccacu     240
   aaaaaauaug ucagaccacc accaauguua accucaccua augacuuucc aaauuggguu     300
   aaaacauaca ucaaauuuuu acaaaacucg aaucucggug guauuauucc gacaguaaac     360
   ggaaaacccg uacguccgau cacugaugau gaacucac                             398
<210> 17
   <211> 1068
   <212> DNA
   <213> Artificial sequence
<220>
   <223> séquence d'adressage (fragment de Gag)
<400> 17
   ccacagcagt gcatgatgac ccaaaaccaa gccaatccat ctggttggtc attttacgga      60
   cacccatcta tgattccgta tacaccttat caaatgtcgc ctatgtactt tccacctggg     120
   ccacaatcac agtttccgca gtatccatca tcagttggaa cgcctctgag cactccatca     180
   cctgagtcag gtaatacatt tactgattca tcctcagcgg actctgatat gacatccact     240
   aaaaaatatg tcagaccacc accaatgtta acctcaccta atgactttcc aaattgggtt     300
   aaaacataca tcaaattttt acaaaactcg aatctcggtg gtattattcc gacagtaaac     360
   ggaaaacccg tacgtccgat cactgatgat gaactcacct tcttgtataa cgcttttcaa     420
   atatttgctc cctctcaatt cctacctacc tgggtcaaag acatcctatc cgttgattat     480
   acggatatca tgaaaattct ttccaaaagt attgaaaaaa tgcaatctga tacccaagag     540
   gcaaacgaca ttgtgaccct ggcaaatttg caatataatg gcagtacacc tgcagatgca     600
   tttgaaacaa aagtcacaaa cattatcgac agactgaaca ataatggcat tcatatcaat     660
   aacaaggtcg catgtcaatt aattatgaga ggtctatctg gcgaatataa atttttacgc     720
   tacacacgtc atcgacatct aaatatgaca gtcgctgaac tgttcttaga tatccatgct     780
   atttatgaag aacaacaggg atcgagaaac agcaaaccta attacaggag aaatccgagt     840
   gatgagaaga atgattctcg cagctatacg aatacaacca aacccaaagt tatagctcgg     900
   aatcctcaaa aaacaaataa ttcgaaatcg aaaacagcca gggctcacaa tgtatccaca      960
   tctaataact ctcccagcac ggacaacgat tccatcagta aatcaactac tgaaccgatt    1020
   caattgaaca ataagcacga ccttcatctt aggccagaaa cttactga                1068
<210> 18
   <211> 1068
   <212> RNA
   <213> Artificial sequence
<220>
   <223> séquence d'adressage (fragment de Gag)
<400> 18
   ccacagcagu gcaugaugac ccaaaaccaa gccaauccau cugguugguc auuuuacgga    60
   cacccaucua ugauuccgua uacaccuuau caaaugucgc cuauguacuu uccaccuggg   120
   ccacaaucac aguuuccgca guauccauca ucaguuggaa cgccucugag cacuccauca   180
   ccugagucag guaauacauu uacugauuca uccucagcgg acucugauau gacauccacu   240
   aaaaaauaug ucagaccacc accaauguua accucaccua augacuuucc aaauuggguu   300
   aaaacauaca ucaaauuuuu acaaaacucg aaucucggug guauuauucc gacaguaaac   360
   ggaaaacccg uacguccgau cacugaugau gaacucaccu ucuuguauaa cgcuuuucaa   420
   auauuugcuc ccucucaauu ccuaccuacc ugggucaaag acauccuauc cguugauuau   480
   acggauauca ugaaaauucu uuccaaaagu auugaaaaaa ugcaaucuga uacccaagag   540
   gcaaacgaca uugugacccu ggcaaauuug caauauaaug gcaguacacc ugcagaugca   600
   uuugaaacaa aagucacaaa cauuaucgac agacugaaca auaauggcau ucauaucaau   660
   aacaaggucg caugucaauu aauuaugaga ggucuaucug gcgaauauaa auuuuuacgc   720
   uacacacguc aucgacaucu aaauaugaca gucgcugaac uguucuuaga uauccaugcu   780
   auuuaugaag aacaacaggg aucgagaaac agcaaaccua auuacaggag aaauccgagu   840
   gaugagaaga augauucucg cagcuauacg aauacaacca aacccaaagu uauagcucgg   900
   aauccucaaa aaacaaauaa uucgaaaucg aaaacagcca gggcucacaa uguauccaca   960
   ucuaauaacu cucccagcac ggacaacgau uccaucagua aaucaacuac ugaaccgauu  1020
   caauugaaca auaagcacga ccuucaucuu aggccagaaa cuuacuga              1068
<210> 19
   <211> 73
   <212> DNA
   <213> Artificial sequence
<220>
   <223> amorce FQ3
<400> 19
   tagcactagt atgcaccacc accaccacca cggcagcggc agcggcatgg aatcccaaca    60
   attatctaat tac                                                       73
<210> 20
   <211> 31
   <212> DNA
   <213> Artificial sequence
<220>
   <223> amorce RQ3
<400> 20
   gctaaagctt tcagtaagtt tctggcctaa g 31
<210> 21
   <211> 37
   <212> DNA
   <213> Artificial sequence
<220>
   <223> amorce FQ4
<400> 21
   tagcactagt atggaatccc aacaattatc taattac 37
<210> 22
   <211> 92
   <212> DNA
   <213> Artificial sequence
<220>
   <223> amorce RQ4
<400> 22
   gctaatgact cgaggtcgac ggtatcgata agctttcagt ggtggtggtg gtggtggccg 60
   ctgccgctgc cgtaagtttc tggcctaaga tg 92
<210> 23
   <211> 23
   <212> DNA
   <213> Artificial sequence
<220>
   <223> amorce qGAGF
<400> 23
   gctgtttctc gatccctgtt gtt 23
<210> 24
   <211> 22
   <212> DNA
   <213> Artificial sequence
<220>
   <223> amorce qGAGR
<400> 24
   agaggcaaac gacattgtga cc 22
<210> 25
   <211> 720
   <212> DNA
   <213> Bacillus cereus
<400> 25
   atggtgagca agggcgagga gctgttcacc ggggtggtgc ccatcctggt cgagctggac    60
   ggcgacgtaa acggccacaa gttcagcgtg tccggcgagg gcgagggcga tgccacctac   120
   ggcaagctga ccctgaagtt catctgcacc accggcaagc tgcccgtgcc ctggcccacc   180
   ctcgtgacca ccctgaccta cggcgtgcag tgcttcagcc gctaccccga ccacatgaag   240
   cagcacgact tcttcaagtc cgccatgccc gaaggctacg tccaggagcg caccatcttc   300
   ttcaaggacg acggcaacta caagacccgc gccgaggtga agttcgaggg cgacaccctg   360
   gtgaaccgca tcgagctgaa gggcatcgac ttcaaggagg acggcaacat cctggggcac   420
   aagctggagt acaactacaa cagccacaac gtctatatca tggccgacaa gcagaagaac   480
   ggcatcaagg tgaacttcaa gatccgccac aacatcgagg acggcagcgt gcagctcgcc   540
   gaccactacc agcagaacac ccccatcggc gacggccccg tgctgctgcc cgacaaccac   600
   tacctgagca cccagtccgc cctgagcaaa gaccccaacg agaagcgcga tcacatggtc   660
   ctgctggagt tcgtgaccgc cgccgggatc actctcggca tggacgagct gtacaagtaa   720
<210> 26
   <211> 720
   <212> RNA
   <213> Bacillus cereus
<400> 26
   auggugagca agggcgagga gcuguucacc gggguggugc ccauccuggu cgagcuggac    60
   ggcgacguaa acggccacaa guucagcgug uccggcgagg gcgagggcga ugccaccuac   120
   ggcaagcuga cccugaaguu caucugcacc accggcaagc ugcccgugcc cuggcccacc   180
   cucgugacca cccugaccua cggcgugcag ugcuucagcc gcuaccccga ccacaugaag   240
   cagcacgacu ucuucaaguc cgccaugccc gaaggcuacg uccaggagcg caccaucuuc   300
   uucaaggacg acggcaacua caagacccgc gccgaggugu aguucgaggg cgacacccug   360
   gugaaccgca ucgagcugaa gggcaucgac uucaaggagg acggcaacau ccuggggcac   420
   aagcuggagu acaacuacaa cagccacaac gucuauauca uggccgacaa gcagaagaac   480
   ggcaucaagg ugaacuucaa gauccgccac aacaucgagg acggcagcgu gcagcucgcc   540
   gaccacuacc agcagaacac ccccaucggc gacggccccg ugcugcugcc cgacaaccac   600
   uaccugagca cccaguccgc ccugagcaaa gaccccaacg agaagcgcga ucacaugguc   660
   cugcuggagu ucgugaccgc cgccgggauc acucucggca uggacgagcu guacaaguaa   720
<210> 27
   <211> 239
   <212> PRT
   <213> Bacillus cereus
<400> 27
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 28
   <211> 1653
   <212> DNA
   <213> Artificial sequence
<220>
   <223> Luciferase
<400> 28
   atggaagacg ccaaaaacat aaagaaaggc ccggcgccat tctatccgct ggaagatgga      60
   accgctggag agcaactgca taaggctatg aagagatacg ccctggttcc tggaacaatt     120
   gcttttacag atgcacatat cgaggtggac atcacttacg ctgagtactt cgaaatgtcc     180
   gttcggttgg cagaagctat gaaacgatat gggctgaata caaatcacag aatcgtcgta     240
   tgcagtgaaa actctcttca attctttatg ccggtgttgg gcgcgttatt tatcggagtt     300
   gcagttgcgc ccgcgaacga catttataat gaacgtgaat tgctcaacag tatgggcatt     360
   tcgcagccta ccgtggtgtt cgtttccaaa aaggggttgc aaaaaatttt gaacgtgcaa     420
   aaaaagctcc caatcatcca aaaaattatt atcatggatt ctaaaacgga ttaccaggga     480
   tttcagtcga tgtacacgtt cgtcacatct catctacctc ccggttttaa tgaatacgat     540
   tttgtgccag agtccttcga tagggacaag acaattgcac tgatcatgaa ctcctctgga     600
   tctactggtc tgcctaaagg tgtcgctctg cctcatagaa ctgcctgcgt gagattctcg     660
   catgccagag atcctatttt tggcaatcaa atcattccgg atactgcgat tttaagtgtt     720
   gttccattcc atcacggttt tggaatgttt actacactcg gatatttgat atgtggattt     780
   cgagtcgtct taatgtatag atttgaagaa gagctgtttc tgaggagcct tcaggattac     840
   aagattcaaa gtgcgctgct ggtgccaacc ctattctcct tcttcgccaa aagcactctg     900
   attgacaaat acgatttatc taatttacac gaaattgctt ctggtggcgc tcccctctct     960
   aaggaagtcg gggaagcggt tgccaagagg ttccatctgc caggtatcag gcaaggatat    1020
   gggctcactg agactacatc agctattctg attacacccg agggggatga taaaccgggc  1080
   gcggtcggta aagttgttcc attttttgaa gcgaaggttg tggatctgga taccgggaaa    1140
   acgctgggcg ttaatcaaag aggcgaactg tgtgtgagag gtcctatgat tatgtccggt    1200
   tatgtaaaca atccggaagc gaccaacgcc ttgattgaca aggatggatg gctacattct    1260
   ggagacatag cttactggga cgaagacgaa cacttcttca tcgttgaccg cctgaagtct    1320
   ctgattaagt acaaaggcta tcaggtggct cccgctgaat tggaatccat cttgctccaa    1380
   caccccaaca tcttcgacgc aggtgtcgca ggtcttcccg acgatgacgc cggtgaactt    1440
   cccgccgccg ttgttgtttt ggagcacgga aagacgatga cggaaaaaga gatcgtggat    1500
   tacgtcgcca gtcaagtaac aaccgcgaaa aagttgcgcg gaggagttgt gtttgtggac    1560
   gaagtaccga aaggtcttac cggaaaactc gacgcaagaa aaatcagaga gatcctcata    1620
   aaggccaaga agggcggaaa gatcgccgtg taa                                1653
<210> 29
   <211> 1653
   <212> RNA
   <213> Artificial sequence
<220>
   <223> Luciferase
<400> 29
   auggaagacg ccaaaaacau aaagaaaggc ccggcgccau ucuauccgcu ggaagaugga      60
   accgcuggag agcaacugca uaaggcuaug aagagauacg cccugguucc uggaacaauu     120
   gcuuuuacag augcacauau cgagguggac aucacuuacg cugaguacuu cgaaaugucc     180
   guucgguugg cagaagcuau gaaacgauau gggcugaaua caaaucacag aaucgucgua     240
   ugcagugaaa acucucuuca auucuuuaug ccgguguugg gcgcguuauu uaucggaguu     300
   gcaguugcgc ccgcgaacga cauuuauaau gaacgugaau ugcucaacag uaugggcauu     360
   ucgcagccua ccgugguguu cguuuccaaa aagggguugc aaaaaauuuu gaacgugcaa     420
   aaaaagcucc caaucaucca aaaaauuauu aucauggauu cuaaaacgga uuaccaggga     480
   uuucagucga uguacacguu cgucacaucu caucuaccuc ccgguuuuaa ugaauacgau     540
   uuugugccag aguccuucga uagggacaag acaauugcac ugaucaugaa cuccucugga     600
   ucuacugguc ugccuaaagg ugucgcucug ccucauagaa cugccugcgu gagauucucg     660
   caugccagag auccuauuuu uggcaaucaa aucauuccgg auacugcgau uuuaaguguu     720
   guuccauucc aucacgguuu uggaauguuu acuacacucg gauauuugau auguggauuu     780
   cgagucgucu uaauguauag auuugaagaa gagcuguuuc ugaggagccu ucaggauuac     840
   aagauucaaa gugcgcugcu ggugccaacc cuauucuccu ucuucgccaa aagcacucug     900
   auugacaaau acgauuuauc uaauuuacac gaaauugcuu cugguggcgc uccccucucu     960
   aaggaagucg gggaagcggu ugccaagagg uuccaucugc cagguaucag gcaaggauau    1020
   gggcucacug agacuacauc agcuauucug auuacacccg agggggauga uaaaccgggc    1080
   gcggucggua aaguuguucc auuuuuugaa gcgaagguug uggaucugga uaccgggaaa    1140
   acgcugggcg uuaaucaaag aggcgaacug ugugugagag guccuaugau uauguccggu    1200
   uauguaaaca auccggaagc gaccaacgcc uugauugaca aggauggaug gcuacauucu    1260
   ggagacauag cuuacuggga cgaagacgaa cacuucuuca ucguugaccg ccugaagucu    1320
   cugauuaagu acaaaggcua ucagguggcu cccgcugaau uggaauccau cuugcuccaa    1380
   caccccaaca ucuucgacgc aggugucgca ggucuucccg acgaugacgc cggugaacuu    1440
   cccgccgccg uuguuguuuu ggagcacgga aagacgauga cggaaaaaga gaucguggau    1500
   uacgucgcca gucaaguaac aaccgcgaaa aaguugcgcg gaggaguugu guuuguggac    1560
   gaaguaccga aaggucuuac cggaaaacuc gacgcaagaa aaaucagaga gauccucaua    1620
   aaggccaaga agggcggaaa gaucgccgug uaa                                 1653
<210> 30
   <211> 550
   <212> PRT
   <213> Artificial sequence
<220>
   <223> Luciferase
<400> 30
   Met Glu Asp Ala Lys Asn Ile Lys Lys Gly Pro Ala Pro Phe Tyr Pro
   1               5                   10                  15
   Leu Glu Asp Gly Thr Ala Gly Glu Gln Leu His Lys Ala Met Lys Arg
               20                  25                  30
   Tyr Ala Leu Val Pro Gly Thr Ile Ala Phe Thr Asp Ala His Ile Glu
           35                  40                  45
   Val Asp Ile Thr Tyr Ala Glu Tyr Phe Glu Met Ser Val Arg Leu Ala
       50                  55                  60
   Glu Ala Met Lys Arg Tyr Gly Leu Asn Thr Asn His Arg Ile Val Val
   65                  70                  75                  80
   Cys Ser Glu Asn Ser Leu Gln Phe Phe Met Pro Val Leu Gly Ala Leu
                   85                  90                  95
   Phe Ile Gly Val Ala Val Ala Pro Ala Asn Asp Ile Tyr Asn Glu Arg
               100                 105                 110
   Glu Leu Leu Asn Ser Met Gly Ile Ser Gln Pro Thr Val Val Phe Val
           115                 120                 125
   Ser Lys Lys Gly Leu Gln Lys Ile Leu Asn Val Gln Lys Lys Leu Pro
       130                 135                 140
   Ile Ile Gln Lys Ile Ile Ile Met Asp Ser Lys Thr Asp Tyr Gln Gly
   145                 150                 155                 160
   Phe Gln Ser Met Tyr Thr Phe Val Thr Ser His Leu Pro Pro Gly Phe
                   165                 170                 175
   Asn Glu Tyr Asp Phe Val Pro Glu Ser Phe Asp Arg Asp Lys Thr Ile
               180                 185                 190
   Ala Leu Ile Met Asn Ser Ser Gly Ser Thr Gly Leu Pro Lys Gly Val
           195                 200                 205
   Ala Leu Pro His Arg Thr Ala Cys Val Arg Phe Ser His Ala Arg Asp
       210                 215                 220
   Pro Ile Phe Gly Asn Gln Ile Ile Pro Asp Thr Ala Ile Leu Ser Val
   225                 230                 235                 240
   Val Pro Phe His His Gly Phe Gly Met Phe Thr Thr Leu Gly Tyr Leu
                   245                 250                 255
   Ile Cys Gly Phe Arg Val Val Leu Met Tyr Arg Phe Glu Glu Glu Leu
               260                 265                 270
   Phe Leu Arg Ser Leu Gln Asp Tyr Lys Ile Gln Ser Ala Leu Leu Val
           275                 280                 285
   Pro Thr Leu Phe Ser Phe Phe Ala Lys Ser Thr Leu Ile Asp Lys Tyr
       290                 295                 300
   Asp Leu Ser Asn Leu His Glu Ile Ala Ser Gly Gly Ala Pro Leu Ser
   305                 310                 315                 320
   Lys Glu Val Gly Glu Ala Val Ala Lys Arg Phe His Leu Pro Gly Ile
                   325                 330                 335
   Arg Gln Gly Tyr Gly Leu Thr Glu Thr Thr Ser Ala Ile Leu Ile Thr
               340                 345                 350
   Pro Glu Gly Asp Asp Lys Pro Gly Ala Val Gly Lys Val Val Pro Phe
           355                 360                 365
   Phe Glu Ala Lys Val Val Asp Leu Asp Thr Gly Lys Thr Leu Gly Val
       370                 375                 380
   Asn Gln Arg Gly Glu Leu Cys Val Arg Gly Pro Met Ile Met Ser Gly
   385                 390                 395                 400
   Tyr Val Asn Asn Pro Glu Ala Thr Asn Ala Leu Ile Asp Lys Asp Gly
                   405                 410                 415
   Trp Leu His Ser Gly Asp Ile Ala Tyr Trp Asp Glu Asp Glu His Phe
               420                 425                 430
   Phe Ile Val Asp Arg Leu Lys Ser Leu Ile Lys Tyr Lys Gly Tyr Gln
           435                 440                 445
   Val Ala Pro Ala Glu Leu Glu Ser Ile Leu Leu Gln His Pro Asn Ile
       450                 455                 460
   Phe Asp Ala Gly Val Ala Gly Leu Pro Asp Asp Asp Ala Gly Glu Leu
   465                 470                 475                 480
   Pro Ala Ala Val Val Val Leu Glu His Gly Lys Thr Met Thr Glu Lys
                   485                 490                 495
   Glu Ile Val Asp Tyr Val Ala Ser Gln Val Thr Thr Ala Lys Lys Leu
               500                 505                 510
   Arg Gly Gly Val Val Phe Val Asp Glu Val Pro Lys Gly Leu Thr Gly
           515                 520                 525
   Lys Leu Asp Ala Arg Lys Ile Arg Glu Ile Leu Ile Lys Ala Lys Lys
       530                 535                 540
   Gly Gly Lys Ile Ala Val
   545                 550
<210> 31
   <211> 37
   <212> DNA
   <213> Artificial sequence
<220>
   <223> Amorce FQ5
<400> 31
   tgatactagt atggaatccc aacaattatc taattac 37
<210> 32
   <211> 30
   <212> DNA
   <213> Artificial sequence
<220>
   <223> Amorce RQ5
<400> 32
   atcgaattcg taagtttctg gcctaagatg 30
<210> 33
   <211> 55
   <212> DNA
   <213> Artificial sequence
<220>
   <223> Amorce FQ6
<400> 33
   tacgactcac tatagggcga attggagctc tagtacggat tagaagccgc cgagc 55
<210> 34
   <211> 55
   <212> DNA
   <213> Artificial sequence
<220>
   <223> Amorce RQ6
<400> 34
   cctcactaaa gggaacaaaa gctgggtacc ggccgcaaat taaagccttc gagcg 55
<210> 35
   <211> 98
   <212> DNA
   <213> Artificial sequence
<220>
   <223> 3MS2
<400> 35
   atatccgtac accatcaggg tacgagctag cccatggcgt acaccatcag ggtacgacga 60
   gtagatctcg tacaccatca gggtacggaa tcgctgac 98
<210> 36
   <211> 98
   <212> RNA
   <213> Artificial sequence
<220>
   <223> 3MS2
<400> 36
   auauccguac accaucaggg uacgagcuag cccauggcgu acaccaucag gguacgacga 60
   guagaucucg uacaccauca ggguacggaa ucgcugac 98
<210> 37
   <211> 1320
   <212> DNA
   <213> Saccharomyces cerevisiae
<400> 37
   atggaatccc aacaattatc taattaccca catatatctc atggtagcgc ctgtgcttcg      60
   gttacttcta aggaagtcca cacaaatcaa gatccgttag acgtttcagc ttccaaaatt     120
   caagaatatg ataaggcttc cactaaggct aactctcaac agacaacaac acctgcttca     180
   tcagctgttc cagagaaccc ccatcatgcc tctcctcaac ctgcttcagt accacctcca     240
   cagaatgggc cgtacccaca gcagtgcatg atgacccaaa accaagccaa tccatctggt     300
   tggtcatttt acggacaccc atctatgatt ccgtatacac cttatcaaat gtcgcctatg     360
   tactttccac ctgggccaca atcacagttt ccgcagtatc catcatcagt tggaacgcct     420
   ctgagcactc catcacctga gtcaggtaat acatttactg attcatcctc agcggactct     480
   gatatgacat ccactaaaaa atatgtcaga ccaccaccaa tgttaacctc acctaatgac     540
   tttccaaatt gggttaaaac atacatcaaa tttttacaaa actcgaatct cggtggtatt     600
   attccgacag taaacggaaa acccgtacgt ccgatcactg atgatgaact caccttcttg     660
   tataacgctt ttcaaatatt tgctccctct caattcctac ctacctgggt caaagacatc     720
   ctatccgttg attatacgga tatcatgaaa attctttcca aaagtattga aaaaatgcaa     780
   tctgataccc aagaggcaaa cgacattgtg accctggcaa atttgcaata taatggcagt     840
   acacctgcag atgcatttga aacaaaagtc acaaacatta tcgacagact gaacaataat     900
   ggcattcata tcaataacaa ggtcgcatgt caattaatta tgagaggtct atctggcgaa     960
   tataaatttt tacgctacac acgtcatcga catctaaata tgacagtcgc tgaactgttc    1020
   ttagatatcc atgctattta tgaagaacaa cagggatcga gaaacagcaa acctaattac    1080
   aggagaaatc cgagtgatga gaagaatgat tctcgcagct atacgaatac aaccaaaccc    1140
   aaagttatag ctcggaatcc tcaaaaaaca aataattcga aatcgaaaac agccagggct    1200
   cacaatgtat ccacatctaa taactctccc agcacggaca acgattccat cagtaaatca    1260
   actactgaac cgattcaatt gaacaataag cacgaccttc atcttaggcc agaaacttac    1320
<210> 38
   <211> 2371
   <212> DNA
   <213> Artificial sequence
<220>
   <223> GAG de Ty1 - eGFP - 6His - 3MS2
<400> 38
   actagttcta tggaatccca acaattatct aattacccac atatatctca tggtagcgcc      60
   tgtgcttcgg ttacttctaa ggaagtccac acaaatcaag atccgttaga cgtttcagct     120
   tccaaaattc aagaatatga taaggcttcc actaaggcta actctcaaca gacaacaaca     180
   cctgcttcat cagctgttcc agagaacccc catcatgcct ctcctcaacc tgcttcagta     240
   ccacctccac agaatgggcc gtacccacag cagtgcatga tgacccaaaa ccaagccaat     300
   ccatctggtt ggtcatttta cggacaccca tctatgattc cgtatacacc ttatcaaatg     360
   tcgcctatgt actttccacc tgggccacaa tcacagtttc cgcagtatcc atcatcagtt     420
   ggaacgcctc tgagcactcc atcacctgag tcaggtaata catttactga ttcatcctca     480
   gcggactctg atatgacatc cactaaaaaa tatgtcagac caccaccaat gttaacctca     540
   cctaatgact ttccaaattg ggttaaaaca tacatcaaat ttttacaaaa ctcgaatctc 600
   ggtggtatta ttccgacagt aaacggaaaa cccgtacgtc cgatcactga tgatgaactc 660
   accttcttgt ataacgcttt tcaaatattt gctccctctc aattcctacc tacctgggtc 720
   aaagacatcc tatccgttga ttatacggat atcatgaaaa ttctttccaa aagtattgaa 780
   aaaatgcaat ctgataccca agaggcaaac gacattgtga ccctggcaaa tttgcaatat 840
   aatggcagta cacctgcaga tgcatttgaa acaaaagtca caaacattat cgacagactg 900
   aacaataatg gcattcatat caataacaag gtcgcatgtc aattaattat gagaggtcta 960
   tctggcgaat ataaattttt acgctacaca cgtcatcgac atctaaatat gacagtcgct 1020
   gaactgttct tagatatcca tgctatttat gaagaacaac agggatcgag aaacagcaaa 1080
   cctaattaca ggagaaatcc gagtgatgag aagaatgatt ctcgcagcta tacgaataca 1140
   accaaaccca aagttatagc tcggaatcct caaaaaacaa ataattcgaa atcgaaaaca 1200
   gccagggctc acaatgtatc cacatctaat aactctccca gcacggacaa cgattccatc 1260
   agtaaatcaa ctactgaacc gattcaattg aacaataagc acgaccttca tcttaggcca 1320
   gaaacttacg aattgatatc aagcttaatg gtgagcaagg gcgaggagct gttcaccggg 1380
   gtggtgccca tcctggtcga gctggacggc gacgtaaacg gccacaagtt cagcgtgtcc 1440
   ggcgagggcg agggcgatgc cacctacggc aagctgaccc tgaagttcat ctgcaccacc 1500
   ggcaagctgc ccgtgccctg gcccaccctc gtgaccaccc tgacctacgg cgtgcagtgc 1560
   ttcagccgct accccgacca catgaagcag cacgacttct tcaagtccgc catgcccgaa 1620
   ggctacgtcc aggagcgcac catcttcttc aaggacgacg gcaactacaa gacccgcgcc 1680
   gaggtgaagt tcgagggcga caccctggtg aaccgcatcg agctgaaggg catcgacttc 1740
   aaggaggacg gcaacatcct ggggcacaag ctggagtaca actacaacag ccacaacgtc 1800
   tatatcatgg ccgacaagca gaagaacggc atcaaggtga acttcaagat ccgccacaac 1860
   atcgaggacg gcagcgtgca gctcgccgac cactaccagc agaacacccc catcggcgac 1920
   ggccccgtgc tgctgcccga caaccactac ctgagcaccc agtccgccct gagcaaagac 1980
   cccaacgaga agcgcgatca catggtcctg ctggagttcg tgaccgccgc cgggatcact 2040
   ctcggcatgg acgagctgta caagcggcag cggcagcgcc atcatcatca tcatcattaa 2100
   gcggccgctg taattagtta tgtcacgctt acattcacgc cctccatatc cgtacaccat 2160
   cagggtacga gctagcccat ggcgtacacc atcagggtac gacgagtaga tctcgtacac 2220
   catcagggta cggaatcgct gacgtacctt taatatagtg tgatttttaa aaactttcga 2280
   acaagaatca gtaatataat atatataatt aataaaacta atggaatttg tttaattgaa 2340
   cttgacaccc gagaagtgcg gccgcggtac c 2371
<210> 39
   <211> 2101
   <212> DNA
   <213> Artificial sequence
<220>
   <223> GAG de Ty1 - eGFP - 6His
<400> 39
   ctagttctat ggaatcccaa caattatcta attacccaca tatatctcat ggtagcgcct      60
   gtgcttcggt tacttctaag gaagtccaca caaatcaaga tccgttagac gtttcagctt     120
   ccaaaattca agaatatgat aaggcttcca ctaaggctaa ctctcaacag acaacaacac     180
   ctgcttcatc agctgttcca gagaaccccc atcatgcctc tcctcaacct gcttcagtac     240
   cacctccaca gaatgggccg tacccacagc agtgcatgat gacccaaaac caagccaatc     300
   catctggttg gtcattttac ggacacccat ctatgattcc gtatacacct tatcaaatgt     360
   cgcctatgta ctttccacct gggccacaat cacagtttcc gcagtatcca tcatcagttg     420
   gaacgcctct gagcactcca tcacctgagt caggtaatac atttactgat tcatcctcag     480
   cggactctga tatgacatcc actaaaaaat atgtcagacc accaccaatg ttaacctcac     540
   ctaatgactt tccaaattgg gttaaaacat acatcaaatt tttacaaaac tcgaatctcg     600
   gtggtattat tccgacagta aacggaaaac ccgtacgtcc gatcactgat gatgaactca     660
   ccttcttgta taacgctttt caaatatttg ctccctctca attcctacct acctgggtca     720
   aagacatcct atccgttgat tatacggata tcatgaaaat tctttccaaa agtattgaaa     780
   aaatgcaatc tgatacccaa gaggcaaacg acattgtgac cctggcaaat ttgcaatata     840
   atggcagtac acctgcagat gcatttgaaa caaaagtcac aaacattatc gacagactga     900
   acaataatgg cattcatatc aataacaagg tcgcatgtca attaattatg agaggtctat     960
   ctggcgaata taaattttta cgctacacac gtcatcgaca tctaaatatg acagtcgctg    1020
   aactgttctt agatatccat gctatttatg aagaacaaca gggatcgaga aacagcaaac    1080
   ctaattacag gagaaatccg agtgatgaga agaatgattc tcgcagctat acgaatacaa    1140
   ccaaacccaa agttatagct cggaatcctc aaaaaacaaa taattcgaaa tcgaaaacag    1200
   ccagggctca caatgtatcc acatctaata actctcccag cacggacaac gattccatca    1260
   gtaaatcaac tactgaaccg attcaattga acaataagca cgaccttcat cttaggccag    1320
   aaacttacga attgatatca agcttaatgg tgagcaaggg cgaggagctg ttcaccgggg    1380
   tggtgcccat cctggtcgag ctggacggcg acgtaaacgg ccacaagttc agcgtgtccg    1440
   gcgagggcga gggcgatgcc acctacggca agctgaccct gaagttcatc tgcaccaccg    1500
   gcaagctgcc cgtgccctgg cccaccctcg tgaccaccct gacctacggc gtgcagtgct    1560
   tcagccgcta ccccgaccac atgaagcagc acgacttctt caagtccgcc atgcccgaag    1620
   gctacgtcca ggagcgcacc atcttcttca aggacgacgg caactacaag acccgcgccg    1680
   aggtgaagtt cgagggcgac accctggtga accgcatcga gctgaagggc atcgacttca    1740
   aggaggacgg caacatcctg gggcacaagc tggagtacaa ctacaacagc cacaacgtct    1800
   atatcatggc cgacaagcag aagaacggca tcaaggtgaa cttcaagatc cgccacaaca    1860
   tcgaggacgg cagcgtgcag ctcgccgacc actaccagca gaacaccccc atcggcgacg    1920
   gccccgtgct gctgcccgac aaccactacc tgagcaccca gtccgccctg agcaaagacc    1980
   ccaacgagaa gcgcgatcac atggtcctgc tggagttcgt gaccgccgcc gggatcactc    2040
   tcggcatgga cgagctgtac aagcggcagc ggcagcgcca tcatcatcat catcattaag    2100
   c                                                                    2101
<210> 40
   <211> 696
   <212> PRT
   <213> Artificial sequence
<220>
   <223> GAG de Ty1 - eGFP - 6His
<400> 40
   Met Glu Ser Gln Gln Leu Ser Asn Tyr Pro His Ile Ser His Gly Ser
   1               5                   10                  15
   Ala Cys Ala Ser Val Thr Ser Lys Glu Val His Thr Asn Gln Asp Pro
               20                  25                  30
   Leu Asp Val Ser Ala Ser Lys Ile Gln Glu Tyr Asp Lys Ala Ser Thr
           35                  40                  45
   Lys Ala Asn Ser Gln Gln Thr Thr Thr Pro Ala Ser Ser Ala Val Pro
       50                  55                  60
   Glu Asn Pro His His Ala Ser Pro Gln Pro Ala Ser Val Pro Pro Pro
   65 70 75 80
   Gln Asn Gly Pro Tyr Pro Gln Gln Cys Met Met Thr Gln Asn Gln Ala
   85 90 95
   Asn Pro Ser Gly Trp Ser Phe Tyr Gly His Pro Ser Met Ile Pro Tyr
   100 105 110
   Thr Pro Tyr Gln Met Ser Pro Met Tyr Phe Pro Pro Gly Pro Gln Ser
   115 120 125
   Gln Phe Pro Gln Tyr Pro Ser Ser Val Gly Thr Pro Leu Ser Thr Pro
   130 135 140
   Ser Pro Glu Ser Gly Asn Thr Phe Thr Asp Ser Ser Ser Ala Asp Ser
   145 150 155 160
   Asp Met Thr Ser Thr Lys Lys Tyr Val Arg Pro Pro Pro Met Leu Thr
   165 170 175
   Ser Pro Asn Asp Phe Pro Asn Trp Val Lys Thr Tyr Ile Lys Phe Leu
   180 185 190
   Gln Asn Ser Asn Leu Gly Gly Ile Ile Pro Thr Val Asn Gly Lys Pro
   195 200 205
   Val Arg Pro Ile Thr Asp Asp Glu Leu Thr Phe Leu Tyr Asn Ala Phe
   210 215 220
   Gln Ile Phe Ala Pro Ser Gln Phe Leu Pro Thr Trp Val Lys Asp Ile
   225 230 235 240
   Leu Ser Val Asp Tyr Thr Asp Ile Met Lys Ile Leu Ser Lys Ser Ile
   245 250 255
   Glu Lys Met Gln Ser Asp Thr Gln Glu Ala Asn Asp Ile Val Thr Leu
   260 265 270
   Ala Asn Leu Gln Tyr Asn Gly Ser Thr Pro Ala Asp Ala Phe Glu Thr
   275 280 285
   Lys Val Thr Asn Ile Ile Asp Arg Leu Asn Asn Asn Gly Ile His Ile
   290 295 300
   Asn Asn Lys Val Ala Cys Gln Leu Ile Met Arg Gly Leu Ser Gly Glu
   305 310 315 320
   Tyr Lys Phe Leu Arg Tyr Thr Arg His Arg His Leu Asn Met Thr Val
   325 330 335
   Ala Glu Leu Phe Leu Asp Ile His Ala Ile Tyr Glu Glu Gln Gln Gly
   340 345 350
   Ser Arg Asn Ser Lys Pro Asn Tyr Arg Arg Asn Pro Ser Asp Glu Lys
   355 360 365
   Asn Asp Ser Arg Ser Tyr Thr Asn Thr Thr Lys Pro Lys Val Ile Ala
   370 375 380
   Arg Asn Pro Gln Lys Thr Asn Asn Ser Lys Ser Lys Thr Ala Arg Ala
   385 390 395 400
   His Asn Val Ser Thr Ser Asn Asn Ser Pro Ser Thr Asp Asn Asp Ser
   405 410 415
   Ile Ser Lys Ser Thr Thr Glu Pro Ile Gln Leu Asn Asn Lys His Asp
               420                 425                 430
   Leu His Leu Arg Pro Glu Thr Tyr Glu Leu Ile Ser Ser Leu Met Val
           435                 440                 445
   Ser Lys Gly Glu Glu Leu Phe Thr Gly Val Val Pro Ile Leu Val Glu
       450                 455                 460
   Leu Asp Gly Asp Val Asn Gly His Lys Phe Ser Val Ser Gly Glu Gly
   465                 470                 475                 480
   Glu Gly Asp Ala Thr Tyr Gly Lys Leu Thr Leu Lys Phe Ile Cys Thr
                   485                 490                 495
   Thr Gly Lys Leu Pro Val Pro Trp Pro Thr Leu Val Thr Thr Leu Thr
               500                 505                 510
   Tyr Gly Val Gln Cys Phe Ser Arg Tyr Pro Asp His Met Lys Gln His
           515                 520                 525
   Asp Phe Phe Lys Ser Ala Met Pro Glu Gly Tyr Val Gln Glu Arg Thr
       530                 535                 540
   Ile Phe Phe Lys Asp Asp Gly Asn Tyr Lys Thr Arg Ala Glu Val Lys
   545                 550                 555                 560
   Phe Glu Gly Asp Thr Leu Val Asn Arg Ile Glu Leu Lys Gly Ile Asp
                   565                 570                 575
   Phe Lys Glu Asp Gly Asn Ile Leu Gly His Lys Leu Glu Tyr Asn Tyr
               580                 585                 590
   Asn Ser His Asn Val Tyr Ile Met Ala Asp Lys Gln Lys Asn Gly Ile
           595                 600                 605
   Lys Val Asn Phe Lys Ile Arg His Asn Ile Glu Asp Gly Ser Val Gln
       610                 615                 620
   Leu Ala Asp His Tyr Gln Gln Asn Thr Pro Ile Gly Asp Gly Pro Val
   625                 630                 635                 640
   Leu Leu Pro Asp Asn His Tyr Leu Ser Thr Gln Ser Ala Leu Ser Lys
                   645                 650                 655
   Asp Pro Asn Glu Lys Arg Asp His Met Val Leu Leu Glu Phe Val Thr
               660                 665                 670
   Ala Ala Gly Ile Thr Leu Gly Met Asp Glu Leu Tyr Lys Arg Gln Arg
           675                 680                 685
   Gln Arg His His His His His His
       690                 695
<210> 41
   <211> 2058
   <212> DNA
   <213> Artificial sequence
<220>
   <223> GAG de Ty1 - eGFP
<400> 41
   atggaatccc aacaattatc taattaccca catatatctc atggtagcgc ctgtgcttcg 60
   gttacttcta aggaagtcca cacaaatcaa gatccgttag acgtttcagc ttccaaaatt 120
   caagaatatg ataaggcttc cactaaggct aactctcaac agacaacaac acctgcttca 180
   tcagctgttc cagagaaccc ccatcatgcc tctcctcaac ctgcttcagt accacctcca 240
   cagaatgggc cgtacccaca gcagtgcatg atgacccaaa accaagccaa tccatctggt 300
   tggtcatttt acggacaccc atctatgatt ccgtatacac cttatcaaat gtcgcctatg 360
   tactttccac ctgggccaca atcacagttt ccgcagtatc catcatcagt tggaacgcct 420
   ctgagcactc catcacctga gtcaggtaat acatttactg attcatcctc agcggactct 480
   gatatgacat ccactaaaaa atatgtcaga ccaccaccaa tgttaacctc acctaatgac 540
   tttccaaatt gggttaaaac atacatcaaa tttttacaaa actcgaatct cggtggtatt 600
   attccgacag taaacggaaa acccgtacgt ccgatcactg atgatgaact caccttcttg 660
   tataacgctt ttcaaatatt tgctccctct caattcctac ctacctgggt caaagacatc 720
   ctatccgttg attatacgga tatcatgaaa attctttcca aaagtattga aaaaatgcaa 780
   tctgataccc aagaggcaaa cgacattgtg accctggcaa atttgcaata taatggcagt 840
   acacctgcag atgcatttga aacaaaagtc acaaacatta tcgacagact gaacaataat 900
   ggcattcata tcaataacaa ggtcgcatgt caattaatta tgagaggtct atctggcgaa 960
   tataaatttt tacgctacac acgtcatcga catctaaata tgacagtcgc tgaactgttc 1020
   ttagatatcc atgctattta tgaagaacaa cagggatcga gaaacagcaa acctaattac 1080
   aggagaaatc cgagtgatga gaagaatgat tctcgcagct atacgaatac aaccaaaccc 1140
   aaagttatag ctcggaatcc tcaaaaaaca aataattcga aatcgaaaac agccagggct 1200
   cacaatgtat ccacatctaa taactctccc agcacggaca acgattccat cagtaaatca 1260
   actactgaac cgattcaatt gaacaataag cacgaccttc atcttaggcc agaaacttac 1320
   gattcgatat caagcttaat ggtgagcaag ggcgaggagc tgttcaccgg ggtggtgccc 1380
   atcctggtcg agctggacgg cgacgtaaac ggccacaagt tcagcgtgtc cggcgagggc 1440
   gagggcgatg ccacctacgg caagctgacc ctgaagttca tctgcaccac cggcaagctg 1500
   cccgtgccct ggcccaccct cgtgaccacc ctgacctacg gcgtgcagtg cttcagccgc 1560
   taccccgacc acatgaagca gcacgacttc ttcaagtccg ccatgcccga aggctacgtc 1620
   caggagcgca ccatcttctt caaggacgac ggcaactaca agacccgcgc cgaggtgaag 1680
   ttcgagggcg acaccctggt gaaccgcatc gagctgaagg gcatcgactt caaggaggac 1740
   ggcaacatcc tggggcacaa gctggagtac aactacaaca gccacaacgt ctatatcatg 1800
   gccgacaagc agaagaacgg catcaaggtg aacttcaaga tccgccacaa catcgaggac 1860
   ggcagcgtgc agctcgccga ccactaccag cagaacaccc ccatcggcga cggccccgtg 1920
   ctgctgcccg acaaccacta cctgagcacc cagtccgccc tgagcaaaga ccccaacgag 1980
   aagcgcgatc acatggtcct gctggagttc gtgaccgccg ccgggatcac tctcggcatg 2040
   gacgagctgt acaagtaa 2058
<210> 42
   <211> 685
   <212> PRT
   <213> Artificial sequence
<220>
   <223> GAG de Ty1 - eGFP
<400> 42
   Met Glu Ser Gln Gln Leu Ser Asn Tyr Pro His Ile Ser His Gly Ser
   1               5                   10                  15
   Ala Cys Ala Ser Val Thr Ser Lys Glu Val His Thr Asn Gln Asp Pro
               20                  25                  30
   Leu Asp Val Ser Ala Ser Lys Ile Gln Glu Tyr Asp Lys Ala Ser Thr
           35                  40                  45
   Lys Ala Asn Ser Gln Gln Thr Thr Thr Pro Ala Ser Ser Ala Val Pro
       50                  55                  60
   Glu Asn Pro His His Ala Ser Pro Gln Pro Ala Ser Val Pro Pro Pro
   65                  70                  75                  80
   Gln Asn Gly Pro Tyr Pro Gln Gln Cys Met Met Thr Gln Asn Gln Ala
                   85                  90                  95
   Asn Pro Ser Gly Trp Ser Phe Tyr Gly His Pro Ser Met Ile Pro Tyr
               100                 105                 110
   Thr Pro Tyr Gln Met Ser Pro Met Tyr Phe Pro Pro Gly Pro Gln Ser
           115                 120                 125
   Gln Phe Pro Gln Tyr Pro Ser Ser Val Gly Thr Pro Leu Ser Thr Pro
       130                 135                 140
   Ser Pro Glu Ser Gly Asn Thr Phe Thr Asp Ser Ser Ser Ala Asp Ser
   145                 150                 155                 160
   Asp Met Thr Ser Thr Lys Lys Tyr Val Arg Pro Pro Pro Met Leu Thr
                   165                 170                 175
   Ser Pro Asn Asp Phe Pro Asn Trp Val Lys Thr Tyr Ile Lys Phe Leu
               180                 185                 190
   Gln Asn Ser Asn Leu Gly Gly Ile Ile Pro Thr Val Asn Gly Lys Pro
           195                 200                 205
   Val Arg Pro Ile Thr Asp Asp Glu Leu Thr Phe Leu Tyr Asn Ala Phe
       210                 215                 220
   Gln Ile Phe Ala Pro Ser Gln Phe Leu Pro Thr Trp Val Lys Asp Ile
   225                 230                 235                 240
   Leu Ser Val Asp Tyr Thr Asp Ile Met Lys Ile Leu Ser Lys Ser Ile
                   245                 250                 255
   Glu Lys Met Gln Ser Asp Thr Gln Glu Ala Asn Asp Ile Val Thr Leu
               260                 265                 270
   Ala Asn Leu Gln Tyr Asn Gly Ser Thr Pro Ala Asp Ala Phe Glu Thr
           275                 280                 285
   Lys Val Thr Asn Ile Ile Asp Arg Leu Asn Asn Asn Gly Ile His Ile
       290                 295                 300
   Asn Asn Lys Val Ala Cys Gln Leu Ile Met Arg Gly Leu Ser Gly Glu
   305                 310                 315                 320
   Tyr Lys Phe Leu Arg Tyr Thr Arg His Arg His Leu Asn Met Thr Val
                   325                 330                 335
   Ala Glu Leu Phe Leu Asp Ile His Ala Ile Tyr Glu Glu Gln Gln Gly
               340                 345                 350
   Ser Arg Asn Ser Lys Pro Asn Tyr Arg Arg Asn Pro Ser Asp Glu Lys
           355                 360                 365
   Asn Asp Ser Arg Ser Tyr Thr Asn Thr Thr Lys Pro Lys Val Ile Ala
       370                 375                 380
   Arg Asn Pro Gln Lys Thr Asn Asn Ser Lys Ser Lys Thr Ala Arg Ala
   385                 390                 395                 400
   His Asn Val Ser Thr Ser Asn Asn Ser Pro Ser Thr Asp Asn Asp Ser
                   405                 410                 415
   Ile Ser Lys Ser Thr Thr Glu Pro Ile Gln Leu Asn Asn Lys His Asp
               420                 425                 430
   Leu His Leu Arg Pro Glu Thr Tyr Asp Ser Ile Ser Ser Leu Met Val
           435                 440                 445
   Ser Lys Gly Glu Glu Leu Phe Thr Gly Val Val Pro Ile Leu Val Glu
       450                 455                 460
   Leu Asp Gly Asp Val Asn Gly His Lys Phe Ser Val Ser Gly Glu Gly
   465                 470                 475                 480
   Glu Gly Asp Ala Thr Tyr Gly Lys Leu Thr Leu Lys Phe Ile Cys Thr
                   485                 490                 495
   Thr Gly Lys Leu Pro Val Pro Trp Pro Thr Leu Val Thr Thr Leu Thr
               500                 505                 510
   Tyr Gly Val Gln Cys Phe Ser Arg Tyr Pro Asp His Met Lys Gln His
           515                 520                 525
   Asp Phe Phe Lys Ser Ala Met Pro Glu Gly Tyr Val Gln Glu Arg Thr
       530                 535                 540
   Ile Phe Phe Lys Asp Asp Gly Asn Tyr Lys Thr Arg Ala Glu Val Lys
   545                 550                 555                 560
   Phe Glu Gly Asp Thr Leu Val Asn Arg Ile Glu Leu Lys Gly Ile Asp
                   565                 570                 575
   Phe Lys Glu Asp Gly Asn Ile Leu Gly His Lys Leu Glu Tyr Asn Tyr
               580                 585                 590
   Asn Ser His Asn Val Tyr Ile Met Ala Asp Lys Gln Lys Asn Gly Ile
           595                 600                 605
   Lys Val Asn Phe Lys Ile Arg His Asn Ile Glu Asp Gly Ser Val Gln
       610                 615                 620
   Leu Ala Asp His Tyr Gln Gln Asn Thr Pro Ile Gly Asp Gly Pro Val
   625                 630                 635                 640
   Leu Leu Pro Asp Asn His Tyr Leu Ser Thr Gln Ser Ala Leu Ser Lys
                   645                 650                 655
   Asp Pro Asn Glu Lys Arg Asp His Met Val Leu Leu Glu Phe Val Thr
               660                 665                 670
   Ala Ala Gly Ile Thr Leu Gly Met Asp Glu Leu Tyr Lys
           675                 680                 685
<210> 43
   <211> 3208
   <212> DNA
   <213> Artificial sequence
<220>
   <223> Luciférase - EngGAG - 3MS2
<400> 43
   atggaagacg ccaaaaacat aaagaaaggc ccggcgccat tctatccgct ggaagatgga      60
   accgctggag agcaactgca taaggctatg aagagatacg ccctggttcc tggaacaatt     120
   gcttttacag atgcacatat cgaggtggac atcacttacg ctgagtactt cgaaatgtcc     180
   gttcggttgg cagaagctat gaaacgatat gggctgaata caaatcacag aatcgtcgta     240
   tgcagtgaaa actctcttca attctttatg ccggtgttgg gcgcgttatt tatcggagtt     300
   gcagttgcgc ccgcgaacga catttataat gaacgtgaat tgctcaacag tatgggcatt     360
   tcgcagccta ccgtggtgtt cgtttccaaa aaggggttgc aaaaaatttt gaacgtgcaa     420
   aaaaagctcc caatcatcca aaaaattatt atcatggatt ctaaaacgga ttaccaggga     480
   tttcagtcga tgtacacgtt cgtcacatct catctacctc ccggttttaa tgaatacgat     540
   tttgtgccag agtccttcga tagggacaag acaattgcac tgatcatgaa ctcctctgga     600
   tctactggtc tgcctaaagg tgtcgctctg cctcatagaa ctgcctgcgt gagattctcg     660
   catgccagag atcctatttt tggcaatcaa atcattccgg atactgcgat tttaagtgtt     720
   gttccattcc atcacggttt tggaatgttt actacactcg gatatttgat atgtggattt     780
   cgagtcgtct taatgtatag atttgaagaa gagctgtttc tgaggagcct tcaggattac     840
   aagattcaaa gtgcgctgct ggtgccaacc ctattctcct tcttcgccaa aagcactctg     900
   attgacaaat acgatttatc taatttacac gaaattgctt ctggtggcgc tcccctctct     960
   aaggaagtcg gggaagcggt tgccaagagg ttccatctgc caggtatcag gcaaggatat    1020
   gggctcactg agactacatc agctattctg attacacccg agggggatga taaaccgggc    1080
   gcggtcggta aagttgttcc attttttgaa gcgaaggttg tggatctgga taccgggaaa    1140
   acgctgggcg ttaatcaaag aggcgaactg tgtgtgagag gtcctatgat tatgtccggt    1200
   tatgtaaaca atccggaagc gaccaacgcc ttgattgaca aggatggatg gctacattct    1260
   ggagacatag cttactggga cgaagacgaa cacttcttca tcgttgaccg cctgaagtct    1320
   ctgattaagt acaaaggcta tcaggtggct cccgctgaat tggaatccat cttgctccaa    1380
   caccccaaca tcttcgacgc aggtgtcgca ggtcttcccg acgatgacgc cggtgaactt    1440
   cccgccgccg ttgttgtttt ggagcacgga aagacgatga cggaaaaaga gatcgtggat    1500
   tacgtcgcca gtcaagtaac aaccgcgaaa aagttgcgcg gaggagttgt gtttgtggac    1560
   gaagtaccga aaggtcttac cggaaaactc gacgcaagaa aaatcagaga gatcctcata    1620
   aaggccaaga agggcggaaa gatcgccgtg taattctaga gtcggggcgg ccggccgctt    1680
   cgagcagaca tgataagata cattgatgag tttggacaaa ccacaactag aatgcagcat    1740
   atgccacagc agtgcatgat gacccaaaac caagccaatc catctggttg gtcattttac    1800
   ggacacccat ctatgattcc gtatacacct tatcaaatgt cgcctatgta ctttccacct    1860
   gggccacaat cacagtttcc gcagtatcca tcatcagttg gaacgcctct gagcactcca    1920
   tcacctgagt caggtaatac atttactgat tcatcctcag cggactctga tatgacatcc    1980
   actaaaaaat atgtcagacc accaccaatg ttaacctcac ctaatgactt tccaaattgg    2040
   gttaaaacat acatcaaatt tttacaaaac tcgaatctcg gtggtattat tccgacagta    2100
   aacggaaaac ccgtacgtcc gatcactgat gatgaactca ccttcttgta taacgctttt    2160
   caaatatttg ctccctctca attcctacct acctgggtca aagacatcct atccgttgat    2220
   tatacggata tcatgaaaat tctttccaaa agtattgaaa aaatgcaatc tgatacccaa    2280
   gaggcaaacg acattgtgac cctggcaaat ttgcaatata atggcagtac acctgcagat    2340
   gcatttgaaa caaaagtcac aaacattatc gacagactga acaataatgg cattcatatc    2400
   aataacaagg tcgcatgtca attaattatg agaggtctat ctggcgaata taaattttta    2460
   cgctacacac gtcatcgaca tctaaatatg acagtcgctg aactgttctt agatatccat    2520
   gctatttatg aagaacaaca gggatcgaga aacagcaaac ctaattacag gagaaatccg    2580
   agtgatgaga agaatgattc tcgcagctat acgaatacaa ccaaacccaa agttatagct     2640
   cggaatcctc aaaaaacaaa taattcgaaa tcgaaaacag ccagggctca caatgtatcc     2700
   acatctaata actctcccag cacggacaac gattccatca gtaaatcaac tactgaaccg     2760
   attcaattga acaataagca cgaccttcat cttaggccag aaacttactg agcggccgct     2820
   gtaattagtt atgtcacgct tacattcacg ccctccatat ccgtacacca tcagggtacg     2880
   agctagccca tggcgtacac catcagggta cgacgagtag atctcgtaca ccatcagggt     2940
   acggaatcgc tgacggtacc tttaatatag tgtgattttt aaaaactttc gaacaagaat     3000
   cagtaatata atatatataa ttaataaaac taatggaatt tgtttaattg aacttgacac     3060
   ccgagaagga aggagttaga caacctgaag tctaggtccc tatttatttt tttatagtta     3120
   tgttagtatt aagaacgtta tttatatttc aaatttttct tttttttctg tacagacgcg     3180
   tgtacgcatg taacattgcg gccgccct                                         3208
<210> 44
   <211> 2822
   <212> DNA
   <213> Artificial sequence
<220>
   <223> Luciférase - EndGAG
<400> 44
   atggaagacg ccaaaaacat aaagaaaggc ccggcgccat tctatccgct ggaagatgga      60
   accgctggag agcaactgca taaggctatg aagagatacg ccctggttcc tggaacaatt     120
   gcttttacag atgcacatat cgaggtggac atcacttacg ctgagtactt cgaaatgtcc     180
   gttcggttgg cagaagctat gaaacgatat gggctgaata caaatcacag aatcgtcgta     240
   tgcagtgaaa actctcttca attctttatg ccggtgttgg gcgcgttatt tatcggagtt     300
   gcagttgcgc ccgcgaacga catttataat gaacgtgaat tgctcaacag tatgggcatt     360
   tcgcagccta ccgtggtgtt cgtttccaaa aaggggttgc aaaaaatttt gaacgtgcaa     420
   aaaaagctcc caatcatcca aaaaattatt atcatggatt ctaaaacgga ttaccaggga     480
   tttcagtcga tgtacacgtt cgtcacatct catctacctc ccggttttaa tgaatacgat     540
   tttgtgccag agtccttcga tagggacaag acaattgcac tgatcatgaa ctcctctgga     600
   tctactggtc tgcctaaagg tgtcgctctg cctcatagaa ctgcctgcgt gagattctcg     660
   catgccagag atcctatttt tggcaatcaa atcattccgg atactgcgat tttaagtgtt     720
   gttccattcc atcacggttt tggaatgttt actacactcg gatatttgat atgtggattt     780
   cgagtcgtct taatgtatag atttgaagaa gagctgtttc tgaggagcct tcaggattac     840
   aagattcaaa gtgcgctgct ggtgccaacc ctattctcct tcttcgccaa aagcactctg     900
   attgacaaat acgatttatc taatttacac gaaattgctt ctggtggcgc tcccctctct     960
   aaggaagtcg gggaagcggt tgccaagagg ttccatctgc caggtatcag gcaaggatat    1020
   gggctcactg agactacatc agctattctg attacacccg agggggatga taaaccgggc    1080
   gcggtcggta aagttgttcc attttttgaa gcgaaggttg tggatctgga taccgggaaa    1140
   acgctgggcg ttaatcaaag aggcgaactg tgtgtgagag gtcctatgat tatgtccggt    1200
   tatgtaaaca atccggaagc gaccaacgcc ttgattgaca aggatggatg gctacattct    1260
   ggagacatag cttactggga cgaagacgaa cacttcttca tcgttgaccg cctgaagtct    1320
   ctgattaagt acaaaggcta tcaggtggct cccgctgaat tggaatccat cttgctccaa    1380
   caccccaaca tcttcgacgc aggtgtcgca ggtcttcccg acgatgacgc cggtgaactt    1440
   cccgccgccg ttgttgtttt ggagcacgga aagacgatga cggaaaaaga gatcgtggat    1500
   tacgtcgcca gtcaagtaac aaccgcgaaa aagttgcgcg gaggagttgt gtttgtggac    1560
   gaagtaccga aaggtcttac cggaaaactc gacgcaagaa aaatcagaga gatcctcata    1620
   aaggccaaga agggcggaaa gatcgccgtg taattctaga gtcggggcgg ccggccgctt    1680
   cgagcagaca tgataagata cattgatgag tttggacaaa ccacaactag aatgcagcat    1740
   atgccacagc agtgcatgat gacccaaaac caagccaatc catctggttg gtcattttac    1800
   ggacacccat ctatgattcc gtatacacct tatcaaatgt cgcctatgta ctttccacct    1860
   gggccacaat cacagtttcc gcagtatcca tcatcagttg gaacgcctct gagcactcca    1920
   tcacctgagt caggtaatac atttactgat tcatcctcag cggactctga tatgacatcc    1980
   actaaaaaat atgtcagacc accaccaatg ttaacctcac ctaatgactt tccaaattgg    2040
   gttaaaacat acatcaaatt tttacaaaac tcgaatctcg gtggtattat tccgacagta    2100
   aacggaaaac ccgtacgtcc gatcactgat gatgaactca ccttcttgta taacgctttt    2160
   caaatatttg ctccctctca attcctacct acctgggtca aagacatcct atccgttgat    2220
   tatacggata tcatgaaaat tctttccaaa agtattgaaa aaatgcaatc tgatacccaa    2280
   gaggcaaacg acattgtgac cctggcaaat ttgcaatata atggcagtac acctgcagat    2340
   gcatttgaaa caaaagtcac aaacattatc gacagactga acaataatgg cattcatatc    2400
   aataacaagg tcgcatgtca attaattatg agaggtctat ctggcgaata taaattttta    2460
   cgctacacac gtcatcgaca tctaaatatg acagtcgctg aactgttctt agatatccat    2520
   gctatttatg aagaacaaca gggatcgaga aacagcaaac ctaattacag gagaaatccg    2580
   agtgatgaga agaatgattc tcgcagctat acgaatacaa ccaaacccaa agttatagct    2640
   cggaatcctc aaaaaacaaa taattcgaaa tcgaaaacag ccagggctca caatgtatcc    2700
   acatctaata actctcccag cacggacaac gattccatca gtaaatcaac tactgaaccg    2760
   attcaattga acaataagca cgaccttcat cttaggccag aaacttactg agcggccgct    2820
   gt                                                                  2822